(19) Europäisches Patentamt
European Patent Office
Office européen des brevets

(11) EP 3 504 343 B1

(12) **EUROPEAN PATENT SPECIFICATION**

(45) Date of publication and mention of the grant of the patent:
**24.03.2021 Bulletin 2021/12**

(21) Application number: **17768367.9**

(22) Date of filing: **23.08.2017**

(51) Int Cl.:
***C12Q 1/6883*** *(2018.01)*

(86) International application number:
**PCT/EP2017/071196**

(87) International publication number:
**WO 2018/037031 (01.03.2018 Gazette 2018/09)**

(54) **METHOD OF DETECTING ACTIVE TUBERCULOSIS USING MINIMAL GENE SIGNATURE**

VERFAHREN ZUM NACHWEIS VON AKTIVER TUBERKULOSE MIT MINIMALER GENSIGNATUR

PROCÉDÉ DE DÉTECTION DE TUBERCULOSE ACTIVE À L'AIDE D'UNE SIGNATURE GÉNIQUE MINIMALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.08.2016 GB 201614394**

(43) Date of publication of application:
**03.07.2019 Bulletin 2019/27**

(73) Proprietor: **Imperial College Of Science, Technology And Medicine**
**London SW7 2AZ (GB)**

(72) Inventors:
- **LEVIN, Michael**
**London W2 1PZ (GB)**
- **KAFOROU, Myrsini**
**London W2 1PZ (GB)**
- **COIN, Lachlan**
**London W2 1PZ (GB)**

(74) Representative: **Sterling IP**
**Orion House**
**Bessemer Road**
**Welwyn Garden City AL7 1HH (GB)**

(56) References cited:
**WO-A1-2014/019977 WO-A1-2014/093872**

- **KASSA DESTA ET AL: "Discriminative expression of whole blood genes in HIV patients with latent and active TB in Ethiopia", TUBERCULOSIS, C, vol. 100, 13 June 2016 (2016-06-13), pages 25-31, XP029694904, ELSEVIER, GB ISSN: 1472-9792, DOI: 10.1016/J.TUBE.2016.06.003**
- **DAWANY NOOR ET AL: "Identification of a 251 gene expression signature that can accurately detect M. tuberculosis in patients with and without HIV co-infection.", PLOS ONE, vol. 9, no. 2, E89925, 25 February 2014 (2014-02-25), pages 1-8, XP002774752, ISSN: 1932-6203, DOI: 10.1371/journal.pone.0089925**
- **SUZANNE T. ANDERSON ET AL: "Diagnosis of Childhood Tuberculosis and Host RNA Expression in Africa", NEW ENGLAND JOURNAL OF MEDICINE, THE - NEJM -, vol. 370, no. 18, 1 May 2014 (2014-05-01), pages 1712-1723, XP055316396, US ISSN: 0028-4793, DOI: 10.1056/NEJMoa1303657**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 504 343 B1

SEQUENCE LISTING

<110> Imperial Innovations Ltd

<120> METHOD OF DETECTING ACTIVE TUBERCULOSIS USING MINIMAL GENE SIGNATURE

<130> P000193_WO

<160> 16

<170> PatentIn version 3.5

<210> 1
<211> 2268
<212> DNA
<213> Artificial Sequence

<220>
<223> Human FCGR1A mRNA sequence

<400> 1

```
aatatcttgc atgttacaga tttcactgct cccaccagct tggagacaac atgtggttct      60
tgacaactct gctcctttgg gttccagttg atgggcaagt ggacaccaca aaggcagtga     120
tcactttgca gcctccatgg gtcagcgtgt tccaagagga aaccgtaacc ttgcactgtg     180
aggtgctcca tctgcctggg agcagctcta cacagtggtt tctcaatggc acagccactc     240
agacctcgac ccccagctac agaatcacct ctgccagtgt caatgacagt ggtgaataca     300
ggtgccagag aggtctctca gggcgaagtg accccataca gctggaaatc cacagaggct     360
ggctactact gcaggtctcc agcagagtct tcacggaagg agaacctctg gccttgaggt     420
gtcatgcgtg gaaggataag ctggtgtaca atgtgcttta ctatcgaaat ggcaaagcct     480
ttaagttttt ccactggaat tctaacctca ccattctgaa aaccaacata agtcacaatg     540
gcacctacca ttgctcaggc atgggaaagc atcgctacac atcagcagga atatctgtca     600
ctgtgaaaga gctatttcca gctccagtgc tgaatgcatc tgtgacatcc ccactcctgg     660
aggggaatct ggtcaccctg agctgtgaaa caaagttgct cttgcagagg cctggtttgc     720
agctttactt ctccttctac atgggcagca agaccctgcg aggcaggaac acatcctctg     780
aataccaaat actaactgct agaagagaag actctgggtt atactggtgc gaggctgcca     840
cagaggatgg aaatgtcctt aagcgcagcc ctgagttgga gcttcaagtg cttggcctcc     900
agttaccaac tcctgtctgg tttcatgtcc ttttctatct ggcagtggga ataatgtttt     960
tagtgaacac tgttctctgg gtgacaatac gtaaagaact gaaaagaaag aaaaagtggg    1020
atttagaaat ctctttggat tctggtcatg agaagaaggt aatttccagc cttcaagaag    1080
acagacattt agaagaagag ctgaaatgtc aggaacaaaa agaagaacag ctgcaggaag    1140
gggtgcaccg gaaggagccc caggggccca cgtagcagcg gctcagtggg tggccatcga    1200
tctggaccgt cccctgccca cttgctcccc gtgagcactg cgtacaaaca tccaaaagtt    1260
```

caacaacacc agaactgtgt gtctcatggt atgtaactct taaagcaaat aaatgaactg 1320
acttcaactg ggatacattt ggaaatgtgg tcatcaaaga tgacttgaaa tgaggcctac 1380
tctaaagaat tcttgaaaaa cttacaagtc aagcctagcc tgataatcct attacatagt 1440
ttgaaaaata gtattttatt tctcagaaca aggtaaaaag gtgagtgggt gcatatgtac 1500
agaagattaa gacagagaaa cagacagaaa gagacacaca cacagccagg agtgggtaga 1560
tttcagggag acaagaggga atagtataga caataaggaa ggaaatagta cttacaaatg 1620
actcctaagg gactgtgaga ctgagagggc tcacgcctct gtgttcagga tacttagttc 1680
atggcttttc tctttgactt tactaaaaga gaatgtctcc atacgcgttc taggcataca 1740
agggggtaac tcatgatgag aaatggatgt gttattcttg ccctctcttt tgaggctctc 1800
tcataacccc tctatttcta gagacaacaa aaatgctgcc agtcctaggc ccctgccctg 1860
taggaaggca gaatgtaact gttctgtttg tttaacgatt aagtccaaat ctccaagtgc 1920
ggcactgcaa agagacgctt caagtgggga gaagcggcga taccatagag tccagatctt 1980
gcctccagag atttgcttta ccttcctgat tttctggtta ctaattagct tcaggatacg 2040
ctgctctcat acttgggctg tagtttggag acaaaatatt ttcctgccac tgtgtaacat 2100
agctgaggta aaaactgaac tatgtaaatg actctactaa aagtttaggg aaaaaaaaca 2160
ggaggagtat gacacaaaaa aaaaaaaaaa aaaaaaaaaa aaaaaaaaaa aaaaaaaaaa 2220
aaaaaaaaaa aaaaaaaaaa aaaaaaaaaa aaaaaaaaaa aaaaaaaa 2268

<210> 2
<211> 1762
<212> DNA
<213> Artificial Sequence

<220>
<223> Human ZNF296 mRNA sequence

<400> 2

cgccgtcagg gccccagcag ggcggctcgg tcccctccca tgagccccgc cctggtaggc 60
ggagccacgg gcccggccag cacgtgtaag agttcgcggc gggtcgccgc agtcactcac 120
ctgagcgcgc acggtccgcg cgtcctccgc tcgtgcgtcc tccgcccgcc cgcctgcctg 180
cctgcccgcc cgctcgctcg cccggcccgc gactcatgtc ccgccgcaag gccggcagcg 240
cgccccgccg agtagagccc gcgcccgccg ccaacccaga cgacgagatg gaaatgcagg 300
acctcgtcat cgaactcaag cccgagccag acgcgcagcc ccaacaggcc ccaaggctgg 360
ggcccttctc cccgaaggag gtgtcctcgg cggggcggtt cggcggcgaa ccccaccact 420
cccctggccc catgcccgcc ggggccgccc tcctcgccct cggcccgcgg aacccgtgga 480
ccctgtggac gccgttgacc ccgaactatc ccgaccgcca gccctggacc gacaaacacc 540
cagatctgtt gacctgcggc cgctgcctgc agaccttccc gttggaggcc atcactgcct 600
tcatggacca caagaagctg ggctgtcagc tcttcagagg ccccagccgc ggccagggct 660
cagaacgaga ggagctgaag gccttgagct gcctgcgctg tggcaaacag ttcacagtgg 720
cctggaagct gctgcgtcac gcccagtggg accacggact gtccatctac cagacagaat 780
cagaggcccc ggaggccccg ctcctgggcc tggccgaggt ggctgcagcc gtgtcggcag 840
tggtggggcc agcagctgag gccaagagcc cccgtgcaag tggcagcggc ctcacccggc 900
ggagccccac ctgtcctgtg tgcaagaaga ccctcagctc cttcagcaac ctcaaagtgc 960
acatgcgctc acacacaggc gagcggccct atgcttgcga ccagtgtccc tacgcctgcg 1020
cccagagcag caagctcaac cgccacaaga agacccaccg gcaggtgccg ccccagagcc 1080
ccctcatggc cgacaccagc caggagcagg cctctgcagc ccctccggag ccggctgtcc 1140
atgctgctgc ccccaccagc acccttccat gcagcggtgg tgaggggggct ggagccgccg 1200
ccacagcagg tgtccaggaa cccggggctc ctggcagtgg ggctcaagcc ggccctggtg 1260
gagacacttg gggagccatc accacggaac aaagaactga ccctgcaaac agccagaagg 1320
catcacccaa aaagatgccc aagtcagggg gcaagagccg cgggcccggg ggcagctgtg 1380
agttctgcgg gaagcatttt accaacagca gcaacctgac ggtgcaccgg cgctcacaca 1440
ccggggagcg cccctacacc tgtgagttct gcaactacgc ctgcgcccag agcagtaagc 1500
tcaaccgcca ccgccgcatg cacggcatga cgcctggcag cacccgcttc gagtgccccc 1560
actgccatgt gcccttcggc ctgcgagcca ccctggacaa acacctgcgg cagaagcacc 1620
ctgaggcggc cggcgaggcc tgagcccagg aaagcccccc tcactgtccc tggtaccgct 1680
gccaacaccc attgacctcc tcgtttttgc ccgccttctc caagtaaatt ttccctttta 1740
tttaaaaaaa aaaaaaaaaa aa 1762

<210> 3

<211> 1044
<212> DNA
<213> Artificial Sequence

<220>
<223> Human C1QB mRNA sequence

<400> 3

gcccttcccg cctctgggga agggaacttc cgcttcggac cgagggcagt aggctctcgg 60
ctcctggtcc cactgctgct cagcccagtg gcctcacagg acaccagctt cccaggaggc 120
gtctgacaca gtatgatgat gaagatccca tggggcagca tcccagtact gatgttgctc 180
ctgctcctgg gcctaatcga tatctcccag gcccagctca gctgcaccgg gcccccagcc 240
atccctggca tcccgggtat ccctgggaca cctggccccg atggccaacc tgggacccca 300
gggataaaag gagagaaagg gcttccaggg ctggctggag accatggtga gttcggagag 360
aagggagacc cagggattcc tgggaatcca ggaaaagtcg gccccaaggg ccccatgggc 420
cctaaaggtg gcccaggggc ccctggagcc ccaggcccca aaggtgaatc gggagactac 480
aaggccaccc agaaaatcgc cttctctgcc acaagaacca tcaacgtccc cctgcgccgg 540
gaccagacca tccgcttcga ccacgtgatc accaacatga acaacaatta tgagccccgc 600
agtggcaagt tcacctgcaa ggtgcccggt ctctactact tcacctacca cgccagctct 660
cgagggaacc tgtgcgtgaa cctcatgcgt ggccgggagc gtgcacagaa ggtggtcacc 720
ttctgtgact atgcctacaa caccttccag gtcaccaccg gtggcatggt cctcaagctg 780
gagcaggggg agaacgtctt cctgcaggcc accgacaaga actcactact gggcatggag 840
ggtgccaaca gcatcttttc cgggttcctg ctctttccag atatggaggc ctgacctgtg 900
ggctgcttca catccacccc ggctccccct gccagcaacg ctcactctac ccccaacacc 960
acccccttgcc caaccaatgc acacagtagg gcttggtgaa tgctgctgag tgaatgagta 1020
aataaactct tcaaggccaa ggga 1044

<210> 4
<211> 4867
<212> DNA
<213> Artificial Sequence

<220>
<223> Human GBP6 transcript variant 1 mRNA sequence

<400> 4

```
acccaacacc catcctcaaa ctcacatcgg atgattcagg catggctctg ctaacacttt      60
attaaaagca tggattaatt ttacttccaa gtttattttt actgcaccat cccatttgtg     120
gaaacaacta gcttactcag cttttttttc cttttataaa ggaaagaaca gaaaagtaaa     180
aggaggaaag aaaacaagag gtgagtgagg caactgaaaa ctgttcttgg acctgcggtg     240
ctatagagca ggctcttcta ggttggcagt tgccatggaa tctggaccca aaatgttggc     300
ccccgtttgc ctggtggaaa ataacaatga gcagctattg gtgaaccagc aagctataca     360
gattcttgaa aagatttctc agccagtggt ggtggtggcc attgtaggac tgtaccgtac     420
agggaaatcc tacttgatga accatctggc aggacagaat catggcttcc ctctgggctc     480
cacggtgcag tctgaaacca agggcatctg gatgtggtgc gtgccccacc catccaagcc     540
aaaccacacc ctggtccttc tggacaccga aggtctgggc gatgtggaaa agggtgaccc     600
taagaatgac tcctggatct ttgccctggc tgtgctcctg tgcagcacct ttgtctacaa     660
cagcatgagc accatcaacc accaggccct ggagcagctg cattatgtga cggagctcac     720
agaactaatt aaggcaaagt cctccccaag gcctgatgga gtagaagatt ccacagagtt     780
tgtgagtttc ttcccagact ttctttggac agtacgggat ttcactctgg agctgaagtt     840
gaacggtcac cctatcacag aagatgaata cctggagaat gccttgaagc tgattcaagg     900
```

caataatccc agagttcaaa catccaattt tcccagggag tgcatcaggc gtttctttcc 960
aaaacggaag tgtttcgtct ttgaccggcc aacaaatgac aaagaccttc tagccaatat 1020
tgagaaggtg tcagaaaagc aactggatcc caaattccag gaacaaacaa acattttctg 1080
ttcttacatc ttcactcatg caagaaccaa gaccctcagg gagggaatca cagtcactgg 1140
gaatcgtctg ggaactctgg cagtgactta tgtagaggcc atcaacagtg gagcagtgcc 1200
ttgtctggag aatgcagtga taactctggc ccagcgtgag aactcagcgg ccgtgcagag 1260
ggcagctgac tactacagcc agcagatggc ccagcgagtg aagctcccca cagacacgct 1320
ccaggagctg ctggacatgc atgcggcctg tgagagggaa gccattgcaa tcttcatgga 1380
gcactccttc aaggatgaaa atcaggaatt ccagaagaag ttcatggaaa ccacaatgaa 1440
taagaagggg gatttcttgc tgcagaatga agagtcatct gttcaatact gccaggctaa 1500
actcaatgag ctctcaaagg gactaatgga aagtatctca gcaggaagtt tctctgttcc 1560
tggagggcac aagctctaca tggaaacaaa ggaaaggatt gaacaggact attggcaagt 1620
tcccaggaaa ggagtaaagg caaaagaggt cttccagagg ttcctggagt cacagatggt 1680
gatagaggaa tccatcttgc agtcagataa agccctcact gatagagaga aggcagtagc 1740
agtggatcgg gccaagaagg aggcagctga gaaggaacag gaacttttaa aacagaaatt 1800
acaggagcag cagcaacaga tggaggctca agataagagt cgcaaggaaa acatagccca 1860
actgaaggag aagctgcaga tggagagaga acacctactg agagagcaga ttatgatgtt 1920
ggagcacacg cagaaggtcc aaaatgattg gcttcatgaa ggatttaaga agaagtatga 1980
ggagatgaat gcagagataa gtcaatttaa acgtatgatt gatactacaa aaaatgatga 2040
tactccctgg attgcacgaa ccttggacaa ccttgccgat gagctaactg caatattgtc 2100
tgctcctgct aaattaattg gtcatggtgt caaaggtgtg agctcactct ttaaaaagca 2160
taagctcccc ttttaaggat attatagatt gtacatatat gctttggact atttttgatc 2220
tgtatgtttt tcattttcat tcagcaagtt tttttttttt ttcagagtct tactctgttg 2280
cccaggctgg agtacagtgg tgcaatctca gctcactgca acctctgcct cctgggttca 2340
agagattcac ctgcctcagc cccctagtag ctgggattat aggtgtacac caccacaccc 2400
agctaatttt tgtattttta gtagagatgg ggtttcacta tgttggccag gctggtctcg 2460
aactcttgac ctcaaatgat ccacccgcct cggcctccca aagtgctggg tttacaggca 2520
tgagccacca tgcccagccc tcatttagca aagttttaaa cataaaaagt gcttattaga 2580
ggatatcagt gcctggccca catgagagaa cagatccata cacactttga aaactttgt 2640
tcacttttag gaaatataat tttgaaaaat catttacata caagaggtcc actgaggcat 2700
tgcttttaat ggcaaaatat tgcaatgtac ttgaatgtcc ttcacattag attggtaaga 2760

taaattttag tatgtgcatg tactggaata ttatatagcc agtaaacaaa ttgacaatga 2820
agctctattt gtaccagtaa agaatggtct tgaagagaca ttgtaaaatg aaaaaaaaaa 2880
aaaccaagtt gtaaagcaat gtagattatc ttatcagcat ggaaaaaatg caattattat 2940
atggaagcat gcaaatatat ctctggaaag attaatcaaa atctattatt attggctcct 3000
tctgggaaga accaagtgaa tgtaggggtt gaaggaacac atactcttca ctttatactc 3060
ttgaattttg taaagaaatc ttattttacc gattcagaaa taaataagaa aatgtaagag 3120
acaatgctaa taatgataac aataaaaaaa tagtgaaatt ggctatcaga tgatgaaatt 3180
ccttgttgct tctaggaatt acatcagcca gcccaatgag ggtctaaga ctaagatctg 3240
agtactagaa tgcaaaaaat ttgcatcata ttcttgtttt cattttggcc tggtttttcg 3300
atcccttcta tttgtttcga gactaaggca tacacttttc tgtggccttt gaaatctcag 3360
ctacagtagc ctctacttcc tttctctgaa ttcaagtttt aaggtgttag aagcataaat 3420
taccaaacat taagattaag cactgatagg aaggatcctg ccaaccttgt cagtagtggt 3480
ctttttgcgg tagcctcaga tagaaaggac aatgtgcctt tccagaactg aaccccctga 3540
tgggggatga tagacccaga cacatagagg cctggtgttg acccttaatt gttagaatct 3600
aagaagattc agttatagta atgagtggca gactgcaagg cctgatggag ataattctga 3660
agatggtcaa tacaatggac cagccatagg gttaaatggg atggggagaa aacaatcaag 3720
tgaagtaaat gctgtctatc aggatgttga gaggtagccc tgataaagtg tcacatccat 3780
agccttgatc tgagctaatt tcagaccctg aactcagttg gagtggaggg cagtacccca 3840
ggaaaggaca ctgcaactcc aggacccaaa taaatgataa tgatttccca agttctcctc 3900
ctaggaaacg tctggagcca tttactagat atccatatac tagggaaata caaacaccca 3960
aatatttcaa ggacccttgg aatcaggatc caagttgccc atggtaccta gatcatggag 4020
agtcatcgta aaatgcctgt taaaataggg aattagggcc tccaggttgt aattatagtc 4080
ttggcacaca tgaacttatt gcaatatagt atttgtttga aatgtaaagg taaagtttgt 4140
ttgaagacag ataccttatt tttctgtaca tctccttggg agtatgttca ggcataggtt 4200
gtgctaaata agcttctttt ataaaaacaa taggctgatg catgggtgat tattttcaat 4260
tgataaaata tcatgaactt tccagagatc agtggtaata agttgtttga attttcgaca 4320
ccagattaag gtctctgagg tggtttaaag gctatgaggc caggtgcagt ggctcacgcc 4380
tataatctca gcaacttggg aggccgaagc aggcagatca cttgaggtca gaagtttgag 4440
accagcctgg ccaacatggc gaaaccctgc ctttactaaa aatacaaaaa ttaaccaagt 4500
gtggtggcac acgcctgtca tgccagctac acaggaggat gaggcaggaa aatcacttga 4560
acctgggagg tggaggttgc agtgagccga gattgtgcca ttgcactcca gcccgggcaa 4620
cagagcctgg gcgacagagt gaggctctgt ctcaaaaaat taaactaaat taaagctatg 4680

agaagaaaat aaattttcct ggtatagtgt ccttctctag acctcatgtc ctacagtctc 4740

tgtgaaactg ggccaaagat agaatttttt tttctgtgct ttatacagtg actcagacaa 4800

acctatgacc tcacacaaat ctgtaacttc atgtatcaca gtctctataa aactatatca 4860

aagaaag 4867

<210> 5
<211> 4526
<212> DNA
<213> Artificial Sequence

<220>
<223> Human GBP6 transcript variant 2 mRNA sequence

<400> 5

acccaacacc catcctcaaa ctcacatcgg atgattcagg catggctctg ctaacacttt 60
attaaaagca tggattaatt ttacttccaa gtttattttt actgcaccat cccatttgtg 120
gaaacaacta gcttactcag cttttttttc cttttataaa ggaaagaaca gaaaagtaaa 180
aggaggaaag aaaacaagag gtgagtgagg caactgaaaa ctgttcttgg acctgcggtg 240
ctatagagca gggtgaccct aagaatgact cctggatctt tgccctggct gtgctcctgt 300
gcagcacctt tgtctacaac agcatgagca ccatcaacca ccaggccctg gagcagctgc 360
attatgtgac ggagctcaca gaactaatta aggcaaagtc ctccccaagg cctgatggag 420
tagaagattc cacagagttt gtgagtttct tcccagactt tctttggaca gtacgggatt 480
tcactctgga gctgaagttg aacggtcacc ctatcacaga agatgaatac ctggagaatg 540
ccttgaagct gattcaaggc aataatccca gagttcaaac atccaatttt cccagggagt 600
gcatcaggcg tttctttcca aaacggaagt gtttcgtctt tgaccggcca acaaatgaca 660
aagaccttct agccaatatt gagaaggtgt cagaaaagca actggatccc aaattccagg 720
aacaaacaaa cattttctgt tcttacatct tcactcatgc aagaaccaag accctcaggg 780
agggaatcac agtcactggg aatcgtctgg gaactctggc agtgacttat gtagaggcca 840
tcaacagtgg agcagtgcct tgtctggaga atgcagtgat aactctggcc cagcgtgaga 900
actcagcggc cgtgcagagg gcagctgact actacagcca gcagatggcc cagcgagtga 960
agctccccac agacacgctc caggagctgc tggacatgca tgcggcctgt gagagggaag 1020
ccattgcaat cttcatggag cactccttca aggatgaaaa tcaggaattc cagaagaagt 1080
tcatggaaac cacaatgaat aagaaggggg atttcttgct gcagaatgaa gagtcatctg 1140
ttcaatactg ccaggctaaa ctcaatgagc tctcaaaggg actaatggaa agtatctcag 1200
caggaagttt ctctgttcct ggagggcaca agctctacat ggaaacaaag gaaaggattg 1260
aacaggacta ttggcaagtt cccaggaaag gagtaaaggc aaaagaggtc ttccagaggt 1320

tcctggagtc acagatggtg atagaggaat ccatcttgca gtcagataaa gccctcactg 1380
atagagagaa ggcagtagca gtggatcggg ccaagaagga ggcagctgag aaggaacagg 1440
aacttttaaa acagaaatta caggagcagc agcaacagat ggaggctcaa gataagagtc 1500
gcaaggaaaa catagcccaa ctgaaggaga agctgcagat ggagagagaa cacctactga 1560
gagagcagat tatgatgttg gagcacacgc agaaggtcca aaatgattgg cttcatgaag 1620
gatttaagaa gaagtatgag gagatgaatg cagagataag tcaatttaaa cgtatgattg 1680
atactacaaa aaatgatgat actccctgga ttgcacgaac cttggacaac cttgccgatg 1740
agctaactgc aatattgtct gctcctgcta aattaattgg tcatggtgtc aaaggtgtga 1800
gctcactctt taaaaagcat aagctcccct tttaaggata ttatagattg tacatatatg 1860
ctttggacta tttttgatct gtatgttttt cattttcatt cagcaagttt tttttttttt 1920
tcagagtctt actctgttgc ccaggctgga gtacagtggt gcaatctcag ctcactgcaa 1980
cctctgcctc ctgggttcaa gagattcacc tgcctcagcc ccctagtagc tgggattata 2040
ggtgtacacc accacaccca gctaattttt gtatttttag tagagatggg gtttcactat 2100
gttggccagg ctggtctcga actcttgacc tcaaatgatc cacccgcctc ggcctcccaa 2160
agtgctgggt ttacaggcat gagccaccat gcccagccct catttagcaa agttttaaac 2220
ataaaaagtg cttattagag gatatcagtg cctggcccac atgagagaac agatccatac 2280
acactttgaa aaactttgtt cacttttagg aaatataatt ttgaaaaatc atttacatac 2340
aagaggtcca ctgaggcatt gcttttaatg gcaaaatatt gcaatgtact tgaatgtcct 2400
tcacattaga ttggtaagat aaattttagt atgtgcatgt actggaatat tatatagcca 2460
gtaaacaaat tgacaatgaa gctctatttg taccagtaaa gaatggtctt gaagagacat 2520
tgtaaaatga aaaaaaaaaa aaccaagttg taaagcaatg tagattatct tatcagcatg 2580
gaaaaaatgc aattattata tggaagcatg caaatatatc tctggaaaga ttaatcaaaa 2640
tctattatta ttggctcctt ctgggaagaa ccaagtgaat gtaggggttg aaggaacaca 2700
tactcttcac tttatactct tgaattttgt aaagaaatct tattttaccg attcagaaat 2760
aaataagaaa atgtaagaga caatgctaat aatgataaca ataaaaaaat agtgaaattg 2820
gctatcagat gatgaaattc cttgttgctt ctaggaatta catcagccag cccaatgagg 2880
ggtctaagac taagatctga gtactagaat gcaaaaaatt tgcatcatat tcttgttttc 2940
attttggcct ggtttttcga tcccttctat ttgtttcgag actaaggcat acacttttct 3000
gtggcctttg aaatctcagc tacagtagcc tctacttcct ttctctgaat tcaagtttta 3060
aggtgttaga agcataaatt accaaacatt aagattaagc actgatagga aggatcctgc 3120
caaccttgtc agtagtggtc tttttgcggt agcctcagat agaaaggaca atgtgccttt 3180
ccagaactga accccctgat gggggatgat agacccagac acatagaggc ctggtgttga 3240

cccttaattg ttagaatcta agaagattca gttatagtaa tgagtggcag actgcaaggc 3300

ctgatggaga taattctgaa gatggtcaat acaatggacc agccataggg ttaaatggga 3360

tggggagaaa acaatcaagt gaagtaaatg ctgtctatca ggatgttgag aggtagccct 3420

gataaagtgt cacatccata gccttgatct gagctaattt cagaccctga actcagttgg 3480

agtggagggc agtaccccag gaaaggacac tgcaactcca ggacccaaat aaatgataat 3540

gatttcccaa gttctcctcc taggaaacgt ctggagccat ttactagata tccatatact 3600

agggaaatac aaacacccaa atatttcaag gacccttgga atcaggatcc aagttgccca 3660

tggtacctag atcatggaga gtcatcgtaa aatgcctgtt aaaataggga attagggcct 3720

ccaggttgta attatagtct tggcacacat gaacttattg caatatagta tttgtttgaa 3780

atgtaaaggt aaagtttgtt tgaagacaga taccttattt ttctgtacat ctccttggga 3840

gtatgttcag gcataggttg tgctaaataa gcttctttta taaaaacaat aggctgatgc 3900

atgggtgatt attttcaatt gataaaatat catgaacttt ccagagatca gtggtaataa 3960

gttgtttgaa ttttcgacac cagattaagg tctctgaggt ggtttaaagg ctatgaggcc 4020

aggtgcagtg gctcacgcct ataatctcag caacttggga ggccgaagca ggcagatcac 4080

ttgaggtcag aagtttgaga ccagcctggc caacatggcg aaaccctgcc tttactaaaa 4140

atacaaaaat taaccaagtg tggtggcaca cgcctgtcat gccagctaca caggaggatg 4200

aggcaggaaa atcacttgaa cctgggaggt ggaggttgca gtgagccgag attgtgccat 4260

tgcactccag cccgggcaac agagcctggg cgacagagtg aggctctgtc tcaaaaaatt 4320

aaactaaatt aaagctatga gaagaaaata aattttcctg gtatagtgtc cttctctaga 4380

cctcatgtcc tacagtctct gtgaaactgg gccaaagata gaattttttt ttctgtgctt 4440

tatacagtga ctcagacaaa cctatgacct cacacaaatc tgtaacttca tgtatcacag 4500

tctctataaa actatatcaa agaaag 4526

<210> 6
<211> 5496
<212> DNA
<213> Artificial Sequence

<220>
<223> Human TMCC1 transcript variant 3 mRNA sequence

<400> 6

ggcggccgca gtggaaggag caggcgcttg agctcgagcg acggcgctgg cggagacgcc 60

ggctgctcct cccctccccg ccgcttttcc taaaaggatt gtacacctta gaagtgctta 120

aggaagagtg atgaagctct gaatcgtgtc ctgcagcaga ttcgagtgcc acccaagatg 180

aagagaggga caagcttgca tagtaggcgg ggcaagccag aggccccaaa gggaagtccc 240

caaatcaaca ggaagtctgg tcaggagatg acagctgtta tgcagtcagg ccgacccagg 300
tcttcatcca caactgatgc acctaccagc tctgctatga tggaaatagc ttgtgctgct 360
gctgctgctg ctgctgcatg tctaccagga gaggagggaa ctgcggagcg gatcgaacgg 420
ttggaagtaa gcagccttgc ccaaacatcc agtgcagtgg cctccagtac cgatggcagc 480
atccacacag actctgtgga tggaacacca gaccctcagc gcacaaaggc tgccattgct 540
cacctgcagc agaagatcct gaagctcaca gaacaaatca agattgcaca aacagcccgg 600
gacgacaacg ttgctgaata cttgaagctt gccaacagtg cagacaaaca gcaggctgcc 660
cgcatcaagc aagtctttga gaagaagaac cagaaatctg cccaaactat cctccagctg 720
caaaagaaac ttgagcacta ccacaggaag ctcagagagg tagagcagaa tgggatcccc 780
cggcagccaa aggatgtctt cagggacatg caccagggtc tgaaggatgt aggagcaaag 840
gtgactggct tcagtgaagg tgtggtggat agtgtcaaag gtgggttttc cagcttctcc 900
caggccaccc attcagcagc aggcgctgta gtctcaaagc ccagagagat tgcctcactc 960
attcggaaca aatttggcag tgcagacaac atccccaacc tgaaggactc tttagaggaa 1020
gggcaagtgg atgatgcggg gaaggctttg ggagtgattt caaactttca gtctagccca 1080
aaatatggta gtgaagaaga ttgttctagt gccacttcag gctcagtggg agccaacagc 1140
accacagggg gcatcgctgt aggagcatcc agctccaaaa caaacaccct ggacatgcag 1200
agctcaggat ttgatgcact actacatgag atccaggaga tccgggaaac ccaggccaga 1260
ctagaggaat cctttgagac tctcaaggaa cattatcaga gggactattc cttaataatg 1320
cagaccttac aggaggagcg atatagatgt gaacgattgg aagaacagct aaatgaccta 1380
acagagctcc accagaatga aatcttgaac ttgaagcagg aactggcaag catggaagaa 1440
aaaatcgcgt atcagtccta tgaacgggcc cgggacatcc aggaggccct ggaggcatgc 1500
cagacgcgca tctccaagat ggagctgcag cagcagcagc agcaggtggt gcagctagaa 1560
gggctggaga atgccactgc ccggaacctt ctgggcaaac tcatcaacat cctcctggct 1620
gtcatggcag tccttttggt ctttgtctcc actgtagcca actgtgtggt ccccctcatg 1680
aagactcgca acaggacgtt cagcacttta ttccttgtgg tttttattgc ctttctctgg 1740
aagcactggg acgccctctt cagctatgtg gaacggttct tttcatcccc tagatgatgc 1800
tggcacagaa ggcattgttc cctaccctct ggcgagtgca tgcagcagag agttagacag 1860
caacttacct actctgaagt tttctacaac aaaaaaagag ttgagtgaat ctgtttacat 1920
ttagaataat gtttttttct tcaagagacg caattgcaat agtatttttt agattttatc 1980
caagaagttt tttgggcgaa aatcttggat catttttatg tagcatgatt ttccttggga 2040
tgcaaatctt aaaacagtcc tttaatatga accaacaatc tggagcacac cgaagggcaa 2100
tctaaattgt ggcttgaagg actgcactaa aacccactaa aaagatgcga aaacctgatg 2160

agggcaaacc agttaaacct aacaccctgc cttgtctggg ctcatcacct ctccctatcc 2220
cagactaact ttactgtgaa atcctaccac attccatgtc tgaatttttg gattcggggt 2280
ggattttcgt tgtccgtgga agaacacatg gatctctctg gctttctcac ccaagttggc 2340
cacttacgct aatcctggaa gtatgatcac ttttgaacct gccccttaac cttgacgagg 2400
atacaaaagt gaaagcatca tcccccaaag gatcactgca cagtcctact acagtatttt 2460
taagtagccc tctaaatact taattttaag caaaatccct tggccgcact tttaaggttt 2520
ttttatatgt gtatagttac caacctaaaa ataaaaaatc cgaacagcat acttgaagaa 2580
tgtaatactc aaactctcag tgcttcctta tggtttctaa taggattttt tattattgtt 2640
attattatta ttgggttttt ttggacaggg ttgggagggt cttttatttt tcctttgaaa 2700
taaagaagtg atgtttttaa atgaagaaat gtgtggatat ttaagtgtgc tgctccctct 2760
tgtcttgaaa cagtttgagt aagaaagtct tgctgtaaat gctgccctct gccgcctttg 2820
ttttgagatg cagtttaaac tccctctggc tgctgctgct gctttttggt gtcccgacat 2880
acctacgccc ccgttttatg ggtttggctt agttgaagag gaaagggttg tgcaaggaga 2940
gcaggaggct gtttccaaaa accagtgtag taggataggg attttttttt tttttttttg 3000
ccccaagaaa acgttcaccc agtgatcttg ggctggggtt gtctttagga aaagttgaga 3060
ctataagagt cataaataag tccttgtgtt tccttaattt attttgttaa cacccctaat 3120
tacaaccaaa gtgatgatgt ggagtcttct gtcttcattt tggccccagc attcttaatt 3180
tcaaagcttt attctgtctg cctaagagaa tcaaccaaag gtgattctcc taaagagcag 3240
tgaaggaaat gtcaggttag caggacccaa gttttgggtg tgaaatgttg ccagcttcct 3300
ataatgtaaa cggacttgtt aacctaacct aattatgctc agtggacttc tatagatggt 3360
tttgaaaaat gaactgagct gccttccccc gtcgcataac cagttccatc atcctggtgg 3420
aacttgaaca tttagagttt atctagagag cttggttaat ctttccatat tatttgtagt 3480
attggtcaca aatgctgttc cctcttagcc tcattctgtg caaccaagtg catataagat 3540
gccctgaaaa gagtaacaaa gtatgctttg cctgtttcca cttaccagga aattccttca 3600
gaactagatt agcattgccc tgcctgtctg aaaggacagt ttacctaatg gtgccagcct 3660
ccttttgctt tggcaagctg gatttctcag agccagcatg ttgtttccat aactactttg 3720
atattttaac tcaggtactc cagtcttcac cccaacctca gctgattgta gtacacctgc 3780
tagctctgtt gccccctcaa aactgcaccc agagcagggc cacaagggtg cttttttttct 3840
ttaaaaaaaa aaaaattaga accaattcat gttcatgcca aaaacaaatt gtccccaagc 3900
ctatatgtat taaaatgtta actttgccta aaaatattgc agtgactttt taggcaggag 3960
tgccaaagga cactatgaac tttttgaact gacagtttct cctaactttc tgctttagcg 4020

```
taattgctca gagtagagag cccccacaaa gttatttaaa agatgcccta gcagcaatcc      4080
accagttttt ctaagctaga acctttgagt cccccaaact gcctgaagac ttaagttttg      4140
tgggcactgg aagtcacttt gatagatgga ttgaaactgt tcctatttgc cctgggacgg      4200
tttctatcta tcaaaggaag gttttcacct gtagaaagcc ccctgcctcc agccaaatag      4260
tcccatgctg actttctatc ttcctttctc aaactgtctt aggaaggacc ttcagtgcag      4320
atcaggtgca gtaatggctt tcttgtccct taattattca ccagacccag aagttgtacg      4380
catttaatgc tgtttgtaac catgcatctg ttttcattct ttgctgtacc ttttgctgcc      4440
catcctgtta cttttgagtt tctttcattg tggttgttct tgggttcttt tgtcttgtca      4500
gagctcttct ataacctcgc tctaatggct taacagttgt tctgggtgga aacgtcccct      4560
catttgaatg ctcctctaaa aaaaaaagaa aagaaaactg tattcattcc ctttaaaatg      4620
aaacattcct ggtttatttg tccatgcctc tagcctgggt gagtgaagct ggctgtgttg      4680
gcctgggtga ttgttcaggt tgtagaatgc gcattttaca ttgtttatga taattgaagg      4740
gtacttctgt ctgctccaat ttccattcct tggtatactc agtatgtcct agtaaggaag      4800
gctttccact ctactggctc cttaagaagc aaaagtaggt taaattttat acttcactga      4860
ctagggtctt ctctctcccc tgttctgaat ggagtaaaag tctgatgcca agacaaatat      4920
tgggagcgag ccttcctcac tagccatgtc caaataatga gcgtatttc atgtggtctt       4980
cactgcattt ggttttgttt ctgatttcat gttcctttga ggtacagtca gatgaaaatg      5040
ctgagttctg agagagttcc aatgaggagc tgcctttcag ctttggaaaa tatgcaacta      5100
aaacaaaacc aaacattatt gtaatctgac acaggcaaaa ttatggttcc cacaccccaa      5160
ccccaaatga aacctgggat tttgaatgtg gctctaagag ttaacattgc tgtctgtatg      5220
tcgtgtatgc taggtgatat cctcagtagg gattgactac tagactgtgt gttttatcaa      5280
agtgtgtaag aataaaaact cacttgcacg aattgaaacg taaagaaatg acacttgtga      5340
acgtgtgaac gttaacactg tagttgatag atcttaagct gctaattgtt gagagagatt      5400
taaatttatg ttctgtctgg tcgctgcaga ttctcagcag cacttttact gtatatagaa      5460
attgaaataa agacctcagc tattaaaaaa aaaaaa                                5496
```

<210> 7
<211> 6095
<212> DNA
<213> Artificial Sequence

<220>
<223> Human TMCC1 transcript variant 1 mRNA sequence

<400> 7

gtattaatct ctggagaaga cacatccaca gttagcactt tcttcagatg ctgacgctcg 60

gtgaacagtt gcctttggtc acaagattta gaagacacag tgtccatcct cccagattgg 120

```
atctcttttt catatggatc ttctgtttct atgtcttttt aaaaaataac tttttgggaa     180
accttttgga ttacaactgt tcatcctcac ctatgcaaag aaagggaagc tattgctggg     240
attttgagga gcttttccta aaaggattgt acaccttaga agtgcttaag gaagagtgat     300
gaagataggc atgaagcctt cgtctcacag ctgcatgcgt agtcactgtt gaagcaaatg     360
cctacctaat ttgacactct tggtgtgttt aaaaaatttt tttgagtttg caaataagca     420
tattaagtct actgatggag ccttcgggca gtgaacagtt atttgaggac cctgatcctg     480
gaggcaaatc ccaagatgca gaggccagaa agcagacaga atcagaacaa aaattgtcta     540
aaatgaccca caatgctttg gagaacatta acgtgattgg ccaaggcttg aagcatctct     600
tccagcacca gcgcaggagg tcatcagtgt ctccacatga tgtgcagcaa attcaggcag     660
atccagaacc tgaaatggat ctggaaagcc agaacgcatg tgctgagatt gatggtgtcc     720
ccacccaccc cacagctctg aatcgtgtcc tgcagcagat tcgagtgcca cccaagatga     780
agagagggac aagcttgcat agtaggcggg gcaagccaga ggccccaaag ggaagtcccc     840
aaatcaacag gaagtctggt caggagatga cagctgttat gcagtcaggc cgacccaggt     900
cttcatccac aactgatgca cctaccagct ctgctatgat ggaaatagct tgtgctgctg     960
ctgctgctgc tgctgcatgt ctaccaggag aggagggaac tgcggagcgg atcgaacggt    1020
tggaagtaag cagccttgcc caaacatcca gtgcagtggc ctccagtacc gatggcagca    1080
tccacacaga ctctgtggat ggaacaccag accctcagcg cacaaaggct gccattgctc    1140
acctgcagca gaagatcctg aagctcacag aacaaatcaa gattgcacaa acagcccggg    1200
acgacaacgt tgctgaatac ttgaagcttg ccaacagtgc agacaaacag caggctgccc    1260
gcatcaagca agtctttgag aagaagaacc agaaatctgc ccaaactatc ctccagctgc    1320
aaaagaaact tgagcactac cacaggaagc tcagagaggt agagcagaat gggatccccc    1380
ggcagccaaa ggatgtcttc agggacatgc accagggtct gaaggatgta ggagcaaagg    1440
tgactggctt cagtgaaggt gtggtggata gtgtcaaagg tgggttttcc agcttctccc    1500
aggccaccca ttcagcagca ggcgctgtag tctcaaagcc cagagagatt gcctcactca    1560
ttcggaacaa atttggcagt gcagacaaca tccccaacct gaaggactct ttagaggaag    1620
ggcaagtgga tgatgcgggg aaggctttgg gagtgatttc aaactttcag tctagcccaa    1680
aatatggtag tgaagaagat tgttctagtg ccacttcagg ctcagtggga gccaacagca    1740
ccacaggggg catcgctgta ggagcatcca gctccaaaac aaacaccctg gacatgcaga    1800
gctcaggatt tgatgcacta ctacatgaga tccaggagat ccgggaaacc caggccagac    1860
tagaggaatc ctttgagact ctcaaggaac attatcagag ggactattcc ttaataatgc    1920
agaccttaca ggaggagcga tatagatgtg aacgattgga agaacagcta aatgacctaa    1980
```

cagagctcca ccagaatgaa atcttgaact tgaagcagga actggcaagc atggaagaaa 2040
aaatcgcgta tcagtcctat gaacgggccc gggacatcca ggaggccctg gaggcatgcc 2100
agacgcgcat ctccaagatg gagctgcagc agcagcagca gcaggtggtg cagctagaag 2160
ggctggagaa tgccactgcc cggaaccttc tgggcaaact catcaacatc ctcctggctg 2220
tcatggcagt ccttttggtc tttgtctcca ctgtagccaa ctgtgtggtc cccctcatga 2280
agactcgcaa caggacgttc agcactttat tccttgtggt tttattgcc tttctctgga 2340
agcactggga cgccctcttc agctatgtgg aacggttctt ttcatcccct agatgatgct 2400
ggcacagaag gcattgttcc ctaccctctg gcgagtgcat gcagcagaga gttagacagc 2460
aacttaccta ctctgaagtt ttctacaaca aaaaaagagt tgagtgaatc tgtttacatt 2520
tagaataatg tttttttctt caagagacgc aattgcaata gtatttttta gattttatcc 2580
aagaagtttt ttgggcgaaa atcttggatc atttttatgt agcatgattt tccttgggat 2640
gcaaatctta aaacagtcct ttaatatgaa ccaacaatct ggagcacacc gaagggcaat 2700
ctaaattgtg gcttgaagga ctgcactaaa acccactaaa aagatgcgaa aacctgatga 2760
gggcaaacca gttaaaccta acaccctgcc ttgtctgggc tcatcacctc tccctatccc 2820
agactaactt tactgtgaaa tcctaccaca ttccatgtct gaatttttgg attcggggtg 2880
gattttcgtt gtccgtggaa gaacacatgg atctctctgg ctttctcacc caagttggcc 2940
acttacgcta atcctggaag tatgatcact tttgaacctg ccccttaacc ttgacgagga 3000
tacaaaagtg aaagcatcat cccccaaagg atcactgcac agtcctacta cagtattttt 3060
aagtagccct ctaaatactt aattttaagc aaaatccctt ggccgcactt ttaaggtttt 3120
tttatatgtg tatagttacc aacctaaaaa taaaaaatcc gaacagcata cttgaagaat 3180
gtaatactca aactctcagt gcttccttat ggtttctaat aggatttttt attattgtta 3240
ttattattat tgggtttttt tggacagggt tgggagggtc ttttattttt cctttgaaat 3300
aaagaagtga tgtttttaaa tgaagaaatg tgtggatatt taagtgtgct gctccctctt 3360
gtcttgaaac agtttgagta agaaagtctt gctgtaaatg ctgccctctg ccgcctttgt 3420
tttgagatgc agtttaaact ccctctggct gctgctgctg ctttttggtg tcccgacata 3480
cctacgcccc cgttttatgg gtttggctta gttgaagagg aaagggttgt gcaaggagag 3540
caggaggctg tttccaaaaa ccagtgtagt aggataggga tttttttttt ttttttttgc 3600
cccaagaaaa cgttcaccca gtgatcttgg gctggggttg tctttaggaa aagttgagac 3660
tataagagtc ataaataagt ccttgtgttt ccttaattta ttttgttaac accccctaatt 3720
acaaccaaag tgatgatgtg gagtcttctg tcttcatttt ggccccagca ttcttaattt 3780
caaagcttta ttctgtctgc ctaagagaat caaccaaagg tgattctcct aaagagcagt 3840
gaaggaaatg tcaggttagc aggacccaag ttttgggtgt gaaatgttgc cagcttccta 3900

```
taatgtaaac ggacttgtta acctaaccta attatgctca gtggacttct atagatggtt     3960
ttgaaaaatg aactgagctg ccttcccccg tcgcataacc agttccatca tcctggtgga     4020
acttgaacat ttagagttta tctagagagc ttggttaatc tttccatatt atttgtagta     4080
ttggtcacaa atgctgttcc ctcttagcct cattctgtgc aaccaagtgc atataagatg     4140
ccctgaaaag agtaacaaag tatgctttgc ctgtttccac ttaccaggaa attccttcag     4200
aactagatta gcattgccct gcctgtctga aaggacagtt tacctaatgg tgccagcctc     4260
cttttgcttt ggcaagctgg atttctcaga gccagcatgt tgtttccata actactttga     4320
tattttaact caggtactcc agtcttcacc ccaacctcag ctgattgtag tacacctgct     4380
agctctgttg ccccctcaaa actgcaccca gagcagggcc acaagggtgc tttttttctt     4440
taaaaaaaaa aaaattagaa ccaattcatg ttcatgccaa aaacaaattg tccccaagcc     4500
tatatgtatt aaaatgttaa ctttgcctaa aaatattgca gtgacttttt aggcaggagt     4560
gccaaaggac actatgaact ttttgaactg acagtttctc ctaactttct gctttagcgt     4620
aattgctcag agtagagagc ccccacaaag ttatttaaaa gatgccctag cagcaatcca     4680
ccagtttttc taagctagaa cctttgagtc ccccaaactg cctgaagact taagttttgt     4740
gggcactgga agtcactttg atagatggat tgaaactgtt cctatttgcc ctgggacggt     4800
ttctatctat caaaggaagg ttttcacctg tagaaagccc cctgcctcca gccaaatagt     4860
cccatgctga ctttctatct tcctttctca aactgtctta ggaaggacct tcagtgcaga     4920
tcaggtgcag taatggcttt cttgtccctt aattattcac cagacccaga agttgtacgc     4980
atttaatgct gtttgtaacc atgcatctgt tttcattctt tgctgtacct tttgctgccc     5040
atcctgttac ttttgagttt ctttcattgt ggttgttctt gggttctttt gtcttgtcag     5100
agctcttcta taacctcgct ctaatggctt aacagttgtt ctgggtggaa acgtcccctc     5160
atttgaatgc tcctctaaaa aaaaaagaaa agaaaactgt attcattccc tttaaaatga     5220
aacattcctg gtttatttgt ccatgcctct agcctgggtg agtgaagctg gctgtgttgg     5280
cctgggtgat tgttcaggtt gtagaatgcg cattttacat tgtttatgat aattgaaggg     5340
tacttctgtc tgctccaatt tccattcctt ggtatactca gtatgtccta gtaaggaagg     5400
ctttccactc tactggctcc ttaagaagca aaagtaggtt aaattttata cttcactgac     5460
tagggtcttc tctctcccct gttctgaatg gagtaaaagt ctgatgccaa gacaaatatt     5520
gggagcgagc cttcctcact agccatgtcc aaataatgag cgtattttca tgtggtcttc     5580
actgcatttg gttttgtttc tgatttcatg ttcctttgag gtacagtcag atgaaaatgc     5640
tgagttctga gagagttcca atgaggagct gcctttcagc tttggaaaat atgcaactaa     5700
aacaaaacca aacattattg taatctgaca caggcaaaat tatggttccc acaccccaac     5760
```

cccaaatgaa acctgggatt ttgaatgtgg ctctaagagt taacattgct gtctgtatgt 5820

cgtgtatgct aggtgatatc ctcagtaggg attgactact agactgtgtg ttttatcaaa 5880

gtgtgtaaga ataaaaactc acttgcacga attgaaacgt aaagaaatga cacttgtgaa 5940

cgtgtgaacg ttaacactgt agttgataga tcttaagctg ctaattgttg agagagattt 6000

aaatttatgt tctgtctggt cgctgcagat tctcagcagc acttttactg tatatagaaa 6060

ttgaaataaa gacctcagct attaaaaaaa aaaaa 6095

<210> 8
<211> 4753
<212> DNA
<213> Artificial Sequence

<220>
<223> Human PRDM1 transcript variant 2 mRNA sequence

<400> 8

```
agtttgacgt cgtcagccgg cttggtcttc tacccagtga ctcaaagcac taaaagtcag      60
cataatcgga actgaagtca gtagcatcgc ccatttgcca ttcactgcag tagcaaaagt     120
agtactctgt ggtgggttaa tcggtttgag gcagctcctt aaatgaacat ttgtgtttca     180
tttttctgtt attttcccga acatgaaaag acgataaaac tgaaatggaa aagatctatt     240
ccagagggga gcttcaccac ttcattgacg gctttaatga agagaaaagc aactggatgc     300
gctatgtgaa tccagcacac tctccccggg agcaaaacct ggctgcgtgt cagaacggga     360
tgaacatcta cttctacacc attaagccca tccctgccaa ccaggaactt cttgtgtggt     420
attgtcggga ctttgcagaa aggcttcact acccttatcc cggagagctg acaatgatga     480
atctcacaca aacacagagc agtctaaagc aaccgagcac tgagaaaaat gaactctgcc     540
caaagaatgt cccaaagaga gagtacagcg tgaaagaaat cctaaaattg gactccaacc     600
cctccaaagg aaaggacctc taccgttcta acatttcacc cctcacatca gaaaaggacc     660
tcgatgactt tagaagacgt gggagccccg aaatgccctt ctaccctcgg gtcgtttacc     720
ccatccgggc ccctctgcca gaagactttt tgaaagcttc cctggcctac gggatcgaga     780
gacccacgta catcactcgc tcccccattc catcctccac cactccaagc ccctctgcaa     840
gaagcagccc cgaccaaagc ctcaagagct ccagccctca cagcagccct gggaatacgg     900
tgtcccctgt gggccccggc tctcaagagc accgggactc ctacgcttac ttgaacgcgt     960
cctacggcac ggaaggtttg ggctcctacc ctggctacgc acccctgccc cacctcccgc    1020
cagctttcat cccctcgtac aacgctcact accccaagtt cctcttgccc ccctacggca    1080
tgaattgtaa tggcctgagc gctgtgagca gcatgaatgg catcaacaac tttggcctct    1140
tcccgaggct gtgccctgtc tacagcaatc tcctcggtgg gggcagcctg ccccacccca    1200
tgctcaaccc cacttctctc ccgagctcgc tgccctcaga tggagcccgg aggttgctcc    1260
```

agccggagca tcccagggag gtgcttgtcc cggcgcccca cagtgccttc tcctttaccg 1320
gggccgccgc cagcatgaag gacaaggcct gtagccccac aagcgggtct cccacggcgg 1380
gaacagccgc cacggcagaa catgtggtgc agcccaaagc tacctcagca gcgatggcag 1440
cccccagcag cgacgaagcc atgaatctca ttaaaaacaa aagaaacatg accggctaca 1500
agacccttcc ctacccgctg aagaagcaga acggcaagat caagtacgaa tgcaacgttt 1560
gcgccaagac tttcggccag ctctccaatc tgaaggtcca cctgagagtg cacagtggag 1620
aacggccttt caaatgtcag acttgcaaca agggctttac tcagctcgcc cacctgcaga 1680
aacactacct ggtacacacg ggagaaaagc cacatgaatg ccaggtctgc cacaagagat 1740
ttagcagcac cagcaatctc aagacccacc tgcgactcca ttctggagag aaaccatacc 1800
aatgcaaggt gtgccctgcc aagttcaccc agtttgtgca cctgaaactg cacaagcgtc 1860
tgcacacccg ggagcggccc cacaagtgct cccagtgcca caagaactac atccatctct 1920
gtagcctcaa ggttcacctg aaagggaact gcgctgcggc cccggcgcct gggctgccct 1980
tggaagatct gacccgaatc aatgaagaaa tcgagaagtt tgacatcagt gacaatgctg 2040
accggctcga ggacgtggag gatgacatca gtgtgatctc tgtagtggag aaggaaattc 2100
tggccgtggt cagaaaagag aaagaagaaa ctggcctgaa agtgtctttg caaagaaaca 2160
tggggaatgg actcctctcc tcagggtgca gcctttatga gtcatcagat ctacccctca 2220
tgaagttgcc tcccagcaac ccactacctc tggtacctgt aaaggtcaaa caagaaacag 2280
ttgaaccaat ggatccttaa gattttcaga aaacacttat tttgtttctt aagttatgac 2340
ttggtgagtc agggtgcctg taggaagtgg cttgtacata atcccagctc tgcaaagctc 2400
tctcgacagc aaatggtttc ccctcacctc tggaattaaa gaaggaactc caaagttact 2460
gaaatctcag ggcatgaaca aggcaaaggc catatatata tatatatata tatctgtata 2520
catattatat atacttattt acacctgtgt ctatatattt gcccctgtgt attttgaata 2580
tttgtgtgga catgtttgca tagccttccc attactaaga ctattaccta gtcataatta 2640
ttttttcaat gataatcctt cataatttat tatacaattt atcattcaga aagcaataat 2700
taaaaaagtt tacaatgact ggaaagattc cttgtaattt gagtataaat gtatttttgt 2760
cttgtggcca ttctttgtag ataatttctg cacatctgta taagtaccta agatttagtt 2820
aaacaaatat atgacttcag tcaacctctc tctctaataa tggtttgaaa atgaggtttg 2880
ggtaattgcc aatgttggac agttgatgtg ttcattcctg ggatcctatc atttgaacag 2940
cattgtacat aacttggggg tatgtgtgca ggattaccca agaataactt aagtagaaga 3000
aacaagaaag ggaatcttgt atatttttgt tgatagttca tgtttttccc ccagccacaa 3060
ttttaccgga agggtgacag gaaggcttta ccaacctgtc tctccctcca aaagagcaga 3120

atcctcccac cgccctgccc tccccaccga gtcctgtggc cattcagagc ggccacatga 3180
cttttgcatc cattgtatta tcagaaaatg tgaagaagaa aaaaatgcca tgttttaaaa 3240
ccactgcgaa aatttcccca aagcataggt ggctttgtgt gtgtgcgatt tgggggcttg 3300
agtctgggtg gtgttttgtt gttggttttt gttgcttttt tttttttttt ttttttaatg 3360
tcaaaattgc acaaacatgg tgctctacca ggaaggattc gaggtagata ggctcaggcc 3420
acactttaaa aacaaacaca caaacaacaa aaaacgggta ttctagtcat cttggggtaa 3480
aagcgggtaa tgaacattcc tatccccaac acatcaattg tatttttttct gtaaaactca 3540
gattttcctc agtatttgtg tttttacatt ttatggttaa tttaatggaa gatgaaaggg 3600
cattgcaaag ttgttcaaca acagttacct cattgagtgt gtccagtagt gcaggaaatg 3660
atgtcttatc taatgatttg cttctctaga ggagaaaccg agtaaatgtg ctccagcaag 3720
atagactttg tgttattcta tcttttattc tgctaagccc aaagattaca tgttggtgtt 3780
caaagtgtag caaaaaatga tgtatattta taaatctatt tataccacta tatcatatgt 3840
atatatattt ataaccactt aaattgtgag ccaagccatg taaaagatct actttttcta 3900
agggcaaaaa aaaaaaaaaa aaaaaaagaa cactcctttc tgagactttg cttaatactt 3960
ggtgacctca caatcacgtc ggtatgattg ggcacccttg cctactgtaa gagaccctaa 4020
aaccttggtg cagtggtggg gaccacaaaa caaccaggga ggaagagata catcattttt 4080
tagtattaag gaccatctaa gacagctcta tttttttttt gccactttat gattatgtgg 4140
tcacacccaa gtcacagaaa taaaaaactg actttaccgc tgcaattttt ctgttttcct 4200
ccttactaaa tactgataca ttactccaat ctattttata attatatttg acattttgtt 4260
cacatcaact aatgttcacc tgtagaagag aacaaatttc gaataatcca gggaaaccca 4320
agagccttac tggtcttctg taacttccaa gactgacagc tttttatgta tcagtgtttg 4380
ataaacacag tccttaactg aaggtaaacc aaagcatcac gttgacatta gaccaaatac 4440
ttttgattcc caactactcg tttgttcttt ttctcctttt gtgctttccc atagtgagaa 4500
tttttataaa gacttcttgc ttctctcacc atccatcctt ctcttttctg cctcttacat 4560
gtgaatgttg agcccacaat caacagtggt tttatttttt cctctactca aagttaaaac 4620
tgaccaaagt tactggcttt ttactttgct agaacaacaa actatcttat gtttacatac 4680
tggtttacaa tgttatttat gtgcaaattg tcaaaatgta aattaaatat aaatgttcat 4740
gctttaccaa aat 4753

<210> 9
<211> 5165
<212> DNA
<213> Artificial Sequence

<220>
<223> Human PRDM1 transcript variant 1 mRNA sequence

<400> 9

```
gggaagccag acggttaaca cagacaaagt gctgccgtga cactcggccc tccagtgttg 60
cggagaggca agagcagcga ccgcggcacc tgtccgcccg gagctgggac gcgggcgccc 120
gggcggccgg acgaagcgag gagggaccgc cgaggtgcgc gtctgtgcgg ctcagcctgg 180
cgggggacgc ggggagaatg tggactgggt agagatgaac gagacttttc tcagatgttg 240
gatatttgct tggaaaaacg tgtgggtacg accttggctg cccccaagtg taactccagc 300
actgtgaggt ttcagggatt ggcagagggg accaagggga ccatgaaaat ggacatggag 360
gatgcggata tgactctgtg gacagaggct gagtttgaag agaagtgtac atacattgtg 420
aacgaccacc cctgggattc tggtgctgat ggcggtactt cggttcaggc ggaggcatcc 480
ttaccaagga atctgctttt caagtatgcc accaacagtg aagaggttat tggagtgatg 540
agtaaagaat acataccaaa gggcacacgt tttggacccc taataggtga aatctacacc 600
aatgacacag ttcctaagaa cgccaacagg aaatattttt ggaggatcta ttccagaggg 660
gagcttcacc acttcattga cggctttaat gaagagaaaa gcaactggat gcgctatgtg 720
aatccagcac actctccccg ggagcaaaac ctggctgcgt gtcagaacgg gatgaacatc 780
tacttctaca ccattaagcc catccctgcc aaccaggaac ttcttgtgtg gtattgtcgg 840
gactttgcag aaaggcttca ctacccttat cccggagagc tgacaatgat gaatctcaca 900
caaacacaga gcagtctaaa gcaaccgagc actgagaaaa atgaactctg cccaaagaat 960
gtcccaaaga gagagtacag cgtgaaagaa atcctaaaat tggactccaa cccctccaaa 1020
ggaaaggacc tctaccgttc taacatttca cccctcacat cagaaaagga cctcgatgac 1080
tttagaagac gtgggagccc cgaaatgccc ttctaccctc gggtcgttta ccccatccgg 1140
gcccctctgc cagaagactt tttgaaagct tccctggcct acgggatcga gagacccacg 1200
tacatcactc gctcccccat tccatcctcc accactccaa gcccctctgc aagaagcagc 1260
cccgaccaaa gcctcaagag ctccagccct cacagcagcc ctgggaatac ggtgtcccct 1320
gtgggccccg gctctcaaga gcaccgggac tcctacgctt acttgaacgc gtcctacggc 1380
acggaaggtt tgggctccta ccctggctac gcacccctgc ccccacctcc gccagctttc 1440
atcccctcgt acaacgctca ctaccccaag ttcctcttgc ccccctacgg catgaattgt 1500
aatggcctga gcgctgtgag cagcatgaat ggcatcaaca actttggcct cttcccgagg 1560
ctgtgccctg tctacagcaa tctcctcggt gggggcagcc tgccccaccc catgctcaac 1620
cccacttctc tcccgagctc gctgccctca gatggagccc ggaggttgct ccagccggag 1680
catcccaggg aggtgcttgt cccggcgccc cacagtgcct tctcctttac cggggccgcc 1740
gccagcatga aggacaaggc ctgtagcccc acaagcgggt ctcccacggc gggaacagcc 1800
```

gccacggcag aacatgtggt gcagcccaaa gctacctcag cagcgatggc agccccccagc 1860
agcgacgaag ccatgaatct cattaaaaac aaaagaaaca tgaccggcta caagaccctt 1920
ccctacccgc tgaagaagca gaacggcaag atcaagtacg aatgcaacgt ttgcgccaag 1980
actttcggcc agctctccaa tctgaaggtc cacctgagag tgcacagtgg agaacggcct 2040
ttcaaatgtc agacttgcaa caagggcttt actcagctcg cccacctgca gaaacactac 2100
ctggtacaca cgggagaaaa gccacatgaa tgccaggtct gccacaagag atttagcagc 2160
accagcaatc tcaagaccca cctgcgactc cattctggag agaaaccata ccaatgcaag 2220
gtgtgccctg ccaagttcac ccagtttgtg cacctgaaac tgcacaagcg tctgcacacc 2280
cgggagcggc cccacaagtg ctcccagtgc cacaagaact acatccatct ctgtagcctc 2340
aaggttcacc tgaaagggaa ctgcgctgcg gccccggcgc ctgggctgcc cttggaagat 2400
ctgacccgaa tcaatgaaga aatcgagaag tttgacatca gtgacaatgc tgaccggctc 2460
gaggacgtgg aggatgacat cagtgtgatc tctgtagtgg agaaggaaat tctggccgtg 2520
gtcagaaaag agaaagaaga aactggcctg aaagtgtctt tgcaaagaaa catggggaat 2580
ggactcctct cctcagggtg cagcctttat gagtcatcag atctacccct catgaagttg 2640
cctcccagca acccactacc tctggtacct gtaaaggtca aacaagaaac agttgaacca 2700
atggatcctt aagattttca gaaaacactt attttgtttc ttaagttatg acttggtgag 2760
tcagggtgcc tgtaggaagt ggcttgtaca taatcccagc tctgcaaagc tctctcgaca 2820
gcaaatggtt tcccctcacc tctggaatta aagaaggaac tccaaagtta ctgaaatctc 2880
agggcatgaa caaggcaaag gccatatata tatatatata tatatctgta tacatattat 2940
atatacttat ttacacctgt gtctatatat ttgcccctgt gtattttgaa tatttgtgtg 3000
gacatgtttg catagccttc ccattactaa gactattacc tagtcataat tatttttttca 3060
atgataatcc ttcataattt attatacaat ttatcattca gaaagcaata attaaaaaag 3120
tttacaatga ctggaaagat tccttgtaat ttgagtataa atgtattttt gtcttgtggc 3180
cattctttgt agataatttc tgcacatctg tataagtacc taagatttag ttaaacaaat 3240
atatgacttc agtcaacctc tctctctaat aatggtttga aaatgaggtt tgggtaattg 3300
ccaatgttgg acagttgatg tgttcattcc tgggatccta tcatttgaac agcattgtac 3360
ataacttggg ggtatgtgtg caggattacc caagaataac ttaagtagaa gaaacaagaa 3420
agggaatctt gtatattttt gttgatagtt catgtttttc ccccagccac aattttaccg 3480
gaagggtgac aggaaggctt taccaacctg tctctccctc caaaagagca gaatcctccc 3540
accgccctgc cctccccacc gagtcctgtg gccattcaga gcggccacat gacttttgca 3600
tccattgtat tatcagaaaa tgtgaagaag aaaaaaatgc catgttttaa aaccactgcg 3660
aaaatttccc caaagcatag gtggctttgt gtgtgtgcga tttgggggct tgagtctggg 3720

```
tggtgttttg ttgttggttt ttgttgcttt tttttttttt ttttttttaa tgtcaaaatt     3780
gcacaaacat ggtgctctac caggaaggat tcgaggtaga taggctcagg ccacacttta     3840
aaaacaaaca cacaaacaac aaaaaacggg tattctagtc atcttggggt aaaagcgggt     3900
aatgaacatt cctatcccca acacatcaat tgtatttttt ctgtaaaact cagattttcc     3960
tcagtatttg tgtttttaca ttttatggtt aatttaatgg aagatgaaag ggcattgcaa     4020
agttgttcaa caacagttac ctcattgagt gtgtccagta gtgcaggaaa tgatgtctta     4080
tctaatgatt tgcttctcta gaggagaaac cgagtaaatg tgctccagca agatagactt     4140
tgtgttattc tatcttttat tctgctaagc ccaaagatta catgttggtg ttcaaagtgt     4200
agcaaaaaat gatgtatatt tataaatcta tttataccac tatatcatat gtatatatat     4260
ttataaccac ttaaattgtg agccaagcca tgtaaaagat ctacttttc taagggcaaa     4320
aaaaaaaaaa aaaaaaaaag aacactcctt tctgagactt tgcttaatac ttggtgacct     4380
cacaatcacg tcggtatgat tgggcaccct tgcctactgt aagagaccct aaaaccttgg     4440
tgcagtggtg gggaccacaa aacaaccagg gaggaagaga tacatcattt tttagtatta     4500
aggaccatct aagacagctc tatttttttt ttgccacttt atgattatgt ggtcacaccc     4560
aagtcacaga aataaaaaac tgactttacc gctgcaattt ttctgttttc ctccttacta     4620
aatactgata cattactcca atctatttta taattatatt tgacattttg ttcacatcaa     4680
ctaatgttca cctgtagaag agaacaaatt tcgaataatc cagggaaacc caagagcctt     4740
actggtcttc tgtaacttcc aagactgaca gctttttatg tatcagtgtt tgataaacac     4800
agtccttaac tgaaggtaaa ccaaagcatc acgttgacat tagaccaaat acttttgatt     4860
cccaactact cgtttgttct ttttctcctt ttgtgctttc ccatagtgag aatttttata     4920
aagacttctt gcttctctca ccatccatcc ttctcttttc tgcctcttac atgtgaatgt     4980
tgagcccaca atcaacagtg gttttatttt ttcctctact caaagttaaa actgaccaaa     5040
gttactggct ttttactttg ctagaacaac aaactatctt atgtttacat actggtttac     5100
aatgttattt atgtgcaaat tgtcaaaatg taaattaaat ataaatgttc atgctttacc     5160
aaaat                                                                 5165
```

<210> 10
<211> 1475
<212> DNA
<213> Artificial Sequence

<220>
<223> Human ARG1 transcript variant 2 mRNA sequence

<400> 10

ggaaaaaaaa gatgcgccct ctgtcactga gggttgactg actggagagc tcaagtgcag 60

caaagagaag tgtcagagca tgagcgccaa gtccagaacc atagggatta ttggagctcc 120
tttctcaaag ggacagccac gaggaggggt ggaagaaggc cctacagtat tgagaaaggc 180
tggtctgctt gagaaactta aagaacaaga gtgtgatgtg aaggattatg gggacctgcc 240
ctttgctgac atccctaatg acagtccctt tcaaattgtg aagaatccaa ggtctgtggg 300
aaaagcaagc gagcagctgg ctggcaaggt ggcagaagtc aagaagaacg gaagaatcag 360
cctggtgctg ggcggagacc acagtttggc aattggaagc atctctggcc atgccagggt 420
ccaccctgat cttggagtca tctgggtgga tgctcacact gatatcaaca ctccactgac 480
aaccacaagt ggaaacttgc atggacaacc tgtatctttc ctcctgaagg aactaaaagg 540
aaagattccc gatgtgccag gattctcctg ggtgactccc tgtatatctg ccaaggatat 600
tgtgtatatt ggcttgagag acgtggaccc tggggaacac tacattttga aaactctagg 660
cattaaatac ttttcaatga ctgaagtgga cagactagga attggcaagg tgatggaaga 720
aacactcagc tatctactag gaagaaagaa aaggccaatt catctaagtt ttgatgttga 780
cggactggac ccatctttca caccagctac tggcacacca gtcgtgggag gtctgacata 840
cagagaaggt ctctacatca cagaagaaat ctacaaaaca gggctactct caggattaga 900
tataatggaa gtgaacccat ccctggggaa gacaccagaa gaagtaactc gaacagtgaa 960
cacagcagtt gcaataacct tggcttgttt cggacttgct cgggagggta atcacaagcc 1020
tattgactac cttaacccac ctaagtaaat gtggaaacat ccgatataaa tctcatagtt 1080
aatggcataa ttagaaagct aatcattttc ttaagcatag agttatcctt ctaaagactt 1140
gttctttcag aaaaatgttt ttccaattag tataaactct acaaattccc tcttggtgta 1200
aaattcaaga tgtggaaatt ctaacttttt tgaaatttaa aagcttatat tttctaactt 1260
ggcaaaagac ttatccttag aaagagaagt gtacattgat ttccaattaa aaatttgctg 1320
gcattaaaaa taagcacact tacataagcc cccatacata gagtgggact cttggaatca 1380
ggagacaaag ctaccacatg tggaaaggta ctatgtgtcc atgtcattca aaaaatgtga 1440
ttttttataa taaactcttt ataacaagat taaaa 1475

<210> 11
<211> 1499
<212> DNA
<213> Artificial Sequence

<220>
<223> Human ARG1 transcript variant 1 mRNA sequence

<400> 11

```
ggaaaaaaaa gatgcgccct ctgtcactga gggttgactg actggagagc tcaagtgcag       60
caaagagaag tgtcagagca tgagcgccaa gtccagaacc atagggatta ttggagctcc      120
tttctcaaag ggacagccac gaggaggggt ggaagaaggc cctacagtat tgagaaaggc      180
tggtctgctt gagaaactta aagaacaagt aactcaaaac tttttaattt tagagtgtga      240
tgtgaaggat tatggggacc tgccctttgc tgacatccct aatgacagtc cctttcaaat      300
tgtgaagaat ccaaggtctg tgggaaaagc aagcgagcag ctggctggca aggtggcaga      360
agtcaagaag aacggaagaa tcagcctggt gctgggcgga gaccacagtt tggcaattgg      420
aagcatctct ggccatgcca gggtccaccc tgatcttgga gtcatctggg tggatgctca      480
cactgatatc aacactccac tgacaaccac aagtggaaac ttgcatggac aacctgtatc      540
tttcctcctg aaggaactaa aaggaaagat tcccgatgtg ccaggattct cctgggtgac      600
tccctgtata tctgccaagg atattgtgta tattggcttg agagacgtgg accctgggga      660
acactacatt ttgaaaactc taggcattaa atacttttca atgactgaag tggacagact      720
aggaattggc aaggtgatgg aagaaacact cagctatcta ctaggaagaa agaaaaggcc      780
aattcatcta agttttgatg ttgacggact ggacccatct ttcacaccag ctactggcac      840
accagtcgtg ggaggtctga catacagaga aggtctctac atcacagaag aaatctacaa      900
aacagggcta ctctcaggat tagatataat ggaagtgaac ccatccctgg ggaagacacc      960
agaagaagta actcgaacag tgaacacagc agttgcaata accttggctt gtttcggact     1020
tgctcgggag ggtaatcaca agcctattga ctaccttaac ccacctaagt aaatgtggaa     1080
acatccgata taaatctcat agttaatggc ataattagaa agctaatcat ttcttaagc      1140
atagagttat ccttctaaag acttgttctt tcagaaaaat gtttttccaa ttagtataaa     1200
ctctacaaat tccctcttgg tgtaaaattc aagatgtgga aattctaact tttttgaaat     1260
ttaaaagctt atattttcta acttggcaaa agacttatcc ttagaaagag aagtgtacat     1320
tgatttccaa ttaaaaattt gctggcatta aaaataagca cacttacata agccccccata     1380
catagagtgg gactcttgga atcaggagac aaagctacca catgtggaaa ggtactatgt     1440
gtccatgtca ttcaaaaaat gtgatttttt ataataaact ctttataaca agattaaaa      1499
```

<210> 12
<211> 8554
<212> DNA
<213> Artificial Sequence

<220>
<223> Human CREB5 transcript variant 1 mRNA sequence

<400> 12

```
aacatttaca acaaagttga ttctgtgtag ggttggaggc tagacagttc cacaaatttt        60
tagtcacatt ttccatgtca gttaaatcta gggagttcaa gactactgga aaaattagtc       120
tcattactaa aagaaactta gagaacgagg gaggtaccag agtctaggag gtacctctgg       180
gttgcagaag taattgtaaa ataccagacc tgttcttttt actaaaagct agtttcacta       240
```

```
tcttctggtc tgaaatactg aggcaaatac tcaagactta ttttcttcct aatcttgctg      300
gtgaaacaga agttactaga aagaaaggaa gaaaaaactt gatttggtga ctgcaggaag      360
caacacgttg ctgcttttat tctacagata atgatttatg aggaatccaa gatgaatttg      420
gagcaggaga ggccgtttgt ctgcagtgcc ccaggctgct cccagcgctt cccaacagag      480
gaccatctga tgattcatag gcacaaacat gaaatgactt tgaagtttcc ttcaataaaa      540
acagacaata tgttatcaga tcaaactccg accccaacga gattcctgaa gaactgcgag      600
gaggtgggcc tcttcagcga gctggactgc tccctggagc acgagttcag gaaggctcag      660
gaagaggaga gcagcaagcg gaatatctcg atgcataatg cagttggtgg ggccatgacg      720
gggcccggaa ctcaccagct tagcagcgct cggctgccca accatgacac caacgttgtg      780
attcagcaag ccatgccgtc gcctcagtcc agctctgtca tcactcaggc accttccacc      840
aaccgccaga tcgggcctgt cccaggctct ctatcttctc tgctacatct ccacaacaga      900
cagagacagc ccatgccagc ctccatgcct gggaccctgc ccaaccctac aatgccagga      960
tcttccgccg tcttgatgcc aatggagcga caaatgtcag tgaactccag catcatgggg     1020
atgcaaggtc caaatctcag caacccctgt gcttctcccc aggtccagcc aatgcattca     1080
gaagccaaaa tgaggttgaa ggctgcattg actcaccacc ctgctgccat gtcaaatggg     1140
aacatgaaca ccatgggaca catgatggag atgatgggct cccggcagga ccagacgcca     1200
caccatcaca tgcactcgca cccgcatcag caccagacac tgccacccca tcacccttac     1260
ccacaccagc accagcaccc agcacaccat cctcaccctc aaccccatca ccagcagaac     1320
catccacatc accactccca ttcccacctt catgcacacc cagcacatca ccagacctcg     1380
ccacatccgc ccctgcacac cggcaaccaa gcacaggttt caccagcaac acaacagatg     1440
cagccaaccc agacaataca gccaccccag cccacagggg ggcgccggcg aagggtggta     1500
gacgaggatc cggacgagag gcggcggaaa tttctggaac ggaaccgggc agctgccacc     1560
cgctgcagac agaagaggaa ggtctgggtg atgtcattgg aaaagaaagc agaagaactc     1620
acccagacaa acatgcagct tcagaatgaa gtgtctatgt tgaaaaatga ggtggcccag     1680
ctgaaacagt tgttgttaac acataaagac tgcccaataa cagccatgca gaaagaatca     1740
caaggatatc taagtccaga gagtagccct cctgctagtc ctgtcccagc ttgctcccag     1800
caacaagtca tccagcataa taccatcact acttcctcat cggtcagcga ggtggtagga     1860
agctccaccc tcagccagct caccactcac agaacagacc tgaatccgat tctttaaaat     1920
gcaccatcag acctggcctc caagaagagc tgtagcgtac catgcgtcct ttcttttaag     1980
ggcattttta gaattaactc agacctggaa gactcctcag ttcttcaaag actggctttc     2040
atttttatag ttattatgga aatgttgtct tttatactta gttatataag aaaaaaggga     2100
gttatgcaat taatatctat cagcttggga aacgctttgg tgcttttctc cagttttctg     2160
```

gtaccagtta cttgtttata aactgaacct tttctgtata tagccatggt ttcattctta 2220

tcagtccaac cctttgcctg aaacattgaa tcttgttaaa ccacagcttt tagctaaaat 2280

gaggtatacc tagatgtcaa gtaagacaga tccaaggtaa ctgggtagga aatcttttga 2340

catcttaact catgttgagt ttgtgctgtg gtgtcaccag aattccagat aaacacacag 2400

cctttcccat accttttttt ttcttactat aaaatattat aagatccatt gatgtccaaa 2460

taataccacc aagcatctct tcacctctcc tcctcttggt ccacttgcta atgcccagtt 2520

ttcttctcca tttccacttt ttcttaggct ccctatttac tattcatttt gacttccttc 2580

tgttttattt ttttcccttt agcattgcat gtgaataaga aaataatgtt taaagaaaaa 2640

aaaaaaaaag caaacctcca aaacgtggac ctaaccattg cttcacttac acttcaccca 2700

cagctggagt tcattcaact cttgcttttc acaaaatagt aaccaggaga tgtttaatgt 2760

gcctgattta atgtttttaa taatcacagc aaatgaaagg tggtttagtt ataagtgaag 2820

catggttgaa taccagctgg ggagacacta gggaagggag ctttgtaagc cttgattgcg 2880

aaagtccaaa ttttgatgtg gggctataac atgacaccct tggattgcga ctggttttat 2940

acggcctgcc tataacgttg aaaatccatg tactacataa taattcagaa gggctctatt 3000

cactacacag attacattgt tcaatcatca gctgctaata gcctaagatt tatttttttt 3060

tttttcttaa gcctatggaa ccggctttgc tgttctgggg ggtgaaaata gactaactac 3120

tggagaaaca aagagagaaa gaaaacccag tgtttccata ggggcacttt tagccttccc 3180

acaacagtta agcactcttt gactgctgaa ggaaccccat ggatgaggtg caggctactt 3240

cactcttttt ttttcttttt tgagacagag tctcacctat tgcccagact gaagtgcagt 3300

ggtgcgatca tggatcactg cagcagcatc ctccgagttc aagctatcct tccacctccg 3360

cctcctgagt agctgggacc acaggttcac ataaccatgc ctggctaatt tatttttact 3420

tttattttaa aataaaagat gaggtctgtc ttatgttgcc caggctggtc tcaaactatc 3480

ctacttcttc ctcccaaagt gttgggatta taggtgtgag ccactgcacc cagcctactt 3540

cactcttctg aattattctg atttattttc aacaactttt gtgaacttgc ccgtgataca 3600

aagcagatag tccctgaacc acagtcgtgc ctccttgaaa caagccattc tactgtgcta 3660

atgttttaat atcacatctc acaaataaca ggggtgaatg tttctctcta gcaatctagg 3720

caggtgctgg tgtttcatct ccatttgaat gcttgacctc ttaatgtgtg tgtgtgtgtg 3780

tgtgtgtgtg tgtgtgttca tgggttttaa aagaacagta ttttacaaaa ggtgtagctt 3840

ttataagagt gcagaaaagg gaaggatgtg tttttttctc tcactatagt ataagaatct 3900

attttggaga aaaaaagaaa atatgagggt ctcgaagcat gatttttata taactagttt 3960

cagttttatc taataactta ctttttaaat caatatttat caacaatctt tccttgtatg 4020

cagtgctttc aaaagatggt tttgagtgtc cagtgaaact tatgacttgg atatatggtt 4080
gaagaatcaa aacaaaagca aaaaaaaaaa gcaaaaaaag aaaagagaaa aaaagaaaaa 4140
atgcaaatgg aataattttc tattatattt tagacaaaca tatcattttc gagtatttta 4200
aatactgaat tcatagttgt tgttttttaa attccaacag taacagctga atggtttaat 4260
ctgactggct tcctaagaaa tgtttaagac tcagctttaa aaagaagtta acattcatat 4320
ctctgttttg aaatcaaaaa tcatatttca aaattctttc ctaggaccat ctatgtgtct 4380
cccctcccct ccacaaaaag gagaaagagt gcattaaaat gtttagttgg gtttttttaat 4440
ttttaatttt tatgttatgt tttgctttgt tttaagtaaa caaaaatttt tctttcttta 4500
ctgcatgcat agcacttaat aaaatggatt tttaaaaaat ccactagtaa tatcagaatg 4560
tccagggagt gactgtcact acaatgatgg tttagtttac ttctgttcca ccttttgatt 4620
gaaatattta gttgttaggc tgaaagcctc ggcagttaag aacttgcctg agttttcttc 4680
gttcagcaac ttgacagttt gactgatgtg cattatatat agctcaatta tgtctgtttt 4740
ttatgctaag taggaaaacc aaccacacac attagcaaac cggcctcaac atataattag 4800
aataaactgt cttcttgttc tactcagggc ctttaggtgt gttcattcac ggtatggaaa 4860
tacagtaaat gaaagattcc aactagttgt cagtgcttct tgaaattcca aacagaaaga 4920
tacattggtc aaatccaaca cttggcttat caatattaag tcttttacct aaaggcccag 4980
ccgtcaccag acaacagaat aatcaatctg cctgaaaatc cctcctcctt gtcctacact 5040
ttttgcctgt ttgggagaat atctttgtac tccattctcc tccctcagcc agttactggg 5100
tcacccatcc atgtgttcat gaatcaatca tcacggcctg cagagcacct gtcctaagga 5160
gggaaaatcc tgtcacactg cctctcccca ttcgtgtgtg gttttcttga tcggtgagat 5220
ctgtctctga agtcactgcc agcctccctg ggaacgtcta tagtgcctcc cctgccttat 5280
gtgatgggag ttaacaactc agataagtac acctgagagc atttctatca ggtaaactgt 5340
cacttaaatg gaggtgtcca catcttaatt gtttctcctt gacacatttc tcaatccacg 5400
aagccaggag aggtagagtg aaaatcccag ccatggatga atgtactaat ttgaaagcca 5460
agtgttaagt cggatgtttt cccgttacac tactactcag ccctctcctg cggccacatc 5520
aacggatgca agtcacagtc ttaacacagc ctgtgggaga caagcagttt gtgtgctcac 5580
agtatatatt atagtaatta gggtgactta gagcaaatac tcttcagatc ctatgtagtc 5640
agtgaaacaa aatggagagc gtattctgat agaaggacgt cgacggtgaa tgttctggtg 5700
gttgttgcct gttaagtaaa ctttagtgtg taagttgagt ttgtcattaa aatcataaac 5760
cagctgcggt aacagacaag cctttggctg gggagtttta agcctcggta actgctataa 5820
aactagccat ccagttagga tagaatgtgt ttctttctgg ttaaaaaaag gaaaaaccat 5880
ctaagaaaat atatatgtat gtatgtgtgt atacagtgga attcaaagga ccaaagcaaa 5940

atttgaacag gaatctatta atttagaatt ttataagata tttattaata aatgttattt 6000
ttaaacattc catttgaaca gtattctgta ggatctactt gtttttaaag tgttagtcca 6060
taataaacta ctatagttat gtgtattttc atttttcagg gtttcaaatg gctattctcc 6120
atcatttggt ggaaatgttt gcttagatct ctgtgcatag acatttcaag gatttttatt 6180
gctctgtgag ttattttttta atcaacattc tgaacagttt tttttaaaca tttatttctg 6240
tgtgttcatt tttaaagtaa gctctttcat ttaggaagca gagttcagct aaagggaatc 6300
agtaactcta actggaacag ctttcttgta gaagtgtaaa aacagcttca tctctgcctc 6360
tctccacccc accccaattt cctagaaagc cttgcactat tcagctccct tagtgctttt 6420
tgtcccttcc cgaacaatat gcagtagctt taagccattc aagctccatt atgcagtata 6480
tctgagaagg gaaaggaaac aacccattta aatttgaata aaaccgtgcc tatgcgaaca 6540
gtagcaattt agaatctctt ttctgctttt aaaataattt atatttaaaa attgcacttt 6600
agctttttga tccctttgta tttctcttat tctctttcta acctcttctc tgtcctcaaa 6660
cttgcctttg ctctccttta caataccccc cacccctcct ccaaggctct gagcggcatc 6720
atttaaaata ctttacagat atttgcacca ggtacattta tgtgcgtcca ttggtagcac 6780
agctgagacc tgtgtctcac atcagcctag gtgaagccta ctacaagaat gccaaggaga 6840
agagccagta cactatatgg tttatactct ttatcccttt attcatagca tgttttttaa 6900
aaatgttata ttatgcaaca gatgtgaggc agcagctaag ctatacttaa gaattttctc 6960
tcaccttcca aaccaaagtg tcctgaataa gccaggagac ttattctttt gtgcaccctg 7020
gtgcacatct gactgttgtc ctagccatag actctctgag gccactgaaa gaacagtggc 7080
cctatcgatt tcattcctag gtctcaaaaa tacaatgttg ccttgtaaca taattaggga 7140
cagcacctct atttcacaat tataatctaa ggtaggataa gacgacacag cagcaataaa 7200
cttacaagta aaattcaata ccaaaacaaa cacaaagaaa tttaaaaaac aaaaaaccta 7260
gctcatcatg ttgtgaaaat gaaaaagtga atgtccattc aaaatatttt actatttctt 7320
gtggagtttt tcagtgatgt aatgcttgta gccaaattgc ttaaagagtg tttatatatt 7380
tttttcctta taaattgtct attttttaaa aaagctattt aaccacagct gaagtggggg 7440
gtaaggccaa attgccaaca cttgttaaaa gattaatact cttaagtggc actctgatac 7500
ctttccaact tgtcatcaga aaggaatcaa taattaccaa ctgttgtatt tagaccaact 7560
tacaatatct agctcattag aagccaggat ctagaaagct ccttctaagc catttaagat 7620
attcttacat tgagcttcat attatagaac tttataggat tggatatttt acaatagaat 7680
aatttagcct caggactgag aatgtggaag ctgaataaat tagctttaaa tacatcatta 7740
aaatcttatg cacaataagc tcattagatt ctagttttct cctttagaat accaatgcca 7800

```
cagacactac aggagataat gaaaggtatc agttgtgttg agtggaggga gtttaagaga     7860
aaggaccctt cccaaccagc agccagtaga aaatacaacc tactcacctt tttcccttct     7920
aagttctgct aaatcacatc tgcctcatag agaaaggaat gttgcctttg agaactgtct     7980
tggagaacag ataagcttga aatgttctct ctagagagga catagggttt gggatcctct     8040
gaaaaggccc agaaaaatag ctcagttcaa atacaatgtt ctaggacaat tggaatataa     8100
atattgtcca aaaatataat taaaagaaaa aagtttagca ctgtgtaaag taagtgttaa     8160
ctgaggaagt cccaaaaagg tgctgtcact ttaagttctg gacttggggt tctttgtatt     8220
tgtaaacagc aaagcatttg tgtttgtttg tctatttgta aagcaaccac cttccttatt     8280
ggaaggagaa aaaaaggggt acatacatgt aaatacttgc tgcagcattt aatatgttta     8340
attttgtgtt aagctttttg ttgcatcgtg aacacattta ttgttaccaa tggacaatga     8400
gttcattaag actgttcaac taggtcagat tttacatct ctttctagca agaagagaca      8460
agattttgtg catttgtaca aatgttaata tcactgcaat tccaatataa taaagcactc     8520
aaatgcaaat aaaaaaaaaa aaaaaaaaaa aaaa                                8554
```

<210> 13
<211> 7917
<212> DNA
<213> Artificial Sequence

<220>
<223> Human CREB5 transcript variant 4 mRNA sequence

<400> 13

```
agagagcaag tgagcgagag cccagccgga gggagagcca gagctaggcg caaaagaagg      60
gccagcacat tacatcatcg cttggcctgg aatcaaatgg gaaggatatg actcactcaa     120
gctagttaag ctttggctca aattctacac acactcattc cagagctctc atgttctgca     180
cctcaggagg gaattcagcc tcagtgatgt ccatgaggcc tgtcccaggc tctctatctt     240
ctctgctaca tctccacaac agacagagac agcccatgcc agcctccatg cctgggaccc     300
tgcccaaccc tacaatgcca ggatcttccg ccgtcttgat gccaatggag cgacaaatgt     360
cagtgaactc cagcatcatg ggatgcaag gtccaaatct cagcaacccc tgtgcttctc     420
cccaggtcca gccaatgcat tcagaagcca aaatgaggtt gaaggctgca ttgactcacc     480
accctgctgc catgtcaaat gggaacatga acaccatggg acacatgatg gagatgatgg     540
gctcccggca ggaccagacg ccacaccatc acatgcactc gcacccgcat cagcaccaga     600
cactgccacc ccatcaccct tacccacacc agcaccagca cccagcacac catcctcacc     660
ctcaacccca tcaccagcag aaccatccac atcaccactc ccattcccac cttcatgcac     720
acccagcaca tcaccagacc tcgccacatc cgcccctgca caccggcaac caagcacagg     780
tttcaccagc aacacaacag atgcagccaa cccagacaat acagccaccc cagcccacag     840
```

gggggcgccg gcgaagggtg gtagacgagg atccggacga gaggcggcgg aaatttctgg 900
aacggaaccg ggcagctgcc acccgctgca gacagaagag gaaggtctgg gtgatgtcat 960
tggaaaagaa agcagaagaa ctcacccaga caaacatgca gcttcagaat gaagtgtcta 1020
tgttgaaaaa tgaggtggcc cagctgaaac agttgttgtt aacacataaa gactgcccaa 1080
taacagccat gcagaaagaa tcacaaggat atctaagtcc agagagtagc cctcctgcta 1140
gtcctgtccc agcttgctcc cagcaacaag tcatccagca taataccatc actacttcct 1200
catcggtcag cgaggtggta ggaagctcca ccctcagcca gctcaccact cacagaacag 1260
acctgaatcc gattctttaa aatgcaccat cagacctggc ctccaagaag agctgtagcg 1320
taccatgcgt cctttctttt aagggcattt ttagaattaa ctcagacctg gaagactcct 1380
cagttcttca aagactggct ttcattttta tagttattat ggaaatgttg tcttttatac 1440
ttagttatat aagaaaaaag ggagttatgc aattaatatc tatcagcttg ggaaacgctt 1500
tggtgctttt ctccagtttt ctggtaccag ttacttgttt ataaactgaa ccttttctgt 1560
atatagccat ggtttcattc ttatcagtcc aaccctttgc ctgaaacatt gaatcttgtt 1620
aaaccacagc ttttagctaa aatgaggtat acctagatgt caagtaagac agatccaagg 1680
taactgggta ggaaatcttt tgacatctta actcatgttg agtttgtgct gtggtgtcac 1740
cagaattcca gataaacaca cagcctttcc catacctttt tttttcttac tataaaatat 1800
tataagatcc attgatgtcc aaataatacc accaagcatc tcttcacctc tcctcctctt 1860
ggtccacttg ctaatgccca gttttcttct ccatttccac tttttcttag gctccctatt 1920
tactattcat tttgacttcc ttctgtttta tttttttccc tttagcattg catgtgaata 1980
agaaaataat gtttaaagaa aaaaaaaaaa aagcaaacct ccaaaacgtg gacctaacca 2040
ttgcttcact tacacttcac ccacagctgg agttcattca actcttgctt ttcacaaaat 2100
agtaaccagg agatgtttaa tgtgcctgat ttaatgtttt taataatcac agcaaatgaa 2160
aggtggttta gttataagtg aagcatggtt gaataccagc tggggagaca ctagggaagg 2220
gagctttgta agccttgatt gcgaaagtcc aaattttgat gtggggctat aacatgacac 2280
ccttggattg cgactggttt tatacggcct gcctataacg ttgaaaatcc atgtactaca 2340
taataattca gaagggctct attcactaca cagattacat tgttcaatca tcagctgcta 2400
atagcctaag atttattttt tttttttct taagcctatg gaaccggctt tgctgttctg 2460
gggggtgaaa atagactaac tactggagaa acaaagagag aaagaaaacc cagtgtttcc 2520
ataggggcac ttttagcctt cccacaacag ttaagcactc tttgactgct gaaggaaccc 2580
catggatgag gtgcaggcta cttcactctt tttttttctt ttttgagaca gagtctcacc 2640
tattgcccag actgaagtgc agtggtgcga tcatggatca ctgcagcagc atcctccgag 2700

ttcaagctat ccttccacct ccgcctcctg agtagctggg accacaggtt cacataacca 2760
tgcctggcta atttattttt acttttattt taaaataaaa gatgaggtct gtcttatgtt 2820
gcccaggctg gtctcaaact atcctacttc ttcctcccaa agtgttggga ttataggtgt 2880
gagccactgc acccagccta cttcactctt ctgaattatt ctgatttatt ttcaacaact 2940
tttgtgaact tgcccgtgat acaaagcaga tagtccctga accacagtcg tgcctccttg 3000
aaacaagcca ttctactgtg ctaatgtttt aatatcacat ctcacaaata acaggggtga 3060
atgtttctct ctagcaatct aggcaggtgc tggtgtttca tctccatttg aatgcttgac 3120
ctcttaatgt gtgtgtgtgt gtgtgtgtgt gtgtgtgtgt tcatgggttt taaaagaaca 3180
gtattttaca aaaggtgtag cttttataag agtgcagaaa agggaaggat gtgttttttt 3240
ctctcactat agtataagaa tctattttgg agaaaaaaag aaaatatgag ggtctcgaag 3300
catgattttt atataactag tttcagtttt atctaataac ttacttttta aatcaatatt 3360
tatcaacaat ctttccttgt atgcagtgct ttcaaaagat ggttttgagt gtccagtgaa 3420
acttatgact tggatatatg gttgaagaat caaaacaaaa gcaaaaaaaa aaagcaaaaa 3480
aagaaaagag aaaaaaagaa aaaatgcaaa tggaataatt ttctattata ttttagacaa 3540
acatatcatt ttcgagtatt ttaaatactg aattcatagt tgttgttttt taaattccaa 3600
cagtaacagc tgaatggttt aatctgactg gcttcctaag aaatgtttaa gactcagctt 3660
taaaaagaag ttaacattca tatctctgtt ttgaaatcaa aaatcatatt tcaaaattct 3720
ttcctaggac catctatgtg tctcccctcc cctccacaaa aaggagaaag agtgcattaa 3780
aatgtttagt tgggtttttt aatttttaat ttttatgtta tgttttgctt tgttttaagt 3840
aaacaaaaat ttttctttct ttactgcatg catagcactt aataaaatgg atttttaaaa 3900
aatccactag taatatcaga atgtccaggg agtgactgtc actacaatga tggtttagtt 3960
tacttctgtt ccaccttttg attgaaatat ttagttgtta ggctgaaagc ctcggcagtt 4020
aagaacttgc ctgagttttc ttcgttcagc aacttgacag tttgactgat gtgcattata 4080
tatagctcaa ttatgtctgt tttttatgct aagtaggaaa accaaccaca cacattagca 4140
aaccggcctc aacatataat tagaataaac tgtcttcttg ttctactcag ggcctttagg 4200
tgtgttcatt cacggtatgg aaatacagta aatgaaagat tccaactagt tgtcagtgct 4260
tcttgaaatt ccaaacagaa agatacattg gtcaaatcca acacttggct tatcaatatt 4320
aagtctttta cctaaaggcc cagccgtcac cagacaacag aataatcaat ctgcctgaaa 4380
atccctcctc cttgtcctac actttttgcc tgtttgggag aatatctttg tactccattc 4440
tcctccctca gccagttact gggtcaccca tccatgtgtt catgaatcaa tcatcacggc 4500
ctgcagagca cctgtcctaa ggagggaaaa tcctgtcaca ctgcctctcc ccattcgtgt 4560
gtggttttct tgatcggtga gatctgtctc tgaagtcact gccagcctcc ctgggaacgt 4620

ctatagtgcc tcccctgcct tatgtgatgg gagttaacaa ctcagataag tacacctgag 4680
agcatttcta tcaggtaaac tgtcacttaa atggaggtgt ccacatctta attgtttctc 4740
cttgacacat ttctcaatcc acgaagccag gagaggtaga gtgaaaatcc cagccatgga 4800
tgaatgtact aatttgaaag ccaagtgtta agtcggatgt tttcccgtta cactactact 4860
cagccctctc ctgcggccac atcaacggat gcaagtcaca gtcttaacac agcctgtggg 4920
agacaagcag tttgtgtgct cacagtatat attatagtaa ttagggtgac ttagagcaaa 4980
tactcttcag atcctatgta gtcagtgaaa caaaatggag agcgtattct gatagaagga 5040
cgtcgacggt gaatgttctg gtggttgttg cctgttaagt aaactttagt gtgtaagttg 5100
agtttgtcat taaaatcata aaccagctgc ggtaacagac aagcctttgg ctggggagtt 5160
ttaagcctcg gtaactgcta taaaactagc catccagtta ggatagaatg tgtttctttc 5220
tggttaaaaa aaggaaaaac catctaagaa aatatatatg tatgtatgtg tgtatacagt 5280
ggaattcaaa ggaccaaagc aaaatttgaa caggaatcta ttaatttaga attttataag 5340
atatttatta ataaatgtta tttttaaaca ttccatttga acagtattct gtaggatcta 5400
cttgttttta aagtgttagt ccataataaa ctactatagt tatgtgtatt ttcatttttc 5460
agggtttcaa atggctattc tccatcattt ggtggaaatg tttgcttaga tctctgtgca 5520
tagacatttc aaggattttt attgctctgt gagttatttt ttaatcaaca ttctgaacag 5580
tttttttaa acatttattt ctgtgtgttc atttttaaag taagctcttt catttaggaa 5640
gcagagttca gctaaaggga atcagtaact ctaactggaa cagctttctt gtagaagtgt 5700
aaaaacagct tcatctctgc ctctctccac cccaccccaa tttcctagaa agccttgcac 5760
tattcagctc ccttagtgct ttttgtccct tcccgaacaa tatgcagtag ctttaagcca 5820
ttcaagctcc attatgcagt atatctgaga agggaaagga aacaacccat ttaaatttga 5880
ataaaaccgt gcctatgcga acagtagcaa tttagaatct cttttctgct tttaaaataa 5940
tttatattta aaaattgcac tttagctttt tgatcccttt gtatttctct tattctcttt 6000
ctaacctctt ctctgtcctc aaacttgcct ttgctctcct ttacaatacc ccccacccct 6060
cctccaaggc tctgagcggc atcatttaaa atactttaca gatatttgca ccaggtacat 6120
ttatgtgcgt ccattggtag cacagctgag acctgtgtct cacatcagcc taggtgaagc 6180
ctactacaag aatgccaagg agaagagcca gtacactata tggtttatac tctttatccc 6240
tttattcata gcatgttttt taaaaatgtt atattatgca acagatgtga ggcagcagct 6300
aagctatact taagaatttt ctctcacctt ccaaaccaaa gtgtcctgaa taagccagga 6360
gacttattct tttgtgcacc ctggtgcaca tctgactgtt gtcctagcca tagactctct 6420
gaggccactg aaagaacagt ggccctatcg atttcattcc taggtctcaa aaatacaatg 6480

```
ttgccttgta acataattag ggacagcacc tctatttcac aattataatc taaggtagga     6540
taagacgaca cagcagcaat aaacttacaa gtaaaattca ataccaaaac aaacacaaag     6600
aaatttaaaa aacaaaaaac ctagctcatc atgttgtgaa aatgaaaaag tgaatgtcca     6660
ttcaaaatat tttactattt cttgtggagt ttttcagtga tgtaatgctt gtagccaaat     6720
tgcttaaaga gtgtttatat atttttttcc ttataaattg tctatttttt aaaaaagcta     6780
tttaaccaca gctgaagtgg ggggtaaggc caaattgcca acacttgtta aaagattaat     6840
actcttaagt ggcactctga tacctttcca acttgtcatc agaaaggaat caataattac     6900
caactgttgt atttagacca acttacaata tctagctcat tagaagccag gatctagaaa     6960
gctccttcta agccatttaa gatattctta cattgagctt catattatag aactttatag     7020
gattggatat tttacaatag aataatttag cctcaggact gagaatgtgg aagctgaata     7080
aattagcttt aaatacatca ttaaaatctt atgcacaata agctcattag attctagttt     7140
tctcctttag aataccaatg ccacagacac tacaggagat aatgaaaggt atcagttgtg     7200
ttgagtggag ggagtttaag agaaaggacc cttcccaacc agcagccagt agaaaataca     7260
acctactcac ctttttccct tctaagttct gctaaatcac atctgcctca tagagaaagg     7320
aatgttgcct ttgagaactg tcttggagaa cagataagct tgaaatgttc tctctagaga     7380
ggacataggg tttgggatcc tctgaaaagg cccagaaaaa tagctcagtt caaatacaat     7440
gttctaggac aattggaata taaatattgt ccaaaaatat aattaaaaga aaaaagttta     7500
gcactgtgta aagtaagtgt taactgagga agtcccaaaa aggtgctgtc actttaagtt     7560
ctggacttgg ggttctttgt atttgtaaac agcaaagcat ttgtgtttgt ttgtctattt     7620
gtaaagcaac caccttcctt attggaagga gaaaaaaagg ggtacataca tgtaaatact     7680
tgctgcagca tttaatatgt ttaattttgt gttaagcttt ttgttgcatc gtgaacacat     7740
ttattgttac caatggacaa tgagttcatt aagactgttc aactaggtca gatttttaca     7800
tctctttcta gcaagaagag acaagatttt gtgcatttgt acaaatgtta atatcactgc     7860
aattccaata taataaagca ctcaaatgca aataaaaaaa aaaaaaaaaa aaaaaaa        7917
```

<210> 14
<211> 8210
<212> DNA
<213> Artificial Sequence

<220>
<223> Human CREB5 transcript variant 3 mRNA sequence

<400> 14

```
gatctgatga atccaaggag tggagcaaga ggcagatttt ggacacggtt atgagaatga       60
cagaaactgc ctaaagcatt tatgctctgg cattcgtccc tgtttctgga ggtccagtaa      120
gcgcttccca acagaggacc atctgatgat tcataggcac aaacatgaaa tgactttgaa      180
```

gtttccttca ataaaaacag acaatatgtt atcagatcaa actccgaccc caacgagatt 240

cctgaagaac tgcgaggagg tgggcctctt cagcgagctg gactgctccc tggagcacga 300

gttcaggaag gctcaggaag aggagagcag caagcggaat atctcgatgc ataatgcagt 360

tggtggggcc atgacggggc ccggaactca ccagcttagc agcgctcggc tgcccaacca 420

tgacaccaac gttgtgattc agcaagccat gccgtcgcct cagtccagct ctgtcatcac 480

tcaggcacct tccaccaacc gccagatcgg gcctgtccca ggctctctat cttctctgct 540

acatctccac aacagacaga gacagcccat gccagcctcc atgcctggga ccctgcccaa 600

ccctacaatg ccaggatctt ccgccgtctt gatgccaatg gagcgacaaa tgtcagtgaa 660

ctccagcatc atggggatgc aaggtccaaa tctcagcaac ccctgtgctt ctccccaggt 720

ccagccaatg cattcagaag ccaaaatgag gttgaaggct gcattgactc accaccctgc 780

tgccatgtca aatgggaaca tgaacaccat gggacacatg atggagatga tgggctcccg 840

gcaggaccag acgccacacc atcacatgca ctcgcacccg catcagcacc agacactgcc 900

accccatcac ccttacccac accagcacca gcacccagca caccatcctc accctcaacc 960

ccatcaccag cagaaccatc cacatcacca ctcccattcc caccttcatg cacacccagc 1020

acatcaccag acctcgccac atccgcccct gcacaccggc aaccaagcac aggtttcacc 1080

agcaacacaa cagatgcagc caacccagac aatacagcca ccccagccca caggggggcg 1140

ccggcgaagg gtggtagacg aggatccgga cgagaggcgg cggaaatttc tggaacggaa 1200

ccgggcagct gccacccgct gcagacagaa gaggaaggtc tgggtgatgt cattggaaaa 1260

gaaagcagaa gaactcaccc agacaaacat gcagcttcag aatgaagtgt ctatgttgaa 1320

aaatgaggtg gcccagctga aacagttgtt gttaacacat aaagactgcc caataacagc 1380

catgcagaaa gaatcacaag gatatctaag tccagagagt agccctcctg ctagtcctgt 1440

cccagcttgc tcccagcaac aagtcatcca gcataatacc atcactactt cctcatcggt 1500

cagcgaggtg gtaggaagct ccaccctcag ccagctcacc actcacagaa cagacctgaa 1560

tccgattctt taaaatgcac catcagacct ggcctccaag aagagctgta gcgtaccatg 1620

cgtcctttct tttaagggca tttttagaat taactcagac ctggaagact cctcagttct 1680

tcaaagactg gctttcattt ttatagttat tatggaaatg ttgtctttta tacttagtta 1740

tataagaaaa aagggagtta tgcaattaat atctatcagc ttgggaaacg ctttggtgct 1800

tttctccagt tttctggtac cagttacttg tttataaact gaacctttc tgtatatagc 1860

catggtttca ttcttatcag tccaaccctt tgcctgaaac attgaatctt gttaaaccac 1920

agcttttagc taaaatgagg tatacctaga tgtcaagtaa gacagatcca aggtaactgg 1980

gtaggaaatc ttttgacatc ttaactcatg ttgagtttgt gctgtggtgt caccagaatt 2040

ccagataaac acacagcctt tcccatacct ttttttttct tactataaaa tattataaga 2100
tccattgatg tccaaataat accaccaagc atctcttcac ctctcctcct cttggtccac 2160
ttgctaatgc ccagttttct tctccatttc cactttttct taggctccct atttactatt 2220
cattttgact tccttctgtt ttattttttt ccctttagca ttgcatgtga ataagaaaat 2280
aatgtttaaa gaaaaaaaaa aaaaagcaaa cctccaaaac gtggacctaa ccattgcttc 2340
acttacactt cacccacagc tggagttcat tcaactcttg cttttcacaa aatagtaacc 2400
aggagatgtt taatgtgcct gatttaatgt ttttaataat cacagcaaat gaaaggtggt 2460
ttagttataa gtgaagcatg gttgaatacc agctggggag acactaggga agggagcttt 2520
gtaagccttg attgcgaaag tccaaatttt gatgtggggc tataacatga caccccttgga 2580
ttgcgactgg ttttatacgg cctgcctata acgttgaaaa tccatgtact acataataat 2640
tcagaagggc tctattcact acacagatta cattgttcaa tcatcagctg ctaatagcct 2700
aagatttatt tttttttttt tcttaagcct atggaaccgg ctttgctgtt ctggggggtg 2760
aaaatagact aactactgga gaaacaaaga gagaaagaaa acccagtgtt tccatagggg 2820
cacttttagc cttcccacaa cagttaagca ctctttgact gctgaaggaa ccccatggat 2880
gaggtgcagg ctacttcact cttttttttt cttttttgag acagagtctc acctattgcc 2940
cagactgaag tgcagtggtg cgatcatgga tcactgcagc agcatcctcc gagttcaagc 3000
tatccttcca cctccgcctc ctgagtagct gggaccacag gttcacataa ccatgcctgg 3060
ctaatttatt tttactttta ttttaaaata aaagatgagg tctgtcttat gttgcccagg 3120
ctggtctcaa actatcctac ttcttcctcc caaagtgttg ggattatagg tgtgagccac 3180
tgcacccagc ctacttcact cttctgaatt attctgattt attttcaaca acttttgtga 3240
acttgcccgt gatacaaagc agatagtccc tgaaccacag tcgtgcctcc ttgaaacaag 3300
ccattctact gtgctaatgt tttaatatca catctcacaa ataacagggg tgaatgtttc 3360
tctctagcaa tctaggcagg tgctggtgtt tcatctccat ttgaatgctt gacctcttaa 3420
tgtgtgtgtg tgtgtgtgtg tgtgtgtgtg tgttcatggg ttttaaaaga acagtatttt 3480
acaaaaggtg tagcttttat aagagtgcag aaaagggaag gatgtgtttt tttctctcac 3540
tatagtataa gaatctattt tggagaaaaa aagaaaatat gagggtctcg aagcatgatt 3600
tttatataac tagtttcagt tttatctaat aacttacttt ttaaatcaat atttatcaac 3660
aatctttcct tgtatgcagt gctttcaaaa gatggttttg agtgtccagt gaaacttatg 3720
acttggatat atggttgaag aatcaaaaca aaagcaaaaa aaaaaagcaa aaaaagaaaa 3780
gagaaaaaaa gaaaaaatgc aaatggaata attttctatt atattttaga caaacatatc 3840
attttcgagt attttaaata ctgaattcat agttgttgtt ttttaaattc caacagtaac 3900
agctgaatgg tttaatctga ctggcttcct aagaaatgtt taagactcag ctttaaaaag 3960

aagttaacat tcatatctct gttttgaaat caaaaatcat atttcaaaat tctttcctag 4020

gaccatctat gtgtctcccc tcccctccac aaaaaggaga aagagtgcat taaaatgttt 4080

agttgggttt tttaattttt aatttttatg ttatgttttg ctttgtttta agtaaacaaa 4140

aatttttctt tctttactgc atgcatagca cttaataaaa tggattttta aaaaatccac 4200

tagtaatatc agaatgtcca gggagtgact gtcactacaa tgatggttta gtttacttct 4260

gttccacctt ttgattgaaa tatttagttg ttaggctgaa agcctcggca gttaagaact 4320

tgcctgagtt ttcttcgttc agcaacttga cagtttgact gatgtgcatt atatatagct 4380

caattatgtc tgtttttttat gctaagtagg aaaaccaacc acacacatta gcaaaccggc 4440

ctcaacatat aattagaata aactgtcttc ttgttctact cagggccttt aggtgtgttc 4500

attcacggta tggaaataca gtaaatgaaa gattccaact agttgtcagt gcttcttgaa 4560

attccaaaca gaaagataca ttggtcaaat ccaacacttg gcttatcaat attaagtctt 4620

ttacctaaag gcccagccgt caccagacaa cagaataatc aatctgcctg aaaatccctc 4680

ctccttgtcc tacacttttt gcctgtttgg gagaatatct ttgtactcca ttctcctccc 4740

tcagccagtt actgggtcac ccatccatgt gttcatgaat caatcatcac ggcctgcaga 4800

gcacctgtcc taaggaggga aaatcctgtc acactgcctc tccccattcg tgtgtggttt 4860

tcttgatcgg tgagatctgt ctctgaagtc actgccagcc tccctgggaa cgtctatagt 4920

gcctcccctg ccttatgtga tgggagttaa caactcagat aagtacacct gagagcattt 4980

ctatcaggta aactgtcact taaatggagg tgtccacatc ttaattgttt ctccttgaca 5040

catttctcaa tccacgaagc caggagaggt agagtgaaaa tcccagccat ggatgaatgt 5100

actaatttga aagccaagtg ttaagtcgga tgttttcccg ttacactact actcagccct 5160

ctcctgcggc cacatcaacg gatgcaagtc acagtcttaa cacagcctgt gggagacaag 5220

cagtttgtgt gctcacagta tatattatag taattagggt gacttagagc aaatactctt 5280

cagatcctat gtagtcagtg aaacaaaatg gagagcgtat tctgatagaa ggacgtcgac 5340

ggtgaatgtt ctggtggttg ttgcctgtta agtaaacttt agtgtgtaag ttgagtttgt 5400

cattaaaatc ataaaccagc tgcggtaaca gacaagcctt tggctgggga gttttaagcc 5460

tcggtaactg ctataaaact agccatccag ttaggataga atgtgtttct ttctggttaa 5520

aaaaaggaaa aaccatctaa gaaaatatat atgtatgtat gtgtgtatac agtggaattc 5580

aaaggaccaa agcaaaattt gaacaggaat ctattaattt agaattttat aagatattta 5640

ttaataaatg ttatttttaa acattccatt tgaacagtat tctgtaggat ctacttgttt 5700

ttaaagtgtt agtccataat aaactactat agttatgtgt attttcattt ttcagggttt 5760

caaatggcta ttctccatca tttggtggaa atgtttgctt agatctctgt gcatagacat 5820

ttcaaggatt tttattgctc tgtgagttat tttttaatca acattctgaa cagtttttt 5880
taaacattta tttctgtgtg ttcattttta aagtaagctc tttcatttag gaagcagagt 5940
tcagctaaag ggaatcagta actctaactg gaacagcttt cttgtagaag tgtaaaaaca 6000
gcttcatctc tgcctctctc caccccaccc caatttccta gaaagccttg cactattcag 6060
ctcccttagt gctttttgtc ccttcccgaa caatatgcag tagctttaag ccattcaagc 6120
tccattatgc agtatatctg agaagggaaa ggaaacaacc catttaaatt tgaataaaac 6180
cgtgcctatg cgaacagtag caatttagaa tctcttttct gcttttaaaa taatttatat 6240
ttaaaaattg cactttagct ttttgatccc tttgtatttc tcttattctc tttctaacct 6300
cttctctgtc ctcaaacttg cctttgctct cctttacaat accccccacc cctcctccaa 6360
ggctctgagc ggcatcattt aaaatacttt acagatattt gcaccaggta catttatgtg 6420
cgtccattgg tagcacagct gagacctgtg tctcacatca gcctaggtga agcctactac 6480
aagaatgcca aggagaagag ccagtacact atatggttta tactctttat ccctttattc 6540
atagcatgtt ttttaaaaat gttatattat gcaacagatg tgaggcagca gctaagctat 6600
acttaagaat tttctctcac cttccaaacc aaagtgtcct gaataagcca ggagacttat 6660
tcttttgtgc accctggtgc acatctgact gttgtcctag ccatagactc tctgaggcca 6720
ctgaaagaac agtggcccta tcgatttcat tcctaggtct caaaaataca atgttgcctt 6780
gtaacataat tagggacagc acctctattt cacaattata atctaaggta ggataagacg 6840
acacagcagc aataaactta caagtaaaat tcaataccaa aacaaacaca aagaaattta 6900
aaaaacaaaa aacctagctc atcatgttgt gaaaatgaaa aagtgaatgt ccattcaaaa 6960
tattttacta tttcttgtgg agtttttcag tgatgtaatg cttgtagcca aattgcttaa 7020
agagtgttta tatattttt tccttataaa ttgtctattt tttaaaaaag ctatttaacc 7080
acagctgaag tggggggtaa ggccaaattg ccaacacttg ttaaaagatt aatactctta 7140
agtggcactc tgataccttt ccaacttgtc atcagaaagg aatcaataat taccaactgt 7200
tgtatttaga ccaacttaca atatctagct cattagaagc caggatctag aaagctcctt 7260
ctaagccatt taagatattc ttacattgag cttcatatta tagaacttta taggattgga 7320
tattttacaa tagaataatt tagcctcagg actgagaatg tggaagctga ataaattagc 7380
tttaaataca tcattaaaat cttatgcaca ataagctcat tagattctag ttttctcctt 7440
tagaatacca atgccacaga cactacagga gataatgaaa ggtatcagtt gtgttgagtg 7500
gagggagttt aagagaaagg acccttccca accagcagcc agtagaaaat acaacctact 7560
caccttttc ccttctaagt tctgctaaat cacatctgcc tcatagagaa aggaatgttg 7620
cctttgagaa ctgtcttgga gaacagataa gcttgaaatg ttctctctag agaggacata 7680
gggtttggga tcctctgaaa aggcccagaa aaatagctca gttcaaatac aatgttctag 7740

```
gacaattgga atataaatat tgtccaaaaa tataattaaa agaaaaaagt ttagcactgt     7800
gtaaagtaag tgttaactga ggaagtccca aaaaggtgct gtcactttaa gttctggact     7860
tggggttctt tgtatttgta aacagcaaag catttgtgtt tgtttgtcta tttgtaaagc     7920
aaccaccttc cttattggaa ggagaaaaaa aggggtacat acatgtaaat acttgctgca     7980
gcatttaata tgtttaattt tgtgttaagc tttttgttgc atcgtgaaca catttattgt     8040
taccaatgga caatgagttc attaagactg ttcaactagg tcagattttt acatctcttt     8100
ctagcaagaa gagacaagat tttgtgcatt tgtacaaatg ttaatatcac tgcaattcca     8160
atataataaa gcactcaaat gcaaataaaa aaaaaaaaaa aaaaaaaaaa                8210
```

<210> 15
<211> 8235
<212> DNA
<213> Artificial Sequence

<220>
<223> Human CREB5 transcript variant 2 mRNA sequence

<400> 15

```
ttttagtggt ggagtcaatt tatttctgaa acgatctcat ttacctgaat gaggagctca      60
tatttatttt caggatttat gaggaatcca agatgaattt ggagcaggag aggccgtttg     120
tctgcagtgc cccaggctgc tcccagcgct tcccaacaga ggaccatctg atgattcata     180
ggcacaaaca tgaaatgact ttgaagtttc cttcaataaa aacagacaat atgttatcag     240
atcaaactcc gaccccaacg agattcctga agaactgcga ggaggtgggc ctcttcagcg     300
agctggactg ctccctggag cacgagttca ggaaggctca ggaagaggag agcagcaagc     360
ggaatatctc gatgcataat gcagttggtg gggccatgac ggggcccgga actcaccagc     420
ttagcagcgc tcggctgccc aaccatgaca ccaacgttgt gattcagcaa gccatgccgt     480
cgcctcagtc cagctctgtc atcactcagg caccttccac caaccgccag atcgggcctg     540
tcccaggctc tctatcttct ctgctacatc tccacaacag acagagacag cccatgccag     600
cctccatgcc tgggaccctg cccaacccta caatgccagg atcttccgcc gtcttgatgc     660
caatggagcg acaaatgtca gtgaactcca gcatcatggg gatgcaaggt ccaaatctca     720
gcaacccctg tgcttctccc caggtccagc caatgcattc agaagccaaa atgaggttga     780
aggctgcatt gactcaccac cctgctgcca tgtcaaatgg gaacatgaac accatgggac     840
acatgatgga gatgatgggc tcccggcagg accagacgcc acaccatcac atgcactcgc     900
acccgcatca gcaccagaca ctgccacccc atcaccctta cccacaccag caccagcacc     960
cagcacacca tcctcaccct caaccccatc accagcagaa ccatccacat caccactccc    1020
attcccacct tcatgcacac ccagcacatc accagacctc gccacatccg cccctgcaca    1080
```

ccggcaacca agcacaggtt tcaccagcaa cacaacagat gcagccaacc cagacaatac 1140

agccacccca gcccacaggg gggcgccggc gaagggtggt agacgaggat ccggacgaga 1200

ggcggcggaa atttctggaa cggaaccggg cagctgccac ccgctgcaga cagaagagga 1260

aggtctgggt gatgtcattg gaaaagaaag cagaagaact cacccagaca aacatgcagc 1320

ttcagaatga agtgtctatg ttgaaaaatg aggtggccca gctgaaacag ttgttgttaa 1380

cacataaaga ctgcccaata acagccatgc agaaagaatc acaaggatat ctaagtccag 1440

agagtagccc tcctgctagt cctgtcccag cttgctccca gcaacaagtc atccagcata 1500

ataccatcac tacttcctca tcggtcagcg aggtggtagg aagctccacc ctcagccagc 1560

tcaccactca cagaacagac ctgaatccga ttctttaaaa tgcaccatca gacctggcct 1620

ccaagaagag ctgtagcgta ccatgcgtcc tttcttttaa gggcattttt agaattaact 1680

cagacctgga agactcctca gttcttcaaa gactggcttt catttttata gttattatgg 1740

aaatgttgtc ttttatactt agttatataa gaaaaaaggg agttatgcaa ttaatatcta 1800

tcagcttggg aaacgctttg gtgcttttct ccagttttct ggtaccagtt acttgtttat 1860

aaactgaacc ttttctgtat atagccatgg tttcattctt atcagtccaa ccctttgcct 1920

gaaacattga atcttgttaa accacagctt ttagctaaaa tgaggtatac ctagatgtca 1980

agtaagacag atccaaggta actgggtagg aaatcttttg acatcttaac tcatgttgag 2040

tttgtgctgt ggtgtcacca gaattccaga taaacacaca gcctttccca tacctttttt 2100

tttcttacta taaaatatta taagatccat tgatgtccaa ataataccac caagcatctc 2160

ttcacctctc ctcctcttgg tccacttgct aatgcccagt tttcttctcc atttccactt 2220

tttcttaggc tccctattta ctattcattt tgacttcctt ctgttttatt tttttccctt 2280

tagcattgca tgtgaataag aaaataatgt ttaaagaaaa aaaaaaaaaa gcaaacctcc 2340

aaaacgtgga cctaaccatt gcttcactta cacttcaccc acagctggag ttcattcaac 2400

tcttgctttt cacaaaatag taaccaggag atgtttaatg tgcctgattt aatgttttta 2460

ataatcacag caaatgaaag gtggtttagt tataagtgaa gcatggttga ataccagctg 2520

gggagacact agggaaggga gctttgtaag ccttgattgc gaaagtccaa attttgatgt 2580

ggggctataa catgacaccc ttggattgcg actggtttta tacggcctgc ctataacgtt 2640

gaaaatccat gtactacata ataattcaga agggctctat tcactacaca gattacattg 2700

ttcaatcatc agctgctaat agcctaagat ttattttttt ttttttctta agcctatgga 2760

accggctttg ctgttctggg gggtgaaaat agactaacta ctggagaaac aaagagagaa 2820

agaaaaccca gtgtttccat aggggcactt ttagccttcc cacaacagtt aagcactctt 2880

tgactgctga aggaacccca tggatgaggt gcaggctact tcactctttt tttttctttt 2940

ttgagacaga gtctcaccta ttgcccagac tgaagtgcag tggtgcgatc atggatcact 3000

gcagcagcat cctccgagtt caagctatcc ttccacctcc gcctcctgag tagctgggac 3060
cacaggttca cataaccatg cctggctaat ttatttttac ttttatttta aaataaaaga 3120
tgaggtctgt cttatgttgc ccaggctggt ctcaaactat cctacttctt cctcccaaag 3180
tgttgggatt ataggtgtga gccactgcac ccagcctact tcactcttct gaattattct 3240
gatttatttt caacaacttt tgtgaacttg cccgtgatac aaagcagata gtccctgaac 3300
cacagtcgtg cctccttgaa acaagccatt ctactgtgct aatgttttaa tatcacatct 3360
cacaaataac aggggtgaat gtttctctct agcaatctag gcaggtgctg gtgtttcatc 3420
tccatttgaa tgcttgacct cttaatgtgt gtgtgtgtgt gtgtgtgtgt gtgtgtgttc 3480
atgggtttta aaagaacagt attttacaaa aggtgtagct tttataagag tgcagaaaag 3540
ggaaggatgt gttttttct ctcactatag tataagaatc tattttggag aaaaaaagaa 3600
aatatgaggg tctcgaagca tgatttttat ataactagtt tcagttttat ctaataactt 3660
actttttaaa tcaatattta tcaacaatct ttccttgtat gcagtgcttt caaaagatgg 3720
ttttgagtgt ccagtgaaac ttatgacttg gatatatggt tgaagaatca aaacaaaagc 3780
aaaaaaaaaa agcaaaaaaa gaaaagagaa aaaaagaaaa aatgcaaatg gaataatttt 3840
ctattatatt ttagacaaac atatcatttt cgagtatttt aaatactgaa ttcatagttg 3900
ttgtttttta aattccaaca gtaacagctg aatggtttaa tctgactggc ttcctaagaa 3960
atgtttaaga ctcagcttta aaaagaagtt aacattcata tctctgtttt gaaatcaaaa 4020
atcatatttc aaaattcttt cctaggacca tctatgtgtc tcccctcccc tccacaaaaa 4080
ggagaaagag tgcattaaaa tgtttagttg ggttttttaa tttttaattt ttatgttatg 4140
ttttgctttg ttttaagtaa acaaaaattt ttctttcttt actgcatgca tagcacttaa 4200
taaaatggat ttttaaaaaa tccactagta atatcagaat gtccagggag tgactgtcac 4260
tacaatgatg gtttagttta cttctgttcc accttttgat tgaaatattt agttgttagg 4320
ctgaaagcct cggcagttaa gaacttgcct gagttttctt cgttcagcaa cttgacagtt 4380
tgactgatgt gcattatata tagctcaatt atgtctgttt tttatgctaa gtaggaaaac 4440
caaccacaca cattagcaaa ccggcctcaa catataatta gaataaactg tcttcttgtt 4500
ctactcaggg cctttaggtg tgttcattca cggtatggaa atacagtaaa tgaaagattc 4560
caactagttg tcagtgcttc ttgaaattcc aaacagaaag atacattggt caaatccaac 4620
acttggctta tcaatattaa gtcttttacc taaaggccca gccgtcacca gacaacagaa 4680
taatcaatct gcctgaaaat ccctcctcct tgtcctacac tttttgcctg tttgggagaa 4740
tatctttgta ctccattctc ctccctcagc cagttactgg gtcacccatc catgtgttca 4800
tgaatcaatc atcacggcct gcagagcacc tgtcctaagg agggaaaatc ctgtcacact 4860

gcctctcccc attcgtgtgt ggttttcttg atcggtgaga tctgtctctg aagtcactgc 4920
cagcctccct gggaacgtct atagtgcctc ccctgcctta tgtgatggga gttaacaact 4980
cagataagta cacctgagag catttctatc aggtaaactg tcacttaaat ggaggtgtcc 5040
acatcttaat tgtttctcct tgacacattt ctcaatccac gaagccagga gaggtagagt 5100
gaaaatccca gccatggatg aatgtactaa tttgaaagcc aagtgttaag tcggatgttt 5160
tcccgttaca ctactactca gccctctcct gcggccacat caacggatgc aagtcacagt 5220
cttaacacag cctgtgggag acaagcagtt tgtgtgctca cagtatatat tatagtaatt 5280
agggtgactt agagcaaata ctcttcagat cctatgtagt cagtgaaaca aaatggagag 5340
cgtattctga tagaaggacg tcgacggtga atgttctggt ggttgttgcc tgttaagtaa 5400
actttagtgt gtaagttgag tttgtcatta aaatcataaa ccagctgcgg taacagacaa 5460
gcctttggct ggggagtttt aagcctcggt aactgctata aaactagcca tccagttagg 5520
atagaatgtg tttctttctg gttaaaaaaa ggaaaaacca tctaagaaaa tatatatgta 5580
tgtatgtgtg tatacagtgg aattcaaagg accaaagcaa aatttgaaca ggaatctatt 5640
aatttagaat tttataagat atttattaat aaatgttatt tttaaacatt ccatttgaac 5700
agtattctgt aggatctact tgtttttaaa gtgttagtcc ataataaact actatagtta 5760
tgtgtatttt catttttcag ggtttcaaat ggctattctc catcatttgg tggaaatgtt 5820
tgcttagatc tctgtgcata gacatttcaa ggatttttat tgctctgtga gttatttttt 5880
aatcaacatt ctgaacagtt ttttttaaac atttatttct gtgtgttcat ttttaaagta 5940
agctctttca tttaggaagc agagttcagc taaagggaat cagtaactct aactggaaca 6000
gctttcttgt agaagtgtaa aaacagcttc atctctgcct ctctccaccc caccccaatt 6060
tcctagaaag ccttgcacta ttcagctccc ttagtgcttt ttgtcccttc ccgaacaata 6120
tgcagtagct ttaagccatt caagctccat tatgcagtat atctgagaag ggaaaggaaa 6180
caacccattt aaatttgaat aaaaccgtgc ctatgcgaac agtagcaatt tagaatctct 6240
tttctgcttt taaaataatt tatatttaaa aattgcactt tagctttttg atccctttgt 6300
atttctctta ttctctttct aacctcttct ctgtcctcaa acttgccttt gctctccttt 6360
acaatacccc ccacccctcc tccaaggctc tgagcggcat catttaaaat actttacaga 6420
tatttgcacc aggtacattt atgtgcgtcc attggtagca cagctgagac ctgtgtctca 6480
catcagccta ggtgaagcct actacaagaa tgccaaggag aagagccagt acactatatg 6540
gtttatactc tttatccctt tattcatagc atgtttttta aaaatgttat attatgcaac 6600
agatgtgagg cagcagctaa gctatactta agaattttct ctcaccttcc aaaccaaagt 6660
gtcctgaata agccaggaga cttattcttt tgtgcaccct ggtgcacatc tgactgttgt 6720
cctagccata gactctctga ggccactgaa agaacagtgg ccctatcgat ttcattccta 6780

ggtctcaaaa atacaatgtt gccttgtaac ataattaggg acagcacctc tatttcacaa 6840

ttataatcta aggtaggata agacgacaca gcagcaataa acttacaagt aaaattcaat 6900

accaaaacaa acacaaagaa atttaaaaaa caaaaaacct agctcatcat gttgtgaaaa 6960

tgaaaaagtg aatgtccatt caaaatattt tactatttct tgtggagttt ttcagtgatg 7020

taatgcttgt agccaaattg cttaaagagt gtttatatat ttttttcctt ataaattgtc 7080

tattttttaa aaaagctatt taaccacagc tgaagtgggg ggtaaggcca aattgccaac 7140

acttgttaaa agattaatac tcttaagtgg cactctgata cctttccaac ttgtcatcag 7200

aaaggaatca ataattacca actgttgtat ttagaccaac ttacaatatc tagctcatta 7260

gaagccagga tctagaaagc tccttctaag ccatttaaga tattcttaca ttgagcttca 7320

tattatagaa ctttatagga ttggatattt tacaatagaa taatttagcc tcaggactga 7380

gaatgtggaa gctgaataaa ttagctttaa atacatcatt aaaatcttat gcacaataag 7440

ctcattagat tctagttttc tcctttagaa taccaatgcc acagacacta caggagataa 7500

tgaaaggtat cagttgtgtt gagtggaggg agtttaagag aaaggaccct tcccaaccag 7560

cagccagtag aaaatacaac ctactcacct ttttcccttc taagttctgc taaatcacat 7620

ctgcctcata gagaaaggaa tgttgccttt gagaactgtc ttggagaaca gataagcttg 7680

aaatgttctc tctagagagg acatagggtt tgggatcctc tgaaaaggcc cagaaaaata 7740

gctcagttca aatacaatgt tctaggacaa ttggaatata aatattgtcc aaaaatataa 7800

ttaaaagaaa aaagtttagc actgtgtaaa gtaagtgtta actgaggaag tcccaaaaag 7860

gtgctgtcac tttaagttct ggacttgggg ttctttgtat ttgtaaacag caaagcattt 7920

gtgtttgttt gtctatttgt aaagcaacca ccttccttat tggaaggaga aaaaaagggg 7980

tacatacatg taaatacttg ctgcagcatt taatatgttt aattttgtgt taagcttttt 8040

gttgcatcgt gaacacattt attgttacca atggacaatg agttcattaa gactgttcaa 8100

ctaggtcaga tttttacatc tctttctagc aagaagagac aagattttgt gcatttgtac 8160

aaatgttaat atcactgcaa ttccaatata ataaagcact caaatgcaaa taaaaaaaaa 8220

aaaaaaaaaa aaaaa 8235

<210> 16
<211> 602
<212> DNA
<213> Artificial Sequence

<220>
<223> Human VPREB3 mRNA sequence

<400> 16

```
cttcccagcc ctgtgcccca aagcacctgg agcatatagc cttgcagaac ttctacttgc     60
ctgcctccct gcctctggcc atggcctgcc ggtgcctcag cttccttctg atggggacct    120
tcctgtcagt ttcccagaca gtcctggccc agctggatgc actgctggtc ttcccaggcc    180
aagtggctca actctcctgc acgctcagcc cccagcacgt caccatcagg gactacggtg    240
tgtcctggta ccagcagcgg gcaggcagtg cccctcgata tctcctctac taccgctcgg    300
aggaggatca ccaccggcct gctgacatcc ccgatcgatt ctcggcagcc aaggatgagg    360
cccacaatgc ctgtgtcctc accattagtc ccgtgcagcc tgaagacgac gcggattact    420
actgctctgt tggctacggc tttagtccct aggggtgggg tgtgagatgg gtgcctcccc    480
tctgcctccc atttctgccc ctgaccttgg gtccctttta aactttctct gagccttgct    540
tcccctctgt aaaatgggtt aataatattc aacatgtcaa caacaaaaaa aaaaaaaaaa    600
aa                                                                   602
```

## Description

The present disclosure relates to a method of detecting active TB in the presence of a complicating factor, for example, latent TB and/or co-morbidities, such as those that present similar symptoms to TB. The disclosure also relates to a minimal gene signature employed in the said method and to a bespoke gene chip for use in the method. The disclosure further relates to use of gene chips and primer sets in the methods of the disclosure and kits comprising the elements required for performing the method. The disclosure also relates to use of the method to provide a composite expression score which can be used in the diagnosis of TB, particularly in a low resource setting.

## BACKGROUND

An estimated 8.8 million new cases and 1.45 million deaths are caused by Tuberculosis, TB (short for tubercle bacillus) each year (World Health Organisation statistics 2011). TB is an infectious disease caused by various species of mycobacteria, typically *Mycobacterium tuberculosis.* Tuberculosis usually attacks the lungs but can also affect other parts of the body. It is spread through the air when people who have an active TB infection cough, sneeze, or otherwise transmit their saliva. Most infections in humans result in an asymptomatic, latent infection, and about one in ten latent infections eventually progress to active disease, which, if left untreated, kills more than 50% of those infected. Immunosuppression and malnutrition are among the risk factors for developing active TB.

The classic symptoms are a chronic cough with blood-tinged sputum, fever, night sweats, and weight loss (the latter giving rise to the formerly prevalent colloquial term "consumption"). Infection of organs other than the lungs causes a wide range of symptoms. Treatment is difficult and requires long courses of multiple antibiotics. Antibiotic resistance is a growing problem with numbers of multi-drug-resistant tuberculosis cases on the rise. This is, in part, due to the length of treatment needed. Those infected with latent TB are typically asymptomatic and therefore either forget or decided not to take antibiotics. Those infected with active TB often cease treatment when the symptoms clear even though the infection remains.

Correct diagnosis is of utmost importance in the treatment of TB. The treatment regimens for active TB and latent TB are different and so it is important to diagnose the two conditions correctly in order to provide appropriate therapy.

Diagnosis of TB is particularly complicated as it cannot solely be based on symptoms. This is for two reasons: those infected with latent TB exhibit no symptoms and active TB may present similar symptoms to other infections or illnesses. Matters may be further complicated by the fact that TB may not be the only infection or illness that the patient has. Co-morbidities and co-infections often mask the symptoms of active TB and thus the latter goes undiagnosed and untreated. If active TB goes untreated the patient has a high probability of death due to the disease. Not only does TB present similar symptoms to other infectious or non-infectious conditions but it also presents similar radiological features. Thus, identifying the presence of TB definitively can be difficult.

Diagnosis is therefore multi-facetted, relying on clinical and radiological features (commonly chest X-rays), sputum microscopy (with or without culture), tuberculin skin test (TST), blood tests, as well as microscopic examination and microbiological culture of bodily fluids. In many places, such as Africa, which often do not have the resources needed to make a full diagnosis, this is a major impediment to tuberculosis treatment and control. Culture facilities are largely unavailable for TB diagnosis in most African hospitals.

All of the known methods of diagnosis have drawbacks, particularly in HIV co-infected persons in whom radiological features are often atypical:

- Sputum microscopy often has low sensitivity in HIV infected patients with TB because cavitatory lung disease is less common in this group, resulting in sputum negative microscopy (Schultz 2010).
- Tuberculin skin testing (TST) and Interferon Gamma Release Assays (IGRA) do not discriminate TB from latent TB infection (LTBI) and are of limited utility in African countries where LTBI is highly prevalent in the healthy population. In 2010 Metcalfe et al concluded that neither TST nor IGRA have value for active tuberculosis diagnosis in the context of HIV co-infection in low and middle income countries.
- Although molecular diagnosis has improved detection of M. *tuberculosis* DNA in sputum, the sensitivity of this approach is lower in smear negative samples, even if culture positive, and the method does not detect solely extra-pulmonary disease.

Consequently, a high proportion of active TB cases in sub-Saharan Africa remain undiagnosed, and post-mortem studies show TB to be a frequent, undiagnosed cause of death. Thus, there is an urgent need for improved diagnostic tests for TB, particularly in patients co-infected with HIV.

To meet this need, the present inventors previously developed a method for detecting active TB in a subject derived sample in the presence of a complicating factor, involving testing the expression levels in the genes within 3 different gene signatures. See WO2014/019977. They successfully devised a 27 gene signature for discriminating active TB from latent TB, a 44 gene signature for discriminating active TB from other diseases and a 53 gene signature for discriminating active TB from latent TB and other diseases. These gene signatures were demonstrated to detect active TB with a high degree of specificity and sensitivity.

However, despite the potential of these gene signatures, there is a need to further reduce the number of genes to be tested in the gene signatures, in order to further reduce costs, labour and time taken to analyse and obtain the test results especially in resource poor settings, such as remote villages in sub-Saharan Africa.

## SUMMARY OF THE INVENTION

The invention has been defined in the appended claims.

Embodiments in the following passages refer to embodiments of the disclosure which are not necessarily embodiments of the claimed invention. In particular the embodiments which concern the 3-gene signature do not form part of the claimed invention.

Accordingly, the present disclosure provides a method for detecting active tuberculosis (TB) in the presence of a complicating factor in a subject derived sample, comprising the step of detecting modulation in gene expression data, generated from RNA levels in the sample, of the genes in a signature selected from the group consisting of:

a) a 3 gene signature comprising *FCGR1A, ZNF296* and *C1QB* for discriminating active TB from latent TB infection;
b) a 6 gene signature comprising *GBP6, TMCC1, PRDM1, ARG1, CREB5* and *VPREB3* for discriminating active TB from other diseases; and
c) a combination of signatures a) and b).

Advantageously the present inventors developed a novel in-house analysis method called Forward Selection - Partial Least Squares (FS-PLS) and have used it to drastically reduce the original 44 gene signature to a 6 gene signature and the 27 gene signature to a 3 gene signature. They were further able to show that the 6 and 3 gene signatures were capable of detecting active TB with discriminatory power comparable to the original 44 and 27 gene signatures. Accordingly, the presently disclosed method provides the skilled person with the flexibility of using either original 44 gene or 27 gene signatures when a higher sensitivity/specificity is required or the reduced 6 or 3 gene signatures when a reduced number of genes to be tested is desirable.

In one embodiment, the complicating factor is the presence of a co-morbidity, for example wherein the co-morbidity is selected from malignancy, HIV, malaria, pneumonia, Lower Respiratory Tract Infection, Pneumocystis Jirovecii Pneumonia, pelvic inflammatory disease, Urinary Tract Infection, bacterial or viral meningitis, hepatobiliary disease, cryptococcal meningitis, non-TB pleural effusion, empyema, gastroenteritis, peritonitis, gastric ulcer and gastritis.

In one embodiment, wherein the co-morbidity is HIV.

In one embodiment 3 genes in the 6 gene signature are up-regulated, for example wherein the genes *PRDM1, GBP6 and CREB5* are up-regulated.

In one embodiment the remaining genes in the 6 gene signature are down-regulated, for example wherein the genes *VPREB3, ARG1* and *TMCC1* are down-regulated.

In one embodiment 2 genes in the 3 gene signature are up-regulated, for example wherein the gene *FCGR1A* and *C1QB* are up-regulated.

In one embodiment the remaining genes in the 3 gene signature are down-regulated, for example wherein the gene *ZNF296* is down-regulated.

In one embodiment the method further comprises the steps of:

a. optionally normalising and/or scaling numeric values of the modulation,
b. taking the normalised and/or scaled numeric values or the raw numeric values, each of which comprise both positive and/or negative numeric values and designating all said numeric values to be negative or alternatively all positive,
c. optionally refining the discriminatory power of one or more up-regulated genes and down-regulated genes by statistically weighting some of the numeric values associated therewith, and
d. summating the positive or negative numeric values obtained from step b) or step c) to provide a composite expression score,

wherein the composite expression score obtained from step d) is compared to a control and the comparison allows the sample to be designated as positive or negative for the relevant infection.

In one embodiment the gene signature further incorporates one or more such as 1, 2, 3, 4, or 5 housekeeping genes.

In one embodiment a patient derived sample is employed in the method.

In one embodiment the detection of gene expression modulation employs a microarray.
In one embodiment the detection of gene expression modulation employs PCR, such as RT-PCR.
In one embodiment the PCR is a multiplex PCR.
In one embodiment the PCR is quantitative.
In one embodiment primers employed in the PCR comprise a label or a combination of labels.
In one embodiment the label is fluorescent or coloured, for example coloured beads.
In one embodiment the detection of gene expression modulation employs a dual colour reverse transcriptase multiplex ligation dependent probe amplification.
In one embodiment the gene expression modulation is detected by employing fluorescence spectroscopy.
In one embodiment the gene expression modulation is detected by employing colourimetric analysis.
In one embodiment the gene expression modulation is detected by employing impedance spectroscopy.
In one embodiment the method comprises the further step of prescribing a treatment for the subject based on the results of the analysis of said gene signature.
In one embodiment the treatment is a treatment for active TB.
In one aspect, there is provided a set of primers for use in multiplex PCR wherein the set of primers includes nucleic acid sequences specific to a polynucleotide gene transcript for at least one gene from the group consisting of: *FCGR1A, ZNF296* and *C1QB;* and optionally includes nucleic acid sequences specific to a polynucleotide gene transcript for one or more genes selected from the group consisting of: *GBP6, TMCC1, PRDM1, ARG1, CREB5* and *VPREB3.*
In one embodiment the nucleic acid sequences in the set are for no more than a total of 6 genes, such as 2, 3, 4, 5, or 6 genes.
In one embodiment the set of primers, further comprises primers specific to one or more such as 1, 2, 3, 4, or 5 housekeeping genes.
In one embodiment the gene transcript is RNA, for example mRNA.
In one embodiment the primers for each gene are at least a pair of nucleic acid primer sequences.
In one embodiment the primer length is 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100 bases in length.
In one embodiment at least one primer for each gene comprises a label.
In one embodiment the labels on the primers are independently selected from selected from a fluorescent label, a coloured label, and antibody, step tag, his tag.
In one embodiment each primer in a given pair of primers is labelled, for example where one label quenches the fluorescence of the other label when said labels are within proximity of each other.
In one embodiment the primers are specific to a sequence given in any one of SEQ ID NOs: 1 to 16.
In one aspect, there is provided a point of care test for identifying active TB in a subject comprising the set of primers as described above.
In one aspect, there is provided the use of the set of primers described above in an assay to detect active TB infection in a sample, for example a blood sample.

## BRIEF DESCRIPTION OF THE FIGURES

**Figure 1A and B** - **Correlation plots of FS-PLS, Elastic Net and Lasso on the two INTERMAP Metabolomics datasets**
Black indicates a correlation coefficient of 1 whilst medium grey indicates a correlation coefficient of -1. FS-PLS selects uncorrelated predictors as indicated by the blue diagonals compared to lasso and elastic net that bring in the model correlated variables.
**Figure 1 - Simulation results**
Boxplots for RMSE/AUC/ACC (Figure 2A) and boxplots for number of variables selected (Figure 2B) for continuous outputs (a, e, i); discrete outputs with 2 classes (b, f, j); discrete outputs with 3 classes (c, g, k); and discrete outputs with 3 classes (d, h, I).
**Figure 3** - **Reduction of original 27 and 44 gene signatures to minimal 3 and 6 gene signatures**
Overview of Example 2 depicting the reduction of the original 27 and 44 gene signatures, derived using Elastic Net, to the new minimal 3 and 6 gene signatures by using FS-PLS.
**Figure 4** - **Correlation plots of FS-PLS and Elastic Net on the TB datasets**
Black indicates correlation coefficient of 1 whilst medium grey correlation represents a coefficient of -1. FS-PLS selects uncorrelated predictors as indicated by the blue diagonals compared to lasso and elastic net that bring in the model correlated variables.

**Figure 5 - Comparison of Receiver Operator Curves for 27 gene signature vs 3 gene signature**

27 gene signature (Elastic Net) and 3 gene signature (FS-PLS) applied to training cohort [80% of subjects from South African/Malawi HIV +/- patient group described in Kaforou et al (26)], test cohort [20% of subjects from South African/Malawi HIV +/- patient group) and Berry et al dataset (Nature 2010).

## DETAILED DESCRIPTION

In one embodiment of the present disclosure the gene signature is the minimum set of genes required to optimally detect the infection or discriminate the disease.

Optimally is intended to mean the smallest set of genes needed to detect active TB without significant loss of specificity and/or sensitivity of the signature's ability to detect or discriminate.

Detect or detecting as employed herein is intended to refer to the process of identifying an active TB infection in a sample, in particular through detecting modulation of the relevant genes in the signature.

Discriminate refers to the ability of the signature to differentiate between different disease status, for example latent and active TB. Detect and discriminate are interchangeable in the context of the gene signature.

In one embodiment the method is able to detect an active TB infection in a sample.

Subject as employed herein is a human suspected of TB infection from whom a sample is derived. The term patient may be used interchangeably although in one embodiment a patient has a morbidity.

In one embodiment the subject is an adult. Adult is defined herein as a person of 18 years of age or older.

In one embodiment the subject is a child. Child as employed herein refers to a person under the age of 18, such as 5 to 17 years of age.

Modulation of gene expression as employed herein means up-regulation or down-regulation of a gene or genes.

Up-regulated as employed herein is intended to refer to a gene transcript which is expressed at higher levels in a diseased or infected patient sample relative to, for example, a control sample free from a relevant disease or infection, or in a sample with latent disease or infection or a different stage of the disease or infection, as appropriate.

Down-regulated as employed herein is intended to refer to a gene transcript which is expressed at lower levels in a diseased or infected patient sample relative to, for example, a control sample free from a relevant disease or infection or in a sample with latent disease or infection or a different stage of the disease or infection.

The modulation is measured by measuring levels of gene expression by an appropriate technique. Gene expression as employed herein is the process by which information from a gene is used in the synthesis of a functional gene product. These products are often proteins, but in non-protein coding genes such as ribosomal RNA (rRNA), transfer RNA (tRNA) or small nuclear RNA (snRNA) genes, the product is a functional RNA. That is to say, RNA with a function.

Gene expression data as employed herein is intended to refer to any data generated from a patient sample that is indicative of the expression of the two or more genes, for example 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50.

A complicating factor as employed herein refers to at least one clinical status or at least one medical condition that would generally render it more difficult to identify the presence of active TB in the sample, for example a latent TB infection or a co-morbidity.

Co-morbidity as employed herein refers the presence of one or more disorders or diseases in addition to TB, for example malignancy such as cancer or co-infection. Co-morbidity may or may not be endemic in the general population.

In one embodiment the co-morbidity is a co-infection.

Co-infection as employed herein refers to bacterial infection, viral infection such as HIV, fungal infection and/or parasitic infection such as malaria. HIV infection as employed herein also extends to include AIDS.

In one embodiment other disease (OD) is a co-morbidity. The 6 gene signature comprising *GBP6, TMCC1, PRDM1, ARG1, CREB5* and *VPREB3* is able to detect active TB in the presence of a co-morbidity such as a co-infection and is able to discriminate active TB from other diseases. This is despite the increased inflammatory response of the patient to said other infection.

In one embodiment co-morbidity is selected from malignancy, HIV, malaria, pneumonia, Lower Respiratory Tract Infection, Pneumocystis Jirovecii Pneumonia, pelvic inflammatory disease, Urinary Tract Infection, bacterial or viral meningitis, hepatobiliary disease, cryptococcal meningitis, non-TB pleural effusion, empyema, gastroenteritis, peritonitis, gastric ulcer and gastritis.

In one embodiment malignancy is a neoplasia, such as bronchial carcinoma, lymphoma, cervical carcinoma ovarian carcinoma, mesothelioma, gastric carcinoma, metastatic carcinoma, benign salivary tumour, dermatological tumour or Kaposi's sarcoma.

The 3 gene signature comprising *FCGR1A, ZNF296* and *C1QB* is useful in discriminating active TB infection from latent TB infection.

Active TB as employed herein refers to a person who is infected with TB which is not latent.

In one embodiment active TB is where the disease is progressing as opposed to where the disease is latent.

In one embodiment a person with active TB is capable of spreading the infection to others.

In one embodiment a person with active TB has one or more of the following: a skin test or blood test result indicating TB infection, an abnormal chest x-ray, a positive sputum smear or culture, active TB bacteria in his/her body, feels sick and may have symptoms such as coughing, fever, and weight loss.

In one embodiment a person with active TB has one or more of the following symptoms: coughing, bloody sputum, fever and/or weight loss.

In one embodiment the active TB infection is pulmonary and/or extra-pulmonary.

Pulmonary as employed herein refers to an infection in the lungs.

Extra-pulmonary as employed herein refers to infection outside the lungs, for example, infection in the pleura, infection in the lymphatic system, infection in the central nervous system, infection in the genito-urinary tract, infection in the bones, infection in the brain and/or infection in the kidneys.

Symptoms of pulmonary TB include: a persistent cough that brings up thick phlegm, which may be bloody; breathlessness, which is usually mild to begin with and gradually gets worse; weight loss; lack of appetite; a high temperature of 38°C (100.4°F) or above; extreme tiredness; and a sense of feeling unwell.

Symptoms of lymph node TB include: persistent, painless swelling of the lymph nodes, which usually affects nodes in the neck, but swelling can occur in nodes throughout your body; over time, the swollen nodes can begin to release a discharge of fluid through the skin.

Symptoms of skeletal TB include: bone pain; curving of the affected bone or joint; loss of movement or feeling in the affected bone or joint and weakened bone that may fracture easily.

Symptoms of gastrointestinal TB include: abdominal pain; diarrhoea and anal bleeding.

Symptoms of genitourinary TB include: a burning sensation when urinating; blood in the urine; a frequent urge to pass urine during the night and groin pain.

Symptoms of central nervous system TB include: headaches; being sick; stiff neck; changes in your mental state, such as confusion; blurred vision and fits.

Latent TB as employed herein refers to a subject who is infected with TB but is asymptomatic. A sputum test will generally be negative and the infection cannot be spread to others.

In one embodiment a person with latent TB infection has one of more of the following: a skin test or blood test result indicating TB infection, a normal chest x-ray and a negative sputum test, TB bacteria in his/her body that are alive, but inactive, does not feel sick, cannot spread TB bacteria to others

In one embodiment a person with latent TB needs treatment to prevent TB disease becoming active.

In one embodiment the method of the present disclosure is able to differentiate TB from different conditions/diseases or infections which have similar clinical symptoms.

Similar symptoms as employed herein includes one or more symptoms from pulmonary TB, lymph node TB, skeletal TB, gastrointestinal TB, genitourinary TB and/or central nervous system TB.

In one embodiment the method according to the present disclosure is performed on a subject with acute infection.

In a further embodiment the sample is a subject sample from a febrile subject, that is to say a subject with a temperature above the normal body temperature of 37.5°C.

In one embodiment the genes employed have identity with genes listed in the relevant tables, such as Table 3 and 4.

In one embodiment the 6 gene signature comprises or consists of at least up-regulated genes *PRDM1, GBP6 and CREB5.*

In one embodiment the 6 gene signature comprises or consists of at least down-regulated genes *VPREB3, ARG1* and *TMCC1.*

In one embodiment the 6 gene signature comprises or consists of at least up-regulated genes *PRDM1, GBP6* and *CREB5,* and down-regulated genes *VPREB3, ARG1* and *TMCC1.*

In one embodiment the 3 gene signature comprises or consists of at least up-regulated genes *FCGR1A* and *C1QB.*

In one embodiment the 3 gene signature comprises or consists of at least down-regulated gene *ZNF296.*

In one embodiment 3 gene signature comprises or consists of at least up-regulated genes *FCGR1A* and *C1QB* and down-regulated gene *ZNF296.*

In one embodiment the 3 and 6 gene signatures are tested in parallel.

In one embodiment one or more, for example 1 to 21, such as 2, 3, 4, 5, 6, 7, 8, 9, 10, 11, 12, 13, 14, 15, 16, 17, 18, 19 or 20, genes are replaced by a gene with an equivalent function provided the signature retains the ability to detect/discriminate the relevant clinical status without significant loss in specificity and/or sensitivity.

In one embodiment the gene signature is based on two genes of primary importance. Of primary importance as used herein means that the gene expression levels of the two genes is representative of the gene expression levels of other genes. For example, the expression levels of the first gene of primary importance may be highly correlated with the expression levels of a first group of genes, whilst the expression levels of the second gene of primary importance may be highly correlated with the expression levels of a second group of genes.

Therefore, each gene of primary importance may be used as a representative of the other highly correlated genes from their respective groups, thereby eliminating the need to test all of the genes within each group. In other words, testing the expression levels of just the two genes of primary importance provides a similar sensitivity and/or specificity as testing the expression levels of all of the genes.

In one embodiment each of the genes in the 3, 6 gene signatures is significantly differentially expressed in the sample with active TB compared to a comparator group.

Significantly differentially expressed as employed herein means the sample with active TB shows a log2 fold change >0.5 compared to the comparator group.

In one embodiment, in the 3 gene signature the comparator group is LTBI.

In one embodiment, in the 6 gene signature the comparator group is a person with "other disease" (OD), that is a disease that is not active TB but has similar symptoms.

"Presented in the form of" as employed herein refers to the laying down of genes from one or more of the signatures in the form of probes on a microarray.

Accurately and robustly as employed herein refers to the fact that the method can be employed in a practical setting, such as Africa, and that the results of performing the method properly give a high level of confidence that a true result is obtained.

High confidence is provided by the method when it provides few results that are false positives (i.e. the result suggests that the subject has active TB when they do not) and also has few false negatives (i.e. the result suggest that the subject does not have active TB when they do).

High confidence would include 90% or greater confidence, such as 91, 92, 93, 94, 95, 96, 97, 98, 99 or 100% confidence when an appropriate statistical test is employed.

In one embodiment the method provides a sensitivity of 80% or greater such as 90% or greater in particular 95% or greater, for example where the sensitivity is calculated as below:

$$\text{sensitivity} = \frac{\text{number of true positives}}{\text{number of true positives} + \text{number of false negatives}}$$

$$= \text{probability of a positive test given that the patient is ill}$$

In one embodiment the method provides a high level of specificity, for example 80% or greater such as 90% or greater in particular 95% or greater, for example where specificity is calculated as shown below:

$$\text{specificity} = \frac{\text{number of true negatives}}{\text{number of true negatives} + \text{number of false positives}}$$

$$= \text{probability of a negative test given that the patient is well}$$

In one embodiment the sensitivity of method of the 3 gene signature is 85 to 100%, such as 84, 85, 86, 87, 88, 89, 90, 91, 92, 93, 94, 95, 96, 97, 98 or 99%.

In one embodiment the specificity of the method of the 3 gene signature is 85 to 100%, such as 84, 85, 86, 87, 88, 89, 90, 91, 92, 93, 94, 95, 96, 97, 98 or 99%.

In one embodiment the sensitivity of the method of the 6 gene signature is 85 to 100%, such as 84, 85, 86, 87, 88, 89, 90, 91, 92, 93, 94, 95, 96, 97, 98 or 99%.

In one embodiment the specificity of the method of the 6 gene signature is 85 to 100%, such as 84, 85, 86, 87, 88, 89, 90, 91, 92, 93, 94, 95, 96, 97, 98 or 99%.

Thus in one embodiment DNA or RNA, in particular mRNA from the subject sample is analysed. In one embodiment the sample is solid or fluid, for example blood or serum or a processed form of any one of the same.

A fluid sample as employed herein refers to liquids originating from inside the bodies of living people. They include fluids that are excreted or secreted from the body as well as body water that normally is not. Includes amniotic fluid, aqueous humour and vitreous humour, bile, blood serum, breast milk, cerebrospinal fluid, cerumen (earwax), chyle, endolymph and perilymph, gastric juice, mucus (including nasal drainage and phlegm), sputum, peritoneal fluid, pleural fluid, saliva, sebum (skin oil), semen, sweat, tears, vaginal secretion, vomit, urine. Particularly blood and serum.

Blood as employed herein refers to whole blood, that is serum, blood cells and clotting factors, typically peripheral whole blood.

Serum as employed herein refers to the component of whole blood that is not blood cells or clotting factors. It is plasma with fibrinogens removed.

In one embodiment the subject derived sample is a blood sample.

In one or more embodiments the analysis is *ex vivo.*

In one embodiment the sample is whole blood. Hence in one embodiment the RNA sample is derived from whole blood.

The RNA sample may be subjected to further amplification by PCR, such as whole genome amplification in order to increase the amount of starting RNA template available for analysis.

Alternatively, the RNA sample may be converted into cDNA by reverse transcriptase, such as HIV-1 reverse transcriptase, moloney murine leukaemia virus (M-MLV) reverse transcriptase, AMV reverse transcriptase and telomersease reverse transcriptase. Such amplification steps may be necessary for smaller sample volumes, such as blood samples obtained from children.

*Ex vivo* as employed herein means that which takes place outside the body.

There are a number of ways in which gene expression can be measured including microarrays, tiling arrays, DNA or RNA arrays for example on gene chips, RNA-seq and serial analysis of gene expression.

Any suitable method of measuring gene modulation may be employed in the method of the present disclosure.

Polymerase chain reaction (PCR) as employed herein refers to a widely used molecular technique to make multiple copies of a target DNA sequence. The method relies on thermal cycling, consisting of cycles of repeated heating and cooling of the reaction for DNA melting and enzymatic replication of the DNA. Primers containing sequences complementary to the target region along with a DNA polymerase, which the method is named after, are key components to enable selective and repeated amplification. As PCR progresses, the DNA generated is itself used as a template for replication, setting in motion a chain reaction in which the DNA template is exponentially amplified.

Multiplex PCR as employed herein refers to the use of a polymerase chain reaction (PCR) to amplify two or more different DNA sequences simultaneously, i.e. as if performing many separate PCR reactions together in one reaction.

Primer as employed herein is intended to refer to a short strand of nucleic acid sequence, usually a chemically synthesised oligonucleotide, which serve as a starting point for DNA synthesis reactions. Primers are typically about 15 base pairs long but can vary from 5 to 100 bases long. It is required in processes such as PCR because DNA polymerases can only add new nucleotides or base pairs to an existing strand of DNA. During a PCR reaction, the primer hybridises to its complementary sequence in a DNA sample. Next, DNA polymerase starts replication at the 3'end of the primer and extends the primer by copying the sequence of the opposite DNA strand.

In one embodiment the primers of the present disclosure are specific for RNA, such as mRNA, i.e. they are complementary to RNA sequences. In another embodiment, the primers are specific for cDNA, i.e. they are complementary to cDNA sequences.

In one embodiment the primers of the present disclosure comprise a label which enables the primers to be detected or isolated. Examples of labels include but are not limited to a fluorescent label, a coloured label, and antibody, step tag, his tag.

In another embodiment, each primer in a given pair of primers is labelled, for example where one label (also known as a quencher) quenches the fluorescence of the other label when said labels are within proximity of each other. Such labels are particularly useful in real time PCR reactions for example. Examples of such label pairs include 6-carboxyfluorescein (FAM) and tetrachlorofluorescein, or tetramethylrhodamine and tetrachlorofluorescein.

Point of care test or bedside test as used herein is intended to refer to a medical diagnostic test which is conducted at or near the point of care, i.e. at the time and place of patient care. This is in contrast with a conventional diagnostic test which is typically confined to the medical laboratory and involves sending specimens away from the point of care to the laboratory for testing. Such diagnostic tests often require many hours or days before the results of the test can be received. In the meantime, patient care must continue without knowledge of the test results. In comparison, a point of care test is typically a simple medical test that can be performed rapidly.

In one embodiment the gene expression data is generated from a microarray, such as a gene chip.

In one aspect of the disclosure there is provided a gene chip comprising one or more of the gene signatures selected from the group consisting of:

a) a 3 gene signature comprising *FCGR1A, ZNF296* and *C1QB;*

b) a 6 gene signature comprising *GBP6, TMCC1, PRDM1, ARG1, CREB5* and *VPREB36;* and optionally;

c) one or more house-keeping genes.

In a further aspect the present disclosure includes use of a known or commercially available gene chip in the method of the present disclosure.

Advantageously the different expression patterns represented by the gene signatures employed in the method of the present disclosure correlate across geographic location and HIV infected status (i.e. positive or negative). That is to say, the method is applicable to different geographic locations regardless of the presence or absence of HIV.

Microarray as employed herein includes RNA or DNA arrays, such as mRNA arrays.

A gene chip is essentially a microarray that is to say an array of discrete regions, typically nucleic acids, which are separate from one another and are, for example arrayed at a density of between, about 100/cm$^2$ to 1000/cm$^2$, but can be arrayed at greater densities such as 10000/cm$^2$.

The principle of a microarray experiment, is that mRNA from a given cell line or tissue is used to generate a labelled sample typically labelled cDNA or cRNA, termed the 'target', which is hybridised in parallel to a large number of, nucleic acid sequences, typically DNA or RNA sequences, immobilised on a solid surface in an ordered array. Tens of thousands of transcript species can be detected and quantified simultaneously. Although many different microarray systems have been developed the most commonly used systems today can be divided into two groups.

Using this technique, arrays consisting of more than 30,000 cDNAs can be fitted onto the surface of a conventional microscope slide. For oligonucleotide arrays, short 20-25mers are synthesised in situ, either by photolithography onto silicon wafers (high-density-oligonucleotide arrays from Affymetrix) or by ink-jet technology (developed by Rosetta Inpharmatics and licensed to Agilent Technologies).

Alternatively, pre-synthesised oligonucleotides can be printed onto glass slides. Methods based on synthetic oligonucleotides offer the advantage that because sequence information alone is sufficient to generate the DNA to be arrayed, no time-consuming handling of cDNA resources is required. Also, probes can be designed to represent the most unique part of a given transcript, making the detection of closely related genes or splice variants possible. Although short oligonucleotides may result in less specific hybridization and reduced sensitivity, the arraying of pre-synthesised longer oligonucleotides (50-100mers) has recently been developed to counteract these disadvantages.

In one embodiment the gene chip is an off the shelf, commercially available chip, for example HumanHT-12 v4 Expression BeadChip Kit, available from Illumina, NimbleGen microarrays from Roche, Agilent, Eppendorf and Genechips from Affymetrix such as HU-UI 33.Plus 2.0 gene chips.

In an alternate embodiment the gene chip employed in the present invention is a bespoke gene chip, that is to say the chip contains only the target genes which are relevant to the desired profile. Custom made chips can be purchased from companies such as Roche, Affymetrix and the like. In yet a further embodiment the bespoke gene chip comprises a minimal disease specific transcript set.

In one embodiment the chip comprises or consists of the genes in the 6 gene signature comprising *GBP6, TMCC1, PRDM1, ARG1, CREB5* and *VPREB36.*

In one embodiment the chip comprises or consists of the genes in the 3 gene signature comprising *FCGR1A, ZNF296* and *C1QB.*

In one embodiment the chip comprises or consists of the genes in the 6 gene signature in combination with the genes in the 3 gene signature.

In one embodiment the following Illumina transcript ID number probes are used to detect the modulation in gene expression levels: ILMN_2176063 for *FCGR1A,* ILMN_1693242 for *ZNF296,* ILMN_1796409 for *C1QB,* ILMN_2294784 for *PRDM1,* ILMN_1756953 for *GBP6,* ILMN_1728677 for *CREB5,* ILMN_1700147 for *VPREB3,* ILMN_1812281 for *ARG1* and ILMN_1677963 for *TMCC1.*

In one or more embodiments above the chip may further include 1 or more, such as 1 to 10, house-keeping genes.

In one embodiment the gene expression data is generated in solution using appropriate probes for the relevant genes.

Probe as employed herein is intended to refer to a hybridisation probe which is a fragment of DNA or RNA of variable length (usually 100-1000 bases long) which is used in DNA or RNA samples to detect the presence of nucleotide sequences (the DNA target) that are complementary to the sequence in the probe. The probe thereby hybridises to single-stranded nucleic acid (DNA or RNA) whose base sequence allows probe-target base pairing due to complementarity between the probe and target.

In one embodiment the method according to the present disclosure and for example chips employed therein may comprise one or more house-keeping genes. House-keeping genes as employed herein is intended to refer to genes that are not directly relevant to the profile for identifying the disease or infection but are useful for statistical purposes and/or quality control purposes, for example they may assist with normalising the data, in particular a housekeeping gene is a constitutive gene i.e. one that is transcribed at a relatively constant level. The housekeeping gene's products are typically needed for maintenance of the cell.

Examples of housekeeping genes include but are not limited to actin, *GAPDH,* ubiquitin, 18s rRNA, *RPII (POLR2A), TBP, PPIA, GUSB, HSPCB, YWHAZ, SDHA, RPS13, HPRT1 and B4GALT6.*

In one embodiment minimal disease specific transcript set as employed herein means the minimum number of genes need to robustly identify the target disease state.

Minimal discriminatory gene set is interchangeable with minimal disease specific transcript set.

Normalising as employed herein is intended to refer to statistically accounting for background noise by comparison of data to control data, such as the level of fluorescence of house-keeping genes, for example fluorescent scanned data may be normalized using RMA to allow comparisons between individual chips. Irizarry et al 2003 describes this method.

Scaling as employed herein refers to boosting the contribution of specific genes which are expressed at low levels or have a high fold change but still relatively low fluorescence such that their contribution to the diagnostic signature is increased.

Fold change is often used in analysis of gene expression data in microarray and RNA-Seq experiments, for measuring change in the expression level of a gene and is calculated simply as the ratio of the final value to the initial value i.e. if the initial value is A and final value is B, the fold change is B/A. Tusher et al 2001.

In programs such as Arrayminer, fold change of gene expression can be calculated. The statistical value attached to the fold change is calculated and is the more significant in genes where the level of expression is less variable between subjects in different groups and, for example where the difference between groups is larger.

The step of obtaining a suitable sample from the subject is a routine technique, which involves taking a blood sample. This process presents little risk to donors and does not need to be performed by a doctor but can be performed by appropriately trained support staff. In one embodiment the sample derived from the subject is approximately 2.5 ml of blood, however smaller volumes can be used for example 0.5-1ml.

Blood or other tissue fluids are immediately placed in an RNA stabilizing buffer such as included in the Pax gene tubes, or Tempus tubes.

If storage is required then it should usually be frozen within 3 hours of collections at -80°C.

In one embodiment the gene expression data is generated from RNA levels in the sample.

For microarray analysis the blood may be processed using a suitable product, such as PAX gene blood RNA extraction kits (Qiagen).

Total RNA may also be purified using the Tripure method - Tripure extraction (Roche Cat. No. 1 667 165). The manufacturer's protocols may be followed. This purification may then be followed by the use of an RNeasy Mini kit - clean-up protocol with DNAse treatment (Qiagen Cat. No. 74106).

Quantification of RNA may be completed using optical density at 260nm and Quant-IT RiboGreen RNA assay kit (Invitrogen - Molecular probes RI 1490). The Quality of the 28s and 18s ribosomal RNA peaks can be assessed by use of the Agilent bioanalyser.

In another embodiment the method further comprises the step of amplifying the RNA. Amplification may be performed using a suitable kit, for example TotalPrep RNA Amplification kits (Applied Biosystems).

In one embodiment an amplification method may be used in conjunction with the labelling of the RNA for microarray analysis. The Nugen 3' ovation biotin kit (Cat: 2300-12, 2300-60).

The RNA derived from the subject sample is then hybridised to the relevant probes, for example which may be located on a chip. After hybridisation and washing, where appropriate, analysis with an appropriate instrument is performed.

In performing an analysis to ascertain whether a subject presents a gene signature indicative of disease or infection according to the present disclosure, the following steps are performed: obtain mRNA from the sample and prepare nucleic acids targets, hybridise to the array under appropriate conditions, typically as suggested by the manufactures of the microarray (suitably stringent hybridisation conditions such as 3X SSC, 0.1% SDS, at 50 °C) to bind corresponding probes on the array, and wash if necessary to remove unbound nucleic acid targets and analyse the results.

In one embodiment the readout from the analysis is fluorescence.

In one embodiment the readout from the analysis is colorimetric.

In one embodiment physical detection methods, such as changes in electrical impedance, nanowire technology or microfluidics may be used.

In one embodiment there is provided a method which further comprises the step of quantifying RNA from the subject sample.

If a quality control step is desired, software such as Genome Studio software may be employed. Numeric value as employed herein is intended to refer to a number obtained for each relevant gene, from the analysis or readout of the gene expression, for example the fluorescence or colorimetric analysis. The numeric value obtained from the initial analysis may be manipulated, corrected and if the result of the processing is a still a number then it will be continue to be a numeric value.

By converting is meant processing of a negative numeric value to make it into a positive value or processing of a positive numeric value to make it into a negative value by simple conversion of a positive sign to a negative or vice versa.

Analysis of the subject-derived sample will for the genes analysed will give a range of numeric values some of which are positive (preceded by + and in mathematical terms considered greater than zero) and some of which are negative (preceded by - and in strict mathematical terms are considered to less than zero). The positive and negative in the context of gene expression analysis is a convenient mechanism for representing genes which are up-regulated and genes which are down regulated.

In the method of the present disclosure either all the numeric values of genes which are down-regulated and represented by a negative number are converted to the corresponding positive number (i.e. by simply changing the sign) for example -1 would be converted to 1 or all the positive numeric values for the up-regulated genes are converted to the corresponding negative number.

The present inventors have established that this step of rendering the numeric values for the gene expressions positive or alternatively all negative allows the summating of the values to obtain a single value that is indicative of the presence of disease or infection or the absence of the same.

This is a huge simplification of the processing of gene expression data and represents a practical step forward thereby rendering the method suitable for routine use in the clinic.

By discriminatory power is meant the ability to distinguish between a TB infected and a non-infected sample (subject) or between active TB infection and other infections (such as HIV) in particular those with similar symptoms or between a latent infection and an active infection.

The discriminatory power of the method according to the present disclosure may, for example, be increased by attaching greater weighting to genes which are more significant in the signature, even if they are expressed at low or lower absolute levels.

As employed herein, raw numeric value is intended to, for example refer to unprocessed fluorescent values from the gene chip, either absolute fluorescence or relative to a house keeping gene or genes.

Summating as employed herein is intended to refer to act or process of adding numerical values.

Composite expression score as employed herein means the sum (aggregate number) of all the individual numerical values generated for the relevant genes by the analysis, for example the sum of the fluorescence data for all the relevant up and down regulated genes. The score may or may not be normalised and/or scaled and/or weighted.

In one embodiment the composite expression score is normalised.

In one embodiment the composite expression score is scaled.

In one embodiment the composite expression score is weighted.

Weighted or statistically weighted as employed herein is intended to refer to the relevant value being adjusted to more appropriately reflect its contribution to the signature.

In one embodiment the method employs a simplified risk score as employed in the examples herein.

Simplified risk score is also known as disease risk score (DRS).

Control as employed herein is intended to refer to a positive (control) sample and/or a negative (control) sample which, for example is used to compare the subject sample to, and/or a numerical value or numerical range which has been defined to allow the subject sample to be designated as positive or negative for disease/infection by reference thereto.

Positive control sample as employed herein is a sample known to be positive for the pathogen or disease in relation to which the analysis is being performed, such as active TB.

Negative control sample as employed herein is intended to refer to a sample known to be negative for the pathogen or disease in relation to which the analysis is being performed.

In one embodiment the control is a sample, for example a positive control sample or a negative control sample, such as a negative control sample.

In one embodiment the control is a numerical value, such as a numerical range, for example a statistically determined range obtained from an adequate sample size defining the cut-offs for accurate distinction of disease cases from controls.

**Conversion of multi-gene transcript disease signatures into a single number disease score**

Once the RNA expression signature of the disease has been identified by variable selection, the transcripts are separated based on their up- or down-regulation relative to the comparator group. The two groups of transcripts are selected and collated separately.

**Summation of up-regulated and down-regulated RNA transcripts**

To identify the single disease risk score for any individual patient, the raw intensities, for example fluorescent intensities (either absolute or relative to housekeeping standards) of all the up-regulated RNA transcripts associated with the disease are summated. Similarly summation of all down-regulated transcripts for each individual is achieved by combining the raw values (for example fluorescence) for each transcript relative to the unchanged housekeeping gene standards. Since the transcripts have various levels of expression and respectively their fold changes differ as well, instead of summing the raw expression values, they can be scaled and normalised between 0,1. Alternatively they can be weighted to allow important genes to carry greater effect. Then, for every sample the expression values of the signature's transcripts are summated, separately for the up- and down- regulated transcripts.

The total disease score incorporating the summated fluorescence of up- and down-regulated genes is calculated by adding the summated score of the down-regulated transcripts (after conversion to a positive number) to the summated score of the up-regulated transcripts, to give a single number composite expression score. This score maximally distinguishes the cases and controls and reflects the contribution of the up- and down- regulated transcripts to this distinction.

**Comparison of the disease risk score in cases and controls**

The composite expression scores for patients and the comparator group may be compared, in order to derive the means and variance of the groups, from which statistical cut-offs are defined for accurate distinction of cases from controls. Using the disease subjects and comparator populations, sensitivities and specificities for the disease risk score may be calculated using, for example a Support Vector Machine and internal elastic net classification.

Disease risk score as employed herein is an indicator of the likelihood that patient has active TB when comparing their composite expression score to the comparator group's composite expression score.

**Development of the disease risk score into a simple clinical test for disease severity or disease risk prediction**

The approach outlined above in which complex RNA expression signatures of disease or disease processes are converted into a single score which predicts disease risk can be used to develop simple, cheap and clinically applicable tests for disease diagnosis or risk prediction.

The procedure is as follows: For tests based on differential gene expression between cases and controls (or between different categories of cases such as severity), the up- and down- regulated transcripts identified as relevant may be printed onto a suitable solid surface such as microarray slide, bead, tube or well.

Up-regulated transcripts may be co-located separately from down-regulated transcripts either in separate wells or separate tubes. A panel of unchanged housekeeping genes may also be printed separately for normalisation of the results.

RNA recovered from individual patients using standard recovery and quantification methods (with or without amplification) is hybridised to the pools of up- and down-regulated transcripts and the unchanged housekeeping transcripts.

Control RNA is hybridised in parallel to the same pools of up- or down-regulated transcripts.

Total value, for example fluorescence for the subject sample and optionally the control sample is then read for up- and down- regulated transcripts and the results combined to give a composite expression score for patients and controls, which is/are then compared with a reference range of a suitable number of healthy controls or comparator subjects.

**Correcting the detected signal for the relative abundance of RNA species in the subject sample**

The details above explain how a complex signature of many transcripts can be reduced to the minimum set that is maximally able to distinguish between patients and other phenotypes. For example, within the up-regulated transcript set, there will be some transcripts that have a total level of expression many fold lower than that of others. However, these transcripts may be highly discriminatory despite their overall low level of expression. The weighting derived from the elastic net coefficient can be included in the test, in a number of different ways. Firstly, the number of copies of individual transcripts included in the assay can be varied. Secondly, in order to ensure that the signal from rare, important transcripts are not swamped by that from transcripts expressed at a higher level, one option would be to select probes for a test that are neither overly strongly nor too weakly expressed, so that the contribution of multiple probes is maximised. Alternatively, it may be possible to adjust the signal from low-abundance transcripts by a scaling factor.

Whilst this can be done at the analysis stage using current transcriptomic technology as each signal is measured separately, in a simple colorimetric test only the total colour change will be measured, and it would not therefore be possible to scale the signal from selected transcripts. This problem can be circumnavigated by reversing the chemistry usually associated with arrays. In conventional array chemistry, the probes are coupled to a solid surface, and the amount of biotin-labelled, patient-derived target that binds is measured. Instead, we propose coupling the biotin-labelled cRNA derived from the patient to an avidin-coated surface, and then adding DNA probes coupled to a chromogenic enzyme via an adaptor system. At the design and manufacturing stage, probes for low-abundance but important transcripts are coupled to greater numbers, or more potent forms of the chromogenic enzyme, allowing the signal for these transcripts to be 'scaled-up' within the final single-channel colorimetric readout. This approach would be used to normalise the relative input from each probe in the up-regulated, down-regulated and housekeeping channels of the kit, so that each probe makes an appropriately weighted contribution to the final reading, which may take account of its discriminatory power, suggested by the weights of variable selection methods.

The detection system for measuring multiple up or down regulated genes may also be adapted to use rTPCR to detect the transcripts comprising the diagnostic signature, with summation of the separate pooled values for up and down regulated transcripts, or physical detection methods such as changes in electrical impedance. In this approach, the transcripts in question are printed on nanowire surfaces or within microfluidic cartridges, and binding of the corresponding ligand for each transcript is detected by changes in impedance or other physical detection system

The present disclosure extends to a custom made chip comprising a minimal discriminatory gene set for diagnosis of active TB from other conditions, in particular those with similar symptoms, for example comprising probes specific for the genes in the 6 gene signature and/or 3 gene signature.

In one embodiment the gene chip is a fluorescent gene chip that is to say the readout is fluorescence. Fluorescence as employed herein refers to the emission of light by a substance that has absorbed light or other electromagnetic radiation.

Thus in an alternate embodiment the gene chip is a colorimetric gene chip, for example colorimetric gene chip uses microarray technology wherein avidin is used to attach enzymes such as peroxidase or other chromogenic substrates to the biotin probe currently used to attach fluorescent markers to DNA. The present disclosure extends to a microarray chip adapted to read by colorimetric analysis and adapted for the analysis of active TB infection in a patient. The present disclosure also extends to use of a colorimetric chip to analyse a subject sample for active TB infection.

Colorimetric as employed herein refers to as assay wherein the output is in the human visible spectrum.

In an alternative embodiment, a gene set indicative of active TB may be detected by physical detection methods including nanowire technology, changes in electrical impedance, or microfluidics.

The readout for the assay can be converted from a fluorescent readout as used in current microarray technology into a simple colorimetric format or one using physical detection methods such as changes in impedance, which can be read with minimal equipment. For example, this is achieved by utilising the Biotin currently used to attach fluorescent markers to DNA. Biotin has high affinity for avidin which can be used to attach enzymes such as peroxidase or other chromogenic substrates. This process will allow the quantity of cRNA binding to the target transcripts to be quantified using a chromogenic process rather than fluorescence. Simplified assays providing yes/no indications of disease status can then be developed by comparison of the colour intensity of the up- and down-regulated pools of transcripts with control colour standards. Similar approaches can enable detection of multiple gene signatures using physical methods such as changes in electrical impedance.

This aspect of the invention is likely to be particularly advantageous for use in remote or under-resourced settings or for rapid diagnosis in "near patient" tests. For example, places in Africa because the equipment required to read the chip is likely to be simpler.

Multiplex assay as employed herein refers to a type of assay that simultaneously measures several analytes (often dozens or more) in a single run/cycle of the assay. It is distinguished from procedures that measure one analyte at a time.

In one embodiment there is provided a bespoke gene chip for use in the method, in particular as described herein.

In one embodiment there is provided use of a known gene chip for use in the method described herein in particular to identify one or more gene signatures described herein.

In one embodiment there is provided a method of treating latent TB after diagnosis employing the method disclosed herein.

In one embodiment there is provided a method of treating active TB after diagnosis employing the method disclosed herein.

Examples of suitable agents for treating TB include but are not limited to isoniazid, rifampin, ethambutol, pyrazinamide, streptomycin, kanamycin, amikacin, capreomycin, levofloxacin, moxifloxacin, ofloxacin, para-aminosalicylic acid, cycloserine, terizidone, thionamide, protionamide, clofaximine, linezolid, amoxicillin/clavulanate, thioacetazone, imipenem/cilastatin, high dose isoniazid, clarithromycin.

In one embodiment the treatment comprises a combination of two or more of the above agents.

Gene signature, gene set, disease signature, diagnostic signature and gene profile are used interchangeably throughout and should be interpreted to mean gene signature.

In the context of this specification "comprising" is to be interpreted as "including".

Aspects of the invention comprising certain elements are also intended to extend to alternative embodiments "consisting" or "consisting essentially" of the relevant elements.

Where technically appropriate, embodiments of the invention may be combined.

Embodiments are described herein as comprising certain features/elements. The disclosure also extends to separate embodiments consisting or consisting essentially of said features/elements.

Technical references such as patents and applications are incorporated herein by reference.

Any embodiments specifically and explicitly recited herein may form the basis of a disclaimer either alone or in combination with one or more further embodiments.

## EXAMPLES

### Example 1 - Development of Forward Selection - Partial Least Squares (FS-PLS) method

#### Overview of biomarker selection methods in 'omics datasets

Conventional methods for variable selection and model building, as applied to omics data, fall broadly into three categories. A comprehensive review on the methodological challenges behind omics-based biomarker selection is given by Hyam and colleagues (2) but for the scope of this paper, we provide a brief description of methodologies with their relative strengths and limitations.

(A) **Univariate variable selection *followed* by model fitting.** These methods first rank the variables by applying a univariate test statistic. (ie t-test, Cochran-Armitage test) The top ranked variables are then selected based on a threshold and model fitting is achieved using a machine learning classification method (ie. support vector machines (3), decision trees (4) and Maximum Likelihood Discriminant analysis such as Linear Discriminant Analysis and Diagonal Linear Discriminant Analysis (5).These methods benefit by the prediction power of the classification algorithm but depend highly on the original pre-filtering, which requires a threshold that most of the times is arbitrary and if it is too stringent it might miss important variables or if it is too loose it might include redundant variables.

(B) **Multivariate model fitting with *embedded* variable selection.** These methods perform variable selection and model fitting simultaneously. Most regression-based techniques, such as Forward Selection (6, 7)) consider all variables simultaneously and allow each variable to enter/exit the model by penalizing its inclusion/exclusion based on an optimization criterion (6). There are several optimization criteria and among all candidate variables, the next best variable to enter the model is the one which if entered will result in the largest change in the estimated criterion. Regularization-based methods, such the lasso (8) and the elastic net (9) have been extensively applied on 'omics data for feature selection and classification (9, 10). These methods, also referred to as shrinkage methods, select the next best variable to enter the model is the one that would have the most significant coefficient if entered, given all the previous variables selected. The regression coefficients are estimated by penalizing inclusion. Aforementioned methods don't necessarily remove redundant correlation structure between the variables and it has been long argued that they are prone to over-fitting.

(C) **Projection-based methods.** These techniques are especially suited to deal with a much larger number of correlated variables than samples. They reduce the original number of variables by converting them into new latent variables, which are the non-correlated linear transformations of the original (ie. Principal Component Analysis (PCA) (11) and Partial Least Squares( PLS) regression transforms the data into orthogonal, non-correlated latent components and then uses these components in place of the original variables into a logistic regression model to predict the outcome of interest (12). Nevertheless, these techniques do not perform directly variable selection and in order to do so, further steps needs to be taken , as suggested in the penalized regression PLS (13) and the lasso penalized regression PLS (14). PLS-model based methods applied on gene expression and metabolomics data (i.e. PLS-Discriminant Analysis (PLS-DA) (12) or OPLS-DA (15) an extension of PLS-DA featuring an integrated orthogonal signal correction filter to remove variability not relevant to class separation),eagerly over-fit the data and rigorous validation is necessary to ensure generalization ability (16).

To overcome these challenges, we developed a methodology that combines the statistical efficiency of Partial Least Squares (PLS) in reducing the dimensions of highly correlated datasets with the effectiveness of maximum likelihood estimation in Forward Selection in fitting small models. Our proposed methodology, Forward Selection - Partial Least Squares (FS-PLS) combines the dimensionality reduction of projection-based methods with the model simplicity and clinical interpretability of forward selection stepwise regression. It therefore derives small predictive signatures of the disease or the clinical outcome in question.

#### Description of FS-PLS

FS-PLS performs variable selection and model fitting on genome-wide profiles of binary (e.g. 1 if diseased, 0 if healthy control) or linear clinical outcomes (insulin levels). The variables in the model are the measures molecules in an omics dataset, such as the transcript levels in a microarray gene expression experiment, the protein or metabolite intensity peaks in a proteomics or metabolomics study respectively. The algorithm receives as input all the variables included in the study and the goal is to select the minimum set of variables that best classify the clinical outcome of interest.

Given an original set of N molecular variables $\chi_1 .....\ \chi_N$ and a clinical outcome *y* the algorithm **FS-PLS** initially fits N univariate regression models, y= $\beta_i x_i$, for i in [1,N]. As in classical regression, the regression coefficient for each model $\beta_1$, $\beta_2$.. $\beta_N$ is estimated using the Maximum Likelihood Estimation (MLE) function, the goodness of fit is assessed by means of a *t*-test and statistical significance is assessed by comparing the P-value of the *t*-test statistic with a predefined threshold $p_{thres}$ (default $p_{thres}$ =0.05). The first variable to get selected, for example $\chi_8$(*N*>8), is the one with the highest MLE and smallest P-value. We will call this variable $SV_1$. Now *N-1* variables are left for consideration to enter the final model, which for now contains only $SV_1$. In order to select the next variable, the algorithm projects out the variation explained by $SV_1$ using Singular Value Decomposition. The projected variation explained by $SV_1$, is subtracted from all remaining variables and the algorithm fits *N-1* models on the residual variation of each remaining variable. The second variable is selected using the same criteria as for the first one. The algorithm uses this iterative process and at each step the aim is to project out all variations corresponding to the already selected variables and to select a new variable by fitting models on the residual variation. This procedure terminates only when there is no new variable to enter the model with MLE P-value< $p_{thres}$. The final model contains all selected variables selected with the regression coefficients as calculated per individual model. No re-fitting of the coefficients is taking place.

There is the option to exclude all variables with variance less than a predefined threshold, in the default setting being var_thresh=0.01.

## Datasets used in the study

### *Transcriptomics*

**Leukemia.** The Golub et *al.* gene expression study collected bone marrow and peripheral blood samples from leukemia patients and the clinical outcome was either Acute Myeloid Leukemia (AML) or Acute Lymphoblastic Leukemia (ALL). The dataset, as described in the original paper (17), has served as a benchmark dataset in several published molecular classification methodologies. The dataset consists of a training set (N=38: 27 ALL 11 AML all bone marrow samples) and an independent test set (N=34: 24 bone marrow and 10 peripheral blood samples) and 7,125 gene expression transcripts were available butafter pre-processing of the data, as described in Dudoit et *al.* (5), 3,571 transcripts remained as potential biomarkers for disease classification.

**Breast Cancer.** FDA approved. The original study is described in The Parker et *al.* [Parker et al. 2009. J. Clinical Oncology] Breast Cancer "intrinsic" subtyping gene expression study employs the PAM50, a prediction model based on the expression of the 50 classifier genes, to classify subjects into breast cancer intrinsic subtypes. Major intrinsic breast cancer subtypes include Basal-like, Luminal A, Luminal B and HER2-enriched and Normal-like, with each subtype having specific clinical features. The signature of the PAM50 was obtained from an expanded "intrinsic" gene set found in previous microarray studies. The genes were selected so that they have the highest amount of variation between intrinsic subtypes and the least within each subgroup [Peru et al. 2000, Nature]. The employed algorithm, consisted of centroids constructed based on Prediction Analysis of Microarray (PAM) and hence the signature PAM50. The original study is described in Parker et al. 2009. J. Clinical Oncology. In the current study, we used the training set consisting of 225 breast cancers (67 Basal, 77 Luminal A, 34 Luminal B, 35 HER2+ and 12 Normal-like) and the independent test dataset including breast invasive carcinoma expression data (n=547) from The Cancer Genome Atlas TCGA: http://cancergenome.nih.gov/

### *Proteomics*

**Prostate cancer.** The Petricoin et *al* (23) prostate cancer screening trial study from the National Cancer Institute in Maryland aimed to evaluate proteomics as a diagnostics technology to discriminate malignant prostate from benign in men with either normal or elevated PSA levels in the blood. Currently, the amount of Prostate Specific Antigen (PSA) in the blood is followed by a biopsy if recommended, is the common test for prostate cancer detection. Normal PSA levels (serum PSA level <4ng/mL) suggest healthy prostate while elevated levels (serum PSA level >=4ng/mL) indicate increased likelihood of cancer but do not distinguish between malignant and benign unless a biopsy confirms it. The proteomics dataset consists of 322 samples, 191 of which with PSA >=4ng/Ml and confirmed biopsy of malignant prostate, 71 samples with PSA >=4ng/Ml and confirmed biopsy of benign prostate and 64 samples with PSA< 1ng/Ml and healthy prostate. For all samples, SELDI-TOF serum profiling technology generated 15,551 protein peaks.

### *Metabolomics*

**Blood pressure, macro- and micro-nutrients metabolomics.** The INTERnational study of MAcro-nutrients, micro-nutrients and blood Pressure (INTERMAP) was a multi-center cross-sectional epidemiologic investigation that was designed to help clarify unanswered questions regarding the role of dietary factors in the development of unfavorable blood pressure (BP) levels in adults.(24, 25) The study included 4,680 participants aged 40 to 59 years from China, Japan, United Kingdom, and United States of America. The data analysed in this paper was collected during two standardized 48-hour dietary recalls (including dietary supplement use), two standardized 7-day histories of alcohol intake, and two timed 24-hour urine samples corresponding to each of the two dietary recalls. Data were discretized into 7,100 spectral bins of equal width (0.001 ppm). For the purpose of the present study, we selected the subset of 1,299 participants of non-Hispanic White ethnicity from eight centers in the United Kingdom and United States of America who were not undertaking treatment for hypertension.

**Tuberculosis diagnostic transcriptomics study and RT-PCR validation.** The case control Tuberculosis in HIV-infected and -uninfected adults from sub-Saharan Africa transcriptomics study aimed at identifying a host whole blood RNA signature to be used to diagnose active tuberculosis (TB) in high HIV/TB prevalence settings from latent TB infection (LTBI) (26). The signature presented in the paper - comprising of 27 transcripts - was derived from microarray expression data acquired from patients recruited in Cape Town and Malawi. The cohort in the original paper as well as this study was split into a training set (N=285), which was used for discovery using elastic net, and a test set (N=76) which was used for validation along with a previously published microarray dataset (N=51) (27). In order to confirm the FS-PLS microarray results across platforms we performed quantitative real-time PCR (qPCR) analysis (even if microarray and qPCR results sometime disagree [(28)].). Measurements for the transcripts of the FS-PLS signature and housekeeping genes (GAPDH and 18S) were acquired using Fluid Dynamic Arrays for the samples of the training and the test set. 272 samples out of the initial 285 of the training microarray set and 74 out of 76 of the test set passed quality control.

## Results

We applied FS-PLS to six published 'omics datasets including two microarray gene-expression transcriptomics, two mass spectrometry proteomics and two Nuclear magnetic resonance spectroscopy (NMR) metabolomics. We also applied FS-PLS on our Tuberculosis transcriptomics diagnostic study and we validated the results, using an independent published cohort and replicated our findings using alternative diagnostic techniques, namely RT-PCR performed extensive comparison the originally employed methods, which include a centroid classifier, the lasso (8), the elastic net (9). We chose to compare our method with methodologies that perform both variable selection and model fitting, as FS-PLS falls within this category. We assume that predictive power is also a function of the data quality and that predictive performance is similar across methods when applied on the same dataset, as demonstrated before (13, 19-21)

### Leukemia

In the original study, the authors used 50 gene transcripts in a self-organizing map (SOM) classifier, which misclassified 2 out of the 38 samples in the training set and 5 out of the 34 samples in the independent test set. However, FS-PLS selected 3 transcripts and achieved perfect discrimination between the two subtypes of leukemia in the leave-one out cross validation of the training set. FSPLS misclassified two samples in the independent test set, which were also misclassified in the original study and it turned out later that those samples were assigned in the wrong class (29). Unsupervised clustering also demonstrates that the 3 genes selected by FSPLS are powerful to naturally group the samples into their real classes. When comparing our results with other published results on the same dataset, Tibshirani and colleagues reported perfect classification between ALL and AML data in the test set by selecting 45 features (30). We also provide the performance metrics of five methods applied to this dataset, which achieve between zero and 4 misclassified samples in the test set. Notably, all methods select more predictors than there samples in the test set and although a rigorous cross-validation procedure was applied, we still argue that selecting more predictors than there are samples to classify is a sign of over-fitting.

### Breast Cancer

In the published PAM50 model, classification was based on the nearest centroid approach. Gene expression data including all intrinsic subtypes were trained over a supervised algorithm to construct centroids for each subtype. These centroids were then used for subtype prediction of the test samples. The distance of the gene expression profile (based on the expression of 50 classifier genes) of each test sample was measured against each subtype centroid. Samples were assigned to the respective intrinsic subtype based on the nearest centroid [Parker et al. 2009. J. Clinical Oncology].

FS-PLS selected a subset of six genes (*p-value* < 0.001) which performed as well as PAM50 gene signature in classifying breast cancer into 5 known intrinsic subtypes. We employed two datasets: 1) the training set which was used to extract the original PAM50 gene signature as obtained from the Gene Expression Omnibus (GEO: GSE10886), and 2) the gene expression profile of the 547 breast invasive carcinoma as downloaded from The Cancer Genome Atlas (http://cancergenome.nih.gov/). We extracted the extended intrinsic gene set from the training data to which FS-PLS was applied.

**Proteomics.**

In the original study, the authors used 7 protein peaks to classify the test set, however they did not report the classification algorithm. We therefore compare our method only against the results reported, as well as elastic net. The authors used 56 samples for training and 226 samples for testing; the training set consists of 25 normal prostate and 31 biopsy-proven cancer samples, however the test set consists of 38 biopsy-proven samples and 228 normal or benign prostate samples. The classifier was trained in the two extreme classes and aim at distinguishing the intermediate class, which is benign prostate. Within the benign prostate class, there were 75 samples with PSA<4, which is almost normal, 16 with PSA>10 which would otherwise indicate increased likelihood of malignant prostate and 137 samples in the so called indeterminate class of PSA<4 and 10. FS-PLS selected 5 protein peaks to classify the data. We followed the same design as in the original study).

**Metabolomics**

In the original study, linear models were estimated for each spectral bin (as dependent variable) separately, once without adjustment and once adjusting for 11 covariates including study center, gender, and age. A spectral bin was declared to be significantly associated with systolic BP if: (1) the associated p-value was below the Bonferroni-corrected significance threshold controlling the family-wise error rate at the 1% level; (2) the same held true for the two adjacent spectral bins; (3) directions of associations were concordant across the three spectral bins; (4) the previous conditions were satisfied for both visits. In the original paper, the univariate MWAS analysis for systolic BP identified 67 significantly associated spectral bins for unadjusted analyses, and six (three overlapping between the two visits) for adjusted analyses. Analysis of the same dataset using FS-PLS identified a total of 17 significantly associated spectral bins for unadjusted analyses over the two visits (with no overlap, 7 in the first visit and 10 for the second visit), of which five were already identified using the univariate approach described before. As no other multivariate method was applied in the original study, we also performed metabolite selection using the lasso and the elastic net penalized regression to directly compare FSPLS against other powerful similar methods. The three methods, after a split of the data in 80%-20% with a 10-fold Cross-Validation on the training set, achieved almost the same mean-squared error in their predictions, however FSPLS selected only 7 variables compared to 28 and 35 for the lasso and the elastic net respectively.

Elastic net and the lasso tend to allow correlated variables to enter the model even if correlation is not increasing predictive performance, which always results in larger models. FS-PLS through the powerful step of projecting out the explained variation of any new variable, allows correlated variables to enter the model only if they explain additional information in the outcome. We therefore tuned the regularization parameter of elastic net and lasso in such a way as to restrict the maximum number of selected variables to be as many as those selected by FSPLS (i.e. 7 for the first visit and 10 for the second visit dataset). Even in that case, elastic net and lasso selected correlated variables with a slight loss in predictive performance when compared to FSPLS.

We finally adjusted all previous analysis by accounting for eleven covariates, among which the gender, age, smoking, BMI, physical activity and alcohol consumption of the study participants. FSPLS selected 7 variables in both visit datasets, four of which being BMI, gender, age and alcohol in both measurements and in the same order of significance. Of note is the fact that FSPLS selected the metabolite with molecular weight 3.3545 kDa in both datasets. This metabolite was not chosen in the original unadjusted analysis, however the great consistency of FSPLS covariate and spectral bin selection between the two visit datasets serves as a proof of the method's ability to select robust biomarkers.

**Validation and replication transcriptomics study using both microarray gene-expression and RT-PCR**

In the original study (26), elastic net after 10 fold cross validation for tuning its parameters selected 27 transcripts for the comparison of TB vs LTBI, while FS-PLS selected 3. While the number of transcripts selected is reduced 9 times, the difference between the two classifiers AUC is 1% for the test set and 0.3% for the training set. The transcripts that were selected from elastic net and FS-PLS for the classification of TB vs LTBI in adults were taken forward for RT-PCR validation. Out of the 27 transcripts that elastic net selected, 25 were used for the analysis (one failed quality control and one was represented twice in the signature). The three transcripts that FS-PLS selected passed quality control. The raw CT (cycle threshold) value for every patient and every transcript was acquired and normalized against the mean of the two housekeeping genes (18S and GAPDH) OR quantile normalized to account for biases. The samples for which RT-PCR was run, were divided into training and test set according to the microarray grouping. After using the 25 transcript elastic net model and the 3 transcript FS-PLS model for classification of the RT-PCR data we observed that in general the performance was lower compared to the microarrays. The two methods performed almost identical in terms of classificatory power in both the training and the test set. See Table 1.

**Simulations**

Empirical studies on simulated data sets were performed to illustrate the effectiveness of the FS-PLS. The forward selection algorithm (FS), lasso and elastic net were also applied to these data sets as comparisons to FS-PLS. For lasso and elastic net, we used the implementation cv.glmnet in R package glmnet with default parameters.

The root mean square error (RMSE) was employed as an evaluation of the predictions for the data sets with continuous outputs, while the area under ROC curve (AUC) for the data sets with 2-class discrete outputs and the accuracy (ACC) for the data sets with 3- and 5-class discrete outputs. The statistical results for the testing data set show that FS-PLS provided consistently better performance compared to the other three methods. For the few exceptions, FS-PLS still gave competitive results. It is noted that FS-PLS reported dominant performances when the total number of variables or classes is large.

We also studied the number of variables selected by these methods for the final models (regression or classification). FS-PLS selected much less variables for all data sets. Lasso selected about ten times the number of variables as FS-PLS did, and that was even more for elastic net. The differences between FS and FS-PLS were trivial when the total number of variables or classes for the data sets is small, but showed a significant increase then. Interestingly, the number of variables selected by FSPLS remained almost unchanged across data sets TB vs. OD, TB vs. LTBI and INTERMAP, even though the total number of variables had increased from 379 to 7100.

**Discussion**

We have developed a novel method for biomarker discovery, FS-PLS, which derives small predictive signatures of disease and clinical outcomes. We have demonstrated the flexibility and applicability of the method using six publically available 'omics datasets, including transcriptomics, proteomics and metabolomics. We showed that FSPLS in all datasets selects a small number of biomarkers with high predictive performance, when directly compared to the original published biomarker selection methodologies. We finally showcased the reproducibility of the biomarkers selected by FSPLS using a Tuberculosis transcriptomics study generated from our lab, whose gene-expression data have already been published (31) and further validated the findings using RT-PCR on the same patients.

On the transcriptomics study of breast cancer, the gene-set obtained by FS-PLS achieved >90% of sensitivity and specificity in terms of classifying the subjects into their respective groups. Molecular biomarkers obtained from gene-expression profiles play an important role in diagnosis and prognosis of cancer patients. However, clinical validation of these signatures has been slow. Shorter signatures and assays with simplified workflow are required for fast and efficient validation of these biomarkers where they can be easily used in clinical practice [Nielsen et al. 2014. BMC Cancer]. PAM50 model is often used for "intrinsic" subtyping of breast cancer. It measures the expression level of 50 classifier genes from breast cancer samples and has been shown to have good prognostic power in both un-treated and tamoxifen treated patients. This model is also used to determine the risk of relapse for each patient [Parker et al. 2009. J. Clinical Oncology]. However, in this study, we only compare molecular subtyping property of PAM50 with FS-PLS generated signature. In the PAM50 signature, there are 10 genes specific to each intrinsic subtype [Parker et al. 2009. J. Clinical Oncology]. With only six genes, FS-PLS performed as well as the PAM50 gene signature. Although these two signatures have been derived from the same source, they have only two genes in common (FOXC1 and ERBB2).

Proteomic profiling on serum or urine samples for biomarker discovery is now coming of age (37). Studies have yielded optimistic results on Alzheimer's disease(38), HIV(39), cancer(40), pancreatitis(41) and Kawasaki disease(42). Applying bioinformatics to proteomics (43) is just emerging. In a recent paper, Zhai *et al used* support vector machines on SELDI proteomics to derive a 5-protein signature that could discriminate among the different stages of esophageal carcinogenesis.(44) They report 97% specificity with 87% sensitivity. As discussed in a recent review (37), although several biomarkers have been suggested by proteomics studies, few have been actually been validated on a separate cohort or have been discovered in a study that used proper controls. Another major shortcoming has been the lack of appropriate statistical methods for biomarker definition. We expect proteomics technology coupled with biomarker analysis techniques to be in the centre of novel diagnostics. Molecular biomarkers can potentially be used to for diagnosis, disease monitoring or to guide therapy selection.(40) We anticipate such methodologies to be used either as a diagnostic or a molecular decision support tool in distinguishing -at the protein level- diseases, whose accurate diagnosis cannot be achieved using only their clinical features. For example proteomics has successfully yielded results in Kawasaki disease (42), whose etiology is unknown and pathophysiology poorly understood. Kentsis et *al* showed that by using proteomic profiling they identified 190 potential KD biomarkers almost uniquely present in patients with KD and absent in patients of other clinically-mimicking conditions.

In the metabolomics dataset, strong correlations were observed among significantly associated bins, as exemplified in Figure 1. This is due to the complex correlation structure that is commonly found in metabolomics data, and consists of three intertwined levels: (1) a *local* component, reflecting correlations between adjacent spectral bins; (2) a *non-local* component, reflecting the fact that the same metabolite will usually give rise to multiple (correlated) peaks; (3) a *biological* component, reflecting the fact that biological processes are usually driven by sets of molecules. Only the latter correlation structure is of interest for biomarker discovery, but it is frequently hidden behind the first two structures. In particular, it is often necessary to apply some unsupervised clustering techniques to identify groups of spectral bins, which can then be characterized chemically. From this point of view, FS-PLS constitutes an important step forward: since the signals it identifies are uncorrelated by construction, they almost certainly originate from different metabolites. These could in turn be easily identified using established techniques in chemometrics such as STOCSY. (36)

We have not applied FS-PLS on genomic as and epigenetics datasets. We expect that these studies will require special adjustment or the evolution of our method to cope with the even higher dimensionality and the low predictive performance. Further work is needed to extend our method for accommodate multinomial response.

FS-PLS has several advantages over various similar methodologies including the fact that (1) it is computationally very fast and applicable to large-scale 'omics data as opposed to traditional FS (2) it is flexible and not platform sensitive, therefore can be readily applied to any 'omics dataset (3) it facilitates clinical interpretation as the outcome is a regression model with weights, as opposed to PLS models that are difficult to understand. It also outperforms similar methods of the PLS family as it (1) directly selects markers rather than the latent components and (2) does not require a further search step within the component loadings (14). FS-PLS therefore achieves interpretability and high predictive power. The small number of predictors also ensures cost-effectiveness in follow-up studies. An important advantage of FS-PLS over all dimensionality reduction methods is the ability to adjust for known confounders, such as age, sex, ethnicity and others. (1) We select un-correlated biomarkers, as it is clearly demonstrated by the correlation plots. (3)

We anticipate our method to find wide application in studies where identifying the minimum set of biomarkers with the highest predictive potential is key for success and cost-effectiveness, such as the field of novel molecular diagnostics. Translating large transcriptomics signatures into clinical diagnostics tools for disease is a complex and expensive process. However there are methods that allow multi-transcript signature measurements (32-35). For all these methods, a reduced number of transcripts would translate into reduced cost and complexity. Molecular classification of diseases using gene-expression profiling has been ongoing for more than a decade with many signatures achieving FDA approval or being transformed into public health diagnostic tools.

**Example 2 - Applying FS-PLS method to original 44 and 27 gene signatures for detecting active TB Test subjects and Validation datasets**

The samples and validation datasets used in this Example are the same as those described in Kaforou et al (26) and in the present inventors' previously filed application WO2014/019977.

**Minimal gene signatures**

In order to further reduce the number of genes in the original 27 and 44 gene signatures, Forward Selection - Partial Least Squares (FS-PLS) as described in Example 1 was applied to previously obtained gene expression data from Kaforou et al.

The first iteration of the FS-PLS algorithm considers the expression levels of all transcripts (N) and initially fits N univariate regression models. The regression coefficient for each model is estimated using the Maximum Likelihood Estimation (MLE) function, and the goodness of fit is assessed by means of a t-test. The variable with the highest MLE and smallest p-value is selected first (SV1). Before selecting which of the N-1 remaining variables to use next, the algorithm projects the variation explained by SV1 using Singular Value Decomposition. The algorithm iteratively fits up to N-1 models, at each step projecting the variation corresponding to the already selected variables, and selecting new variables based on the residual variation. This process terminates when the MLE p-value exceeds a pre-defined threshold. The final model includes regression coefficients for all selected variables. See also Figure 4.

Using FS-PLS, a new minimal 3 gene signature was identified for discriminating between TB and Latent TB, whilst a new minimal 6 gene signature was identified for discriminating between TB and other diseases (see Tables 3 and 4).

**Performance of 3 gene and 6 gene signatures**

To evaluate the performance of the new minimal 3 and 6 gene signatures, the disease risk score was calculated. The score is based on subtracting the summed intensities of the down-regulated transcripts from the summed intensities of the up-regulated transcripts. The risk score was calculated on normalised intensities. The disease risk score for individual i is:

$$Disease\ Risk\ Score^i = \sum_{k=0}^{n} expr.value_k^i - \sum_{l=0}^{m} expr.value_l^i \qquad (1)$$

where: n the number of upregulated number of probes in the signature in disease of interest compared to comparator group(s).
m the number of downregulated number of probes in the signature in disease of interest compared to comparator group(s).

The threshold for the classification was calculated as the weighted average of risk score within each class, with weights given as inverse of the standard deviation of the score within each class (1/sd1 and 1/sd2 respectively). The threshold for the classification between group *u* and *v* is shown below:

$$threshold(u,v) = \frac{\frac{\mu_u}{\sigma_u} + \frac{\mu_v}{\sigma_v}}{\frac{1}{\sigma_u} + \frac{1}{\sigma_v}} \qquad (2)$$

where: $\mu$ = average of the disease risk score in the group.
$\sigma$ = standard deviation of the disease risk score in the group.

To calculate the indeterminate zone, we calculated the lower and upper threshold which were calculated as the weighted average with weights given by w/sd1, (1-w)/sd2 respectively for variable 0.5 < w <= 1. When w = 0.5 its equivalent formula to main threshold. ROCs were generated using pROCs. Alternatively:
To calculate the indeterminate zone, we calculated the lower and upper threshold which were calculated as the weighted average with weights given by $\frac{w}{\sigma_u}, \frac{2-w}{\sigma_v}$ respectively:

$$weighted_threshold(u,v) = \frac{w * \frac{\mu_u}{\sigma_u} + (2-w) * \frac{\mu_v}{\sigma_v}}{\frac{w}{\sigma_u} + \frac{2-w}{\sigma_v}}, \qquad 0 \leq w \leq 2 \qquad (3)$$

When w=1 the formula is equivalent to the main threshold formula.

To evaluate the performance of the DRS as a classifier we used different measures (AUC, sensitivity, specificity, PPV, NPV, and likelihood ratios).

The calculation of the confidence intervals for the area under a receiver operating characteristic curve (AUC), the sensitivity and the specificity was based on a non-parametric stratified bootstrap resampling (each replicate contained the same number of cases and controls as the original sample) (Robin et al 2011), with 2000 bootstraps, as recommended by Carpenter et al. (2000).

In order to compare directly the differences of the performance of our signatures to the signatures presented in the Berry et *al* (2010), we calculated the differences of the means of the measures of classification (namely the AUC, the sensitivity and the specificity) on our test set along with their 95% confidence intervals, using the following mathematical formulas:

$$(a,b) = \hat{\pi}_1 - \hat{\pi}_2 \pm z_{\alpha/2} \cdot s(D) \qquad s(D) = \sqrt{\frac{\hat{\pi}_1(1-\hat{\pi}_1)}{n_1} + \frac{\hat{\pi}_2(1-\hat{\pi}_2)}{n_2}}$$

## Results

The results of the performance analyses are shown in Tables 4 to 7. As can be seen from Table 4, the 3 gene signature has a very similar and very high AUC for the training, test and Berry et al validation datasets.

Table 5 shows the results based on RT-PCR validation and likewise indicates that the performance of the 3 gene signature is on par with the performance of the 27 gene signature. Table 6 shows the performance of the individual transcripts of the 6 gene signature. Note that the AUC is very high, which suggests a high discriminatory power for discriminating between active TB and other diseases.

Table 7 shows the results of a comparison of classificatory power for discriminating active TB from other diseases between the original 44 gene signature and the new minimal 6 gene signature. Again the AUC values are very similar, suggesting that the 6 gene signature has nearly identical discriminatory power as the 44 gene signature.

The results of this Example thus provide a strong indication that the FS-PLS method can effectively identify smaller gene signatures than the previously employed Elastic Net method. This example also demonstrates that testing as few as 3 genes is sufficient to discriminate active TB from latent TB and that as testing as few as 6 genes is sufficient to discriminate active TB from other diseases, even in the presence of a complicating factor such as HIV infection.

## Tables

**Table 1:** Validation and replication study of FS-PLS.

| FS-PLS was applied to the Tuberculosis gene-expression study and was compared to the original method used, which was Elastic Net. Both Elastic Net (27 gene signature) and FS-PLS (3 gene signature) were also applied to data derived from the same individuals using RT-PCR. FS-PLS achieved similar predictive performance while selecting 9 times less predictors across all comparisons at the replication platform. | | | | | | |
|---|---|---|---|---|---|---|
| **Microarrays** | | | | | | |
| | **Training set** | | **Test set** | | **Berry *et al* validation set** | |
| | **Elastic Net** | **FS-PLS** | **Elastic Net** | **FS-PLS** | **Elastic Net** | **FS-PLS** |
| **AUC** | 0.964 | 0.943 | 0.979 | 0.960 | - | 0.974 |
| **CI** | 0.9456-0.9828 | 0.9188-0.9675 | 0.954-1 | 0.9150-1 | - | 0.9285-1 |
| **RT-PCR** | | | | | | |
| | **Training set** | | **Test set** | | **Combined** | |
| | **Elastic Net** | **FS-PLS** | **Elastic Net** | **FS-PLS** | **Elastic Net** | **FS-PLS** |
| **AUC** | 0.8553 | 0.852 | 0.9671 | 0.9649 | 0.8657 | 0.8615 |
| **CI** | 0.8103-0.8975 | 0.8065-0.8944 | 0.9276-0.992 | 0.924-0.9934 | 0.8227-0.9038 | 0.8227-0.8997 |

**Table 2:** 27 gene signature and new minimal 3 gene signature

| Array ID | Gene Name | Probe ID | Direction of regulation* |
|---|---|---|---|
| 70730 | GAS6 | ILMN_1779558 | Up |
| 130181 | ANKRD22 | ILMN_1799848 | Up |
| 360132 | LHFPL2 | ILMN_1747744 | Up |
| 520086 | FCGR1A# | ILMN_2176063 | Up |
| 1300139 | GNG7 | ILMN_1728107 | Down |
| 1340241 | C5 | ILMN_1746819 | Up |
| 1440341 | C1QC | ILMN_1785902 | Up |
| 1510026 | FLVCR2 | ILMN_2204876 | Up |
| 1780440 | CD79A | ILMN_1659227 | Down |
| 2630195 | VAMP5 | ILMN_1809467 | Up |
| 2650605 | C4ORF18 | ILMN_1672124 | Up |
| 2710709 | FCGR1B | ILMN_2261600 | Up |
| 2810373 | FAM20A | ILMN_1812091 | Up |
| 2970397 | ZNF296# | ILMN_1693242 | Down |
| 3520601 | MPO | ILMN_1705183 | Up |
| 3780047 | GBP6 | ILMN_1756953 | Up |
| 3890400 | CXCR5 | ILMN_2337928 | Down |
| 4280632 | GAS6 | ILMN_1784749 | Up |
| 5570039 | LOC728744 | ILMN_1654389 | Up |
| 5570398 | FCGR1C | ILMN_3247506 | Up |
| 5890470 | CCR6 | ILMN_1690907 | Down |
| 5910019 | C1QB# | ILMN_1796409 | Up |
| 5910632 | SMARCD3 | ILMN_2309180 | Up |
| 6060468 | S100A8 | ILMN_1729801 | Up |
| 6450594 | CD79B | ILMN_1710017 | Down |
| 6560156 | DUSP3 | ILMN_1797522 | Up |
| 6620209 | FCGR1B | ILMN_2391051 | Up |

* in TB patients in relation to patients with latent TB infection.

# Genes in the 3 gene signature

**Table 3:** 44 gene signature and new minimal 6 gene signature

| Array ID | Gene Name | Probe ID | Direction of regulation* |
|---|---|---|---|
| 130086 | CYB561 | ILMN_1771179 | Up |
| 150224 | LOC196752 | ILMN_1803743 | Up |
| 270039 | HM13 | ILMN_1766269 | Up |
| 360132 | LHFPL2 | ILMN_1747744 | Up |
| 380541 | PPPDE2 | ILMN_1737580 | Up |
| 450132 | RBM12B | ILMN_1805778 | Up |
| 450379 | PRDM1[#] | ILMN_2294784 | Up |
| 540041 | CASC1 | ILMN_1708983 | Up |
| 840446 | CYB561 | ILMN_2378376 | Up |
| 1030433 | CALML4 | ILMN_1815707 | Up |
| 1050360 | HLA-DPB1 | ILMN_1749070 | Up |
| 1070477 | ALDH1A1 | ILMN_2096372 | Up |
| 1110592 | EBF1 | ILMN_1778681 | Down |
| 1170332 | AAK1 | ILMN_1688755 | Up |
| 1580437 | PGA5 | ILMN_1717572 | Down |
| 1690184 | RNF19A | ILMN_1812327 | Up |
| 2000682 | HS.131087 | ILMN_1916292 | Down |
| 2030309 | SERPING1 | ILMN_1670305 | Up |
| 2260349 | MIR1974 | ILMN_3308961 | Up |
| 2340241 | IMPA2 | ILMN_2094061 | Down |
| 2350114 | GJA9 | ILMN_1710161 | Up |
| 2850315 | ORM1 | ILMN_1696584 | Down |
| 3120475 | MAP7 | ILMN_2216815 | Down |
| 3130600 | BTN3A1 | ILMN_1802708 | Up |
| 3310504 | PDK4 | ILMN_1684982 | Down |
| 3360553 | RP5-1022P6.2 | ILMN_1701111 | Down |
| 3780047 | GBP6[#] | ILMN_1756953 | Up |
| 3840053 | UGP2 | ILMN_1671969 | Up |

| | | | |
|---|---|---|---|
| 4070524 | CERKL | ILMN_1801091 | Up |
| 4290619 | CREB5# | ILMN_1728677 | Up |
| 4560047 | CD74 | ILMN_1761464 | Up |
| 4570164 | LOC389386 | ILMN_3215715 | Up |
| 4640768 | VPREB3# | ILMN_1700147 | Down |
| 4670458 | SEPT4 | ILMN_1776157 | Up |
| 5260161 | HS.162734 | ILMN_1893697 | Down |
| 5270753 | ARG1# | ILMN_1812281 | Down |
| 5290100 | MAK | ILMN_1803984 | Down |
| 5820491 | MAP7 | ILMN_1712719 | Down |
| 6380681 | C19ORF12 | ILMN_1664920 | Up |
| 6510754 | ALDH1A1 | ILMN_1709348 | Up |
| 6560156 | DUSP3 | ILMN_1797522 | Up |
| 6760056 | LOC100133800 | ILMN_3287952 | Up |
| 6760471 | TMCC1# | ILMN_1677963 | Down |
| 7210110 | HM13 | ILMN_2236655 | Up |

* in TB patients in relation to patients with other diseases.

# Genes in 6 gene signature

**Table 4:** Comparison of classificatory power for discriminating TB vs LTBI (latent TB) for 27 gene signature vs 3 gene signature

| | Training set | | Test set | | Berry et al validation | |
|---|---|---|---|---|---|---|
| | Elastic Net (27) | FS-PLS (3) | Elastic Net (27) | FS-PLS (3) | Elastic Net (27) | FS-PLS (3) |
| **Area Under the Curve** | 0.96 | 0.94 | 0.98 | 0.97 | 0.99 | 0.98 |
| **95% CI** | 0.93-0.97 | 0.91-0.96 | 0.95-1 | 0.94-1 | 0.95-1 | 0.95-1 |

**Table 5:** Performance of 3 gene signature vs 27 gene signature based on RT-PCR validation

| | RT-PCR | |
|---|---|---|
| | Training and Test Sets | |
| | Elastic Net | FS-PLS |
| **Area Under the Curve** | 0.87 | 0.86 |
| **95% Confidence Interval** | 0.82-0.90 | 0.82-0.90 |

**Table 6:** Performance of 6 gene signature

| Transcripts | Area under the curve in the test set |
|---|---|
| ID1 | 0.85 |
| ID2 | 0.88 |
| ID3 | 0.89 |
| ID4 | 0.92 |
| ID5 | 0.92 |
| ID6 | **0.92** |

**Table 7:** Comparison of classificatory power for discriminating active TB vs OD (other disease) for 44 gene signature vs 6 gene signature

| | Training set | | Test set | |
|---|---|---|---|---|
| | Elastic Net (44) | FS-PLS (6) | Elastic Net (44) | FS-PLS (6) |
| **Area Under the Curve** | 0.97 | 0.92 | 0.94 | 0.92 |

**References**

WHO report 2011 Global Tuberculosis Control 2011. (http://www.who.int/tb/publications/global_report/en/)

Schultz 2010 Integrative Genomic Profiling of Human Prostate Cancer Cancer Cell Vol 18, Issue 1, 11-22 Metcalfe et al 2010 ("Interferon-y release assays for active pulmonary tuberculosis diagnosis in adults in low- and middle-income countries: systematic review and meta-analysis" The Journal of infectious diseases 204 Suppl 4).

Berry MP, Graham CM, McNab FW, et al. An interferon-inducible neutrophil-driven blood transcriptional signature in human tuberculosis. Nature 2010;466:973-7.

Denoeud F, Aury JM, Da Silva C, et al, F; Artiguenave (2008). "Annotating genomes with massive-scale RNA sequencing". Genome Biol. 9 (12): R175.

Velculescu VE, Zhang L, Vogelstein B, Kinzler KW. (1995) "Serial analysis of gene expression". Science 270 (5235): 484-7.

Irizarry RA, Hobbs B, Collin F, Beazer-Barclay YD, Antonellis KJ, Scherf U, Speed TP. Exploration, normalization, and summaries of high density oligonucleotide array probe level data. Biostatistics. 2003 Apr; 4(2):249-64.

Tusher, Virginia Goss; Tibshirani, Robert; Chu, Gilbert (2001). "Significance analysis of microarrays applied to the ionizing radiation response". Proceedings of the National Academy of Sciences of the United States of America 98 (18): 5116-5121.

Zou, H., and Hastie, T. 2005. Regularization and variable selection via the elastic net. J Roy Stat Soc Ser B 67:301-320. The relevant algorithms of the fully functioning elastic net are incorporates herein by reference.

Crampin AC, Floyd S, Mwaungulu F, et al. Comparison of two versus three smears in identifying culture-positive tuberculosis patients in a rural African setting with high HIV prevalence. Int J Tuberc Lung Dis 2001;5:994-9.

Hussain R, Kaleem A, Shahid F, et al. Cytokine profiles using whole-blood assays can discriminate between tuberculosis patients and healthy endemic controls in a BCG-vaccinated population. J Immunol Methods 2002;264:95-108.

Franken KL, Hiemstra HS, van Meijgaarden KE, et al. Purification of his-tagged proteins by immobilized chelate affinity chromatography: the benefits from the use of organic solvent. Protein Expr Purif 2000;18:95-9.

Benjamini Y, Hochberg Y. Controlling the False Discovery Rate - a Practical and Powerful Approach to Multiple Testing. J Roy Stat Soc B Met 1995;57:289-300.

Joosten SA, Goeman JJ, Sutherland JS, et al. Identification of biomarkers for tuberculosis disease using a novel dual-color RT-MLPA assay. Genes Immun 2012;13:71-82.

Eldering E, Spek CA, Aberson HL, et al. Expression profiling via novel multiplex assay allows rapid assessment of gene regulation in defined signalling pathways. Nucleic Acids Res 2003;31:e153.

Maertzdorf J, Ota M, Repsilber D, et al. Functional correlations of pathogenesis-driven gene expression signatures in tuberculosis. PLoS One 2011a;6:e26938.

Maertzdorf J, Repsilber D, Parida SK, et al. Human gene expression profiles of susceptibility and resistance in tuberculosis. Genes Immun 2011b;12:15-22.

Jacobsen M, Repsilber D, Gutschmidt A, et al. Candidate biomarkers for discrimination between infection and disease caused by Mycobacterium tuberculosis. J Mol Med (Berl) 2007;85:613-21.

Cox JA, Lukande RL, Lucas S, Nelson AM, Van Marck E, Colebunders R. Autopsy causes of death in HIV-positive individuals in sub-Saharan Africa and correlation with clinical diagnoses. AIDS Rev 2010;12:183-94.

Ansari NA, Kombe AH, Kenyon TA, et al. Pathology and causes of death in a group of 128 predominantly HIV-positive patients in Botswana, 1997-1998. Int J Tuberc Lung Dis 2002;6:55-63.

Maertzdorf J, Weiner J, 3rd, Mollenkopf HJ, et al. Common patterns and disease-related signatures in tuberculosis and sarcoidosis. Proc Natl Acad Sci U S A 2012;109:7853-8.

Robin X, Turck N, Hainard A, Tiberti N, Lisacek F, et al. (2011) pROC: an open-source package for R and S+ to analyze and compare ROC curves. BMC Bioinformatics 12: 77.

Carpenter J, Bithell J (2000) Bootstrap confidence intervals: when, which, what? A practical guide for medical statisticians. Stat Med 19: 1141-1164.

Clopper CJ, Pearson ES (1934) The use of confidence or fiducial limits illustrated in the case of the binomial. Biometrika 26: 404-413.

Altman DG, Bland JM (1994) Diagnostic tests 2: Predictive values. BMJ 309: 102.

Simel DL, Samsa GP, Matchar DB (1991) Likelihood ratios with confidence: sample size estimation for diagnostic test studies. J Clin Epidemiol 44: 763-770.

1. Ideker T, Galitski T, & Hood L (2001) A new approach to decoding life: Systems biology. Annu. Rev. Genomics Hum. Genet. 2:343-372.
2. Chadeau-Hyam M, et al. (2013) Deciphering the complex: Methodological overview of statistical models to derive OMICS-based biomarkers. Environ. Mol. Mutagen. 54(7):542-557.
3. Guyon I, Weston J, Barnhill S, & Vapnik V (2002) Gene selection for cancer classification using support vector machines. Mach. Learn. 46(1-3):389-422.
4. Geurts P, et al. (2005) Proteomic mass spectra classification using decision tree based ensemble methods. Bioinformatics 21(14):3138-3145.
5. Dudoit S, Fridlyand J, & Speed TP (2002) Comparison of discrimination methods for the classification of tumors using gene expression data. J. Am. Stat. Assoc. 97(457):77-87.
6. Greenland S (1989) MODELING AND VARIABLE SELECTION IN EPIDEMIOLOGIC ANALYSIS. Am. J. Public Health 79(3):340-349.
7. Hoggart CJ, Whittaker JC, De Iorio M, & Balding DJ (2008) Simultaneous Analysis of All SNPs in Genome-Wide and Re-Sequencing Association Studies. PLoS Genet. 4(7):8.
8. Tibshirani R (1996) Regression shrinkage and selection via the Lasso. J. R. Stat. Soc. Ser. B-Methodol. 58(1):267-288.
9. Zou H & Hastie T (2005) Regularization and variable selection via the elastic net. J. R. Stat. Soc. Ser. B-Stat. Methodol. 67:301-320.
10. Zhu J & Hastie T (2004) Classification of gene microarrays by penalized logistic regression. Biostatistics 5(3):427-443.
11. Wold S, Esbensen K, & Geladi P (1987) PRINCIPAL COMPONENT ANALYSIS. Chemometrics Intell. Lab. Syst. 2(1-3):37-52.
12. Barker M & Rayens W (2003) Partial least squares for discrimination. J. Chemometr. 17(3):166-173.
13. Fort G & Lambert-Lacroix S (2005) Classification using partial least squares with penalized logistic regression. Bioinformatics 21(7):1104-1111.
14. Le Cao KA, Rossouw D, Robert-Granie C, & Besse P (2008) A Sparse PLS for Variable Selection when Integrating Omics Data. Stat. Appl. Genet. Mol. Biol. 7(1):32.
15. Bylesjo M, et al. (2006) OPLS discriminant analysis: combining the strengths of PLS-DA and SIMCA classification. J. Chemometr. 20(8-10):341-351.
16. Westerhuis JA, et al. (2008) Assessment of PLSDA cross validation. Metabolomics 4(1):81-89.
17. Golub TR, et al. (1999) Molecular classification of cancer: class discovery and class prediction by gene expression monitoring. Science 286(5439):531-537.
18. Meinshausen N & Buhlmann P (2010) Stability selection. J. R. Stat. Soc. Ser. B-Stat. Methodol. 72:417-473.
19. Huang XH, et al. (2005) A comparative study of discriminating human heart failure etiology using gene expression profiles. BMC Bioinformatics 6:15.
20. Liu H, Li J, & Wong L (2002) A comparative study on feature selection and classification methods using gene expression profiles and proteomic patterns. Genome informatics. International Conference on Genome Informatics 13:51-60.
21. Shi L, et al. (2006) The MicroArray Quality Control (MAQC) project shows inter- and intraplatform reproducibility of gene expression measurements. Nature biotechnology 24(9):1151-1161.
22. van't Veer LJ, et al. (2002) Gene expression profiling predicts clinical outcome of breast cancer. Nature 415(6871):530-536.
23. Petricoin EF, et al. (2002) Serum proteomic patterns for detection of prostate cancer. J. Natl. Cancer Inst. 94(20):1576-1578.
24. Stamler J, et al. (2003) INTERMAP: background, aims, design, methods, and descriptive statistics (nondietary). J. Hum. Hypertens. 17(9):591-608.
25. Dennis B, et al. (2003) INTERMAP: the dietary data - process and quality control. J. Hum. Hypertens. 17(9):609-622.
26. Kaforou M, et al. (2013) Detection of tuberculosis in HIV-infected and -uninfected African adults using whole blood RNA expression signatures: a case-control study. PLoS medicine 10(10):e1001538.
27. Berry MP, et al. (2010) An interferon-inducible neutrophil-driven blood transcriptional signature in human tuberculosis. Nature 466(7309):973-977.
28. Morey JS, Ryan JC, & Van Dolah FM (2006) Microarray validation: factors influencing correlation between oligonucleotide microarrays and real-time PCR. Biol Proced Online 8:175-193.
29. Somorjai RL, Dolenko B, & Baumgartner R (2003) Class prediction and discovery using gene microarray and proteomics mass spectroscopy data: curses, caveats, cautions. Bioinformatics 19(12):1484-1491.
30. Tibshirani R, Hastie T, Narasimhan B, & Chu G (2002) Diagnosis of multiple cancer types by shrunken centroids of gene expression. P Natl Acad Sci USA 99(10):6567-6572.
31. Kaforou M, et al. (2013) Detection of Tuberculosis in HIV-Infected and -Uninfected African Adults Using Whole Blood RNA Expression Signatures: A Case-Control Study. PLos Med. 10(10):16.
32. Wang Y, Zheng DL, Tan QL, Wang MX, & Gu LQ (2011) Nanopore-based detection of circulating microRNAs in lung cancer patients. Nat. Nanotechnol. 6(10):668-674.
33. de la Rica R & Stevens MM (2012) Plasmonic ELISA for the ultrasensitive detection of disease biomarkers with the naked eye. Nat. Nanotechnol. 7(12):821-824.
34. Lowe SB, Dick JAG, Cohen BE, & Stevens MM (2012) Multiplex Sensing of Protease and Kinase Enzyme Activity via Orthogonal Coupling of Quantum Dot Peptide Conjugates. ACS Nano 6(1):851-857.
35. Morrow TJ, Li MW, Kim J, Mayer TS, & Keating CD (2009) Programmed Assembly of DNA-Coated Nanowire Devices. Science 323(5912):352-352.
36. Cloarec O, et al. (2005) Statistical total correlation spectroscopy: An exploratory approach for latent biomarker identification from metabolic H-1 NMR data sets. Anal. Chem. 77(5):1282-1289.
37. Altelaar AFM, Munoz J, & Heck AJR (2013) Next-generation proteomics: towards an integrative view of proteome dynamics. Nat Rev Genet 14(1):35-48.
38. Guo L-H, et al. (2013) Plasma Proteomics for the Identification of Alzheimer Disease. Publish Ahead of Print:10.1097/WAD.1090b1013e31827b31860d31822.
39. Stein DR, Burgener A, & Ball TB (2013) Proteomics as a novel HIV immune monitoring tool. 8(2):140-146 110.1097/COH.1090b1013e32835d33271.
40. de Wit M, Fijneman RJA, Verheul HMW, Meijer GA, & Jimenez CR (2013) Proteomics in colorectal cancer translational research: Biomarker discovery for clinical applications. Clinical Biochemistry (0).
41. Paulo JA, et al. (2011) Mass spectrometry-based proteomics of endoscopically collected pancreatic fluid in chronic pancreatitis research. Proteom. Clin. Appl. 5(3-4):109-120.
42. Kentsis A, et al. (2012) Urine proteomics for discovery of improved diagnostic markers of Kawasaki disease. EMBO Molecular Medicine 5(2):210-220.
43. Mattison HA, Stewart T, & Zhang J (2012) Applying bioinformatics to proteomics: Is machine learning the answer to biomarker discovery for PD and MSA? Movement Disorders 27(13):1595-1597.
44. Zhai X-h, Yu J-k, Lin C, Wang L-d, & Zheng S (2013) Combining proteomics, serum biomarkers and bioinformatics to discriminate between esophageal squamous cell carcinoma and pre-cancerous lesion. Journal of Zhejiang University-Science B 13(12):964-971.

**Claims**

1. A method comprising the step of detecting modulation in gene expression data, generated from RNA levels in a subject derived sample, **characterised in that** a 6 gene signature *GBP6, TMCC1, PRDM1, ARG1, CREB5* and *VPREB3* is employed to discriminate active tuberculosis (TB) from other diseases in the presence of a complicating factor.

2. The method according to claim 1, wherein the complicating factor is the presence of a co-morbidity, for example wherein the co-morbidity is selected from a malignancy, HIV, malaria, pneumonia, Lower Respiratory Tract Infection, Pneumocystis Jirovecii Pneumonia, pelvic inflammatory disease, Urinary Tract Infection, bacterial or viral meningitis, hepatobiliary disease, cryptococcal meningitis, non-TB pleural effusion, empyema, gastroenteritis, peritonitis, gastric ulcer and gastritis.

3. The method according to claim 2, wherein the co-morbidity is HIV.

4. The method according to any one of claims 1 to 3, wherein 3 genes in the 6 gene signature are up-regulated, for example wherein the genes PRDM1, GBP6 and CREB5 are up-regulated.

5. The method according to claim 4, wherein the remaining genes in the signature are down-regulated, for example wherein the genes *VPREB3, ARG1* and *TMCC1* are down-regulated.

6. The method according to any one of claims 1 to 5, further comprising the steps of:

a. optionally normalising and/or scaling numeric values of the modulation,
b. taking the normalised and/or scaled numeric values or the raw numeric values, each of which comprise both positive and/or negative numeric values and designating all said numeric values to be negative or alternatively all positive,
c. optionally refining the discriminatory power of one or more up-regulated genes and down-regulated genes by statistically weighting some of the numeric values associated therewith, and
d. summating the positive or negative numeric values obtained from step b) or step c) to provide a composite expression score,

wherein the composite expression score obtained from step d) is compared to a control and the comparison allows the sample to be designated as positive or negative for the relevant infection.

7. The method according to any one of the preceding claims, wherein the gene signature further incorporates one or more such as 1, 2, 3, 4, or 5 housekeeping genes.

8. The method according to any one of the preceding claims, wherein a patient derived sample is employed in the method.

9. The method according to any one of the preceding claims, which comprises the further step of prescribing a treatment for the subject based on the results of the analysis of said gene signature.

10. The method according to claim 9, wherein the treatment is a treatment for active TB.

11. A set of primers for use in multiplex PCR, wherein the set of primers consists of nucleic acid sequences specific to a polynucleotide gene transcript for each of the following genes:
*GBP6, TMCC1, PRDM1, ARG1, CREB5* and *VPREB3.*

12. The set of primers according to claim 11, further comprising primers specific to one or more such as 1, 2, 3, 4, or 5 housekeeping genes.

13. The set of primers according to claims 11 or 12, wherein the primers are specific to a sequence given in any one of SEQ ID NOs: 1 to 16.

14. A point of care test for identifying active TB in a subject consisting of the set of primers defined in any one of claims 11 to 13.

15. Use of the set of primers defined in any one of claims 11 to 13 in an assay to detect active TB infection in a sample, for example a blood sample.

**Patentansprüche**

1. Verfahren, umfassend den Schritt des Erkennens einer Modulation in Genexpressionsdaten, die aus RNA-Pegeln in einer von einem Patienten stammenden Probe erzeugt werden, **dadurch gekennzeichnet, dass** eine 6-Gensignatur *GBP6, TMCC1, PRDM1, ARG1, CREB5* und *VPREB3* verwendet wird, um aktive Tuberkulose (*tuberculosis* - TB) von anderen Krankheiten in Gegenwart eines Komplikationsfaktors zu unterscheiden.

2. Verfahren nach Anspruch 1, wobei der Komplikationsfaktor das Vorhandensein einer Komorbidität ist, wobei die Komorbidität beispielsweise ausgewählt ist aus einer Malignität, HIV, Malaria, Pneumonie, Infektion des unteren Respirationstrakts, Pneumocystis-Jirovecii-Pneumonie, entzündlicher Krankheit im Becken, Harnwegsinfektion, bakterieller oder viraler Meningitis, hepatobiliärer Erkrankung, Kryptokokken-Meningitis, Nicht-TB-Pleuraerguss, Empyem, Gastroenteritis, Peritonitis, Magengeschwür und Gastritis.

3. Verfahren nach Anspruch 2, wobei die Komorbidität HIV ist.

4. Verfahren nach einem der Ansprüche 1 bis 3, wobei 3 Gene in der 6-Gensignatur hochreguliert sind, wobei z. B. die Gene *PRDM1, GBP6* und *CREB5* hochreguliert sind.

5. Verfahren nach Anspruch 4, wobei die verbleibenden Gene in der Signatur herunterreguliert sind, wobei z. B. die Gene *VPREB3, ARG1* und *TMCC1* herunterreguliert sind.

6. Verfahren nach einem der Ansprüche 1 bis 5, ferner umfassend die folgenden Schritte:

a. optionales Normieren und/oder Skalieren von numerischen Werten der Modulation,
b. Nehmen der normierten und/oder skalierten numerischen Werte oder der numerischen Rohwerte, die jeweils sowohl positive und/oder negative numerische Werte umfassen, und Bezeichnen aller numerischen Werte als negativ oder alternativ aller numerischen Werte als positiv,
c. optionales Verfeinern der Unterscheidungskraft von einem oder mehreren hochregulierten Genen und herunterregulierten Genen durch statistisches Gewichten einiger der ihnen zugeordneten numerischen Werte, und
d. Summieren der in Schritt b) oder Schritt c) erhaltenen positiven oder negativen numerischen Werte, um einen zusammengesetzten Expressionswert zu liefern,

wobei der in Schritt d) erhaltene zusammengesetzte Expressionswert mit einer Kontrolle verglichen wird und der Vergleich es erlaubt, die Probe als positiv oder negativ für die relevante Infektion zu bezeichnen.

**7.** Verfahren nach einem der vorhergehenden Ansprüche, wobei die Gensignatur ferner ein oder mehrere wie 1, 2, 3, 4 oder 5 Housekeeping-Gene einschließt.

**8.** Verfahren nach einem der vorhergehenden Ansprüche, wobei eine vom Patienten stammende Probe in dem Verfahren verwendet wird.

**9.** Verfahren nach einem der vorhergehenden Ansprüche, das den weiteren Schritt des Verordnens einer Behandlung für den Patienten basierend auf den Ergebnissen der Analyse der Gensignatur umfasst.

**10.** Verfahren nach Anspruch 9, wobei die Behandlung eine Behandlung für aktive TB ist.

**11.** Satz von Primern zur Verwendung in der Multiplex-PCR, wobei der Satz von Primern aus Nukleinsäuresequenzen besteht, die für ein Polynukleotid-Gentranskript für jedes der folgenden Gene spezifisch sind:
*GBP6, TMCC1, PRDM1, ARG1, CREB5* und *VPREB3.*

**12.** Satz von Primern nach Anspruch 11, ferner umfassend Primer, die spezifisch für ein oder mehrere wie 1, 2, 3, 4 oder 5 Housekeeping-Gene sind.

**13.** Satz von Primern nach Anspruch 11 oder 12, wobei die Primer spezifisch für eine Sequenz sind, die in einer der SEQ ID NOs: 1 bis 16 angegeben ist.

**14.** Point-of-Care-Test zum Identifizieren von aktiver TB in einem Patienten, bestehend aus dem Satz von Primern, der in einem der Ansprüche 11 bis 13 definiert ist.

**15.** Verwendung des in einem der Ansprüche 11 bis 13 definierten Satzes von Primern in einem Assay zum Erkennen einer aktiven TB-Infektion in einer Probe, z. B. einer Blutprobe.

**Revendications**

**1.** Procédé comprenant l'étape consistant à détecter la modulation dans les données d'expression génique, générées à partir des niveaux d'ARN dans un échantillon dérivé du sujet, **caractérisé en ce qu'**une signature à 6 gènes *GBP6, TMCC1, PRDM1, ARG1, CREB5* et *VPREB3* est utilisée pour distinguer la tuberculose active (TB) d'autres maladies en présence d'un facteur de complication.

**2.** Procédé selon la revendication 1, dans lequel le facteur de complication est la présence d'une comorbidité, par exemple dans lequel la comorbidité est choisie parmi une tumeur maligne, le VIH, le paludisme, la pneumonie, l'infection des voies respiratoires inférieures, la pneumonie à Pneumocystis Jirovecii, la maladie inflammatoire pelvienne, l'infection des voies urinaires, la méningite bactérienne ou virale, la maladie hépatobiliaire, la méningite cryptococcique, l'épanchement pleural non tuberculeux, l'empyème, la gastro-entérite, la péritonite, l'ulcère gastrique et la gastrite.

**3.** Procédé selon la revendication 2, dans lequel la comorbidité est le VIH.

**4.** Procédé selon l'une quelconque des revendications 1 à 3, dans lequel 3 gènes dans la signature à 6 gènes sont régulés positivement, par exemple dans lequel les gènes PRDM1, GBP6 et CREB5 sont régulés positivement.

**5.** Procédé selon la revendication 4, dans lequel les gènes restants dans la signature sont régulés négativement, par exemple dans lequel les gènes *VPREB3, ARG1* et *TMCC1* sont régulés négativement.

**6.** Procédé selon l'une quelconque des revendications 1 à 5, comprenant en outre les étapes consistant à :

a. éventuellement normaliser et/ou mettre à l'échelle des valeurs numériques de la modulation,

b. prendre les valeurs numériques normalisées et/ou mises à l'échelle ou les valeurs numériques brutes, chacune comprenant à la fois des valeurs numériques positives et/ou négatives et désigner toutes lesdites valeurs numériques comme étant négatives ou alternativement toutes positives,
c. éventuellement affiner le pouvoir discriminatoire d'un ou de plusieurs gènes régulés positivement et de gènes régulés négativement en pondérant statistiquement certaines des valeurs numériques associées à ceux-ci, et
d. additionner les valeurs numériques positives ou négatives obtenues à l'étape b) ou à l'étape c) pour fournir un score d'expression composite,

dans lequel le score d'expression composite obtenu à l'étape d) est comparé à un témoin et la comparaison permet de désigner l'échantillon comme positif ou négatif pour l'infection concernée.

**7.** Procédé selon l'une quelconque des revendications précédentes, dans lequel la signature génique incorpore en outre un ou plusieurs tels que 1, 2, 3, 4 ou 5 gènes domestiques.

**8.** Procédé selon l'une quelconque des revendications précédentes, dans lequel un échantillon dérivé du patient est utilisé dans le procédé.

**9.** Procédé selon l'une quelconque des revendications précédentes, qui comprend l'étape supplémentaire de prescription d'un traitement pour le sujet en fonction des résultats de l'analyse de ladite signature génique.

**10.** Procédé selon la revendication 9, dans lequel le traitement est un traitement pour la tuberculose active.

**11.** Ensemble d'amorces destiné à être utilisé dans une PCR multiplex, dans lequel l'ensemble d'amorces est constitué de séquences d'acides nucléiques spécifiques d'un transcrit de gène polynucléotidique pour chacun des gènes suivants :
*GBP6, TMCC1, PRDM1, ARG1, CREB5* et *VPREB3.*

**12.** Ensemble d'amorces selon la revendication 11, comprenant en outre des amorces spécifiques à un ou plusieurs tels que 1, 2, 3, 4 ou 5 gènes domestiques.

**13.** Ensemble d'amorces selon les revendications 11 ou 12, dans lequel les amorces sont spécifiques à une séquence donnée dans l'une quelconque des SEQ ID No : 1 à 16.

**14.** Test sur le lieu de soin pour identifier la tuberculose active chez un sujet constitué de l'ensemble d'amorces définies dans l'une quelconque des revendications 11 à 13.

**15.** Utilisation de l'ensemble d'amorces défini dans l'une quelconque des revendications 11 à 13 dans un dosage pour détecter une infection active par la tuberculose dans un échantillon, par exemple un échantillon de sang.

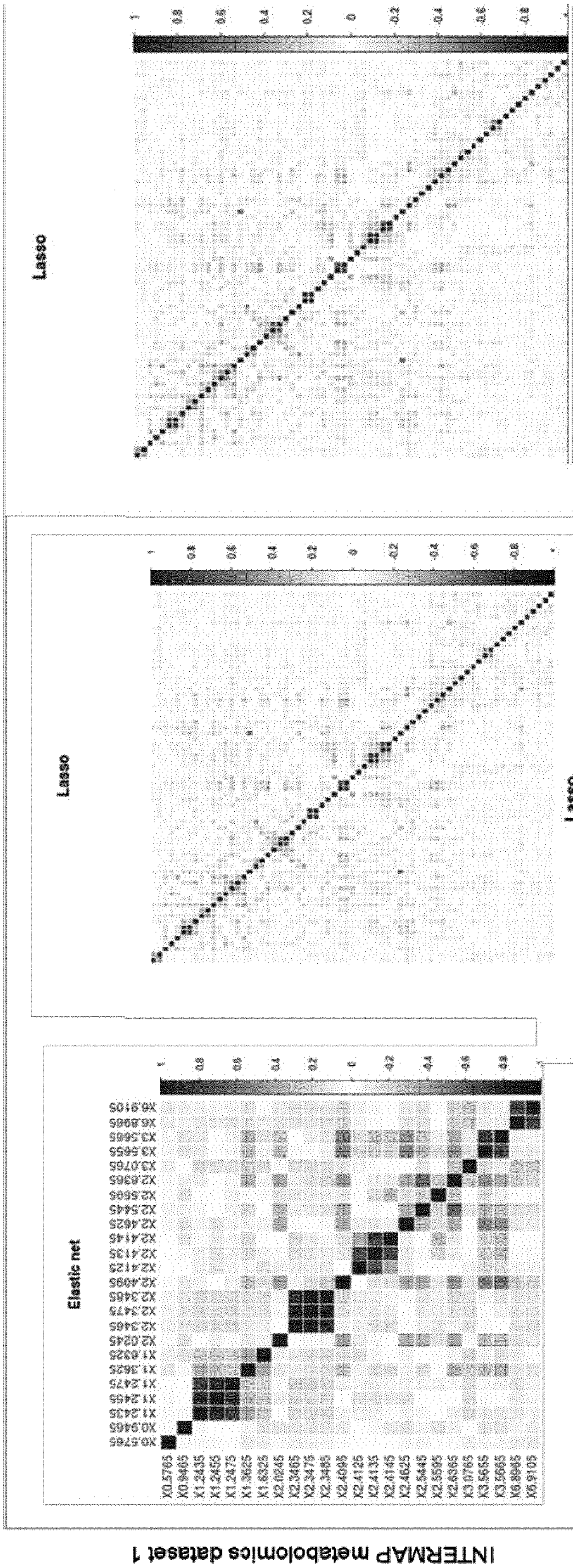


Figure 1A

SUBSTITUTE SHEET (RULE 26)

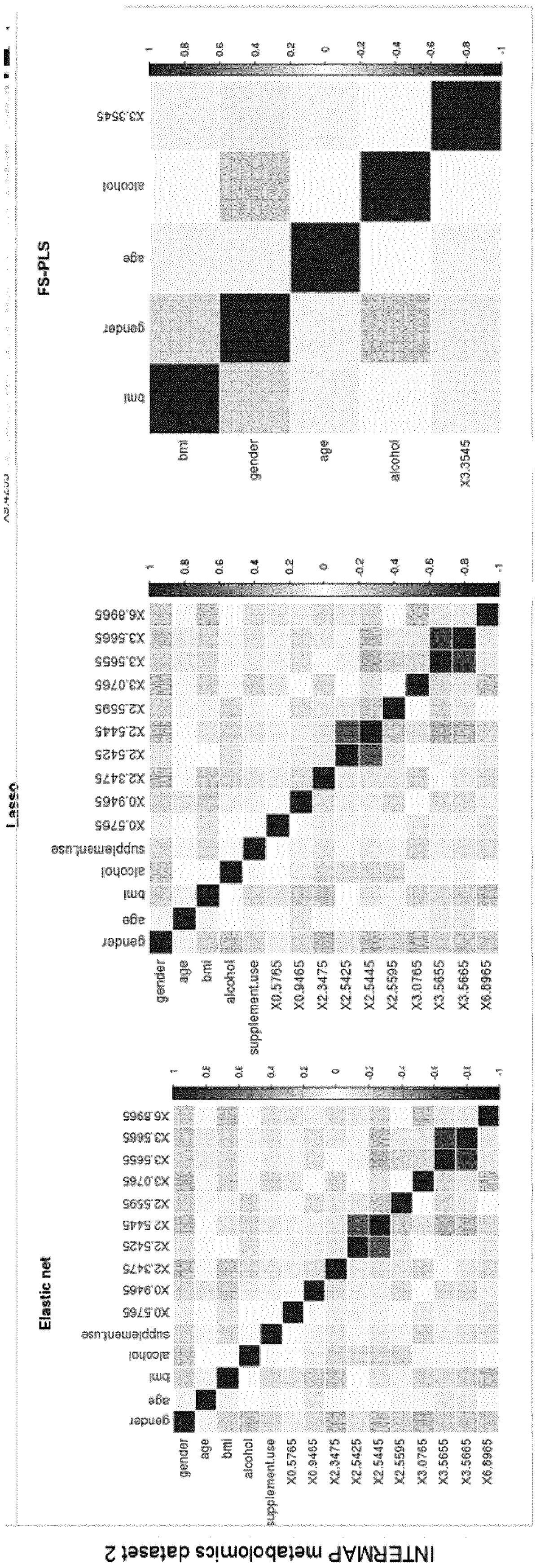
INTERMAP metabolomics dataset 2
Elastic net
Lasso
FS-PLS
gender
age
bmi
alcohol
supplement.use
X0.5765
X0.9465
X2.3475
X2.5425
X2.5445
X2.5595
X3.0765
X3.5655
X3.5665
X6.8965
X3.3545
1
0.8
0.6
0.4
0.2
0
-0.2
-0.4
-0.6
-0.8
-1

Figure 1B

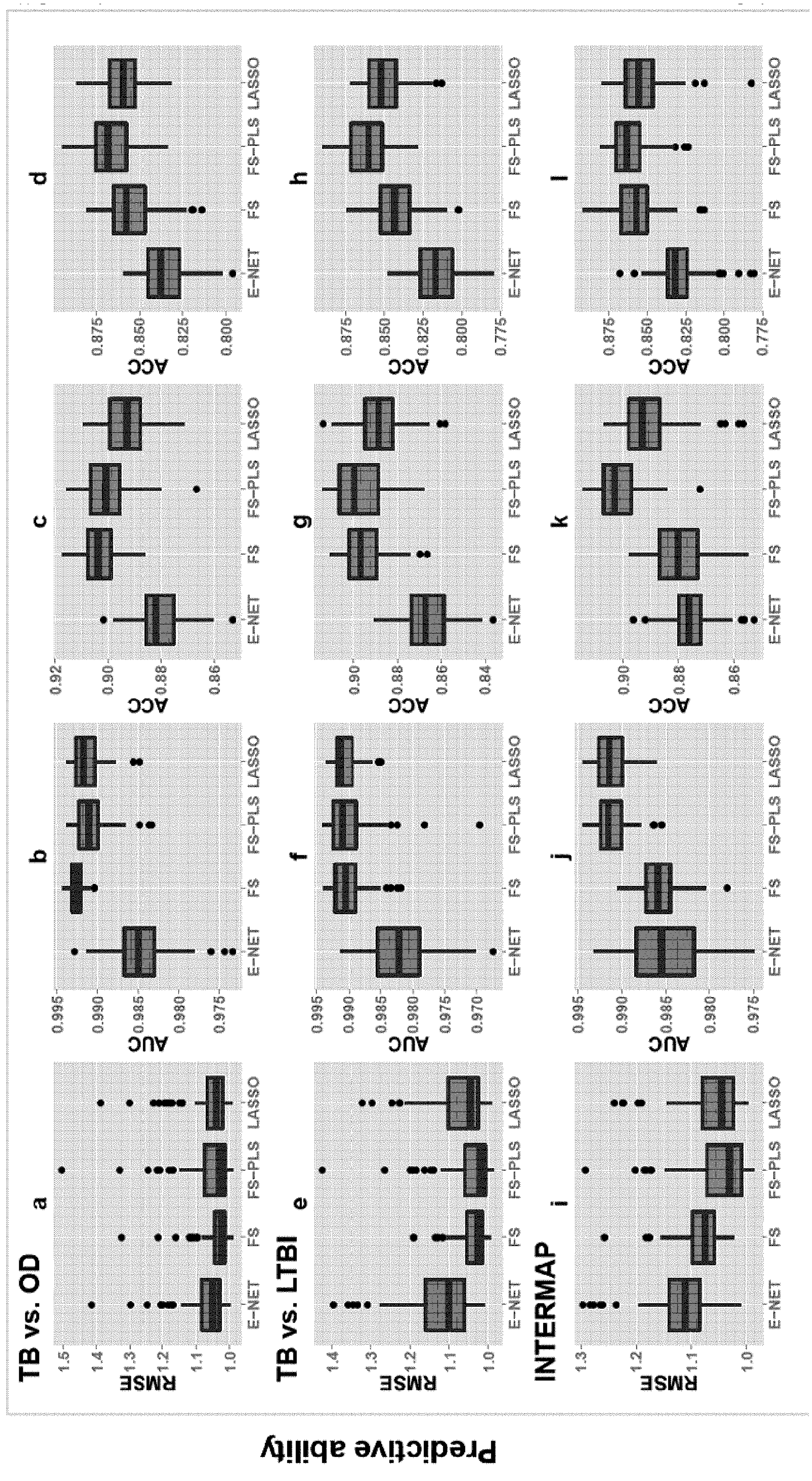
Predictive ability
TB vs. OD
TB vs. LTBI
INTERMAP
a
b
c
d
e
f
g
h
i
j
k
l
RMSE
AUC
ACC
E-NET
FS
FS-PLS
LASSO

Figure 2A

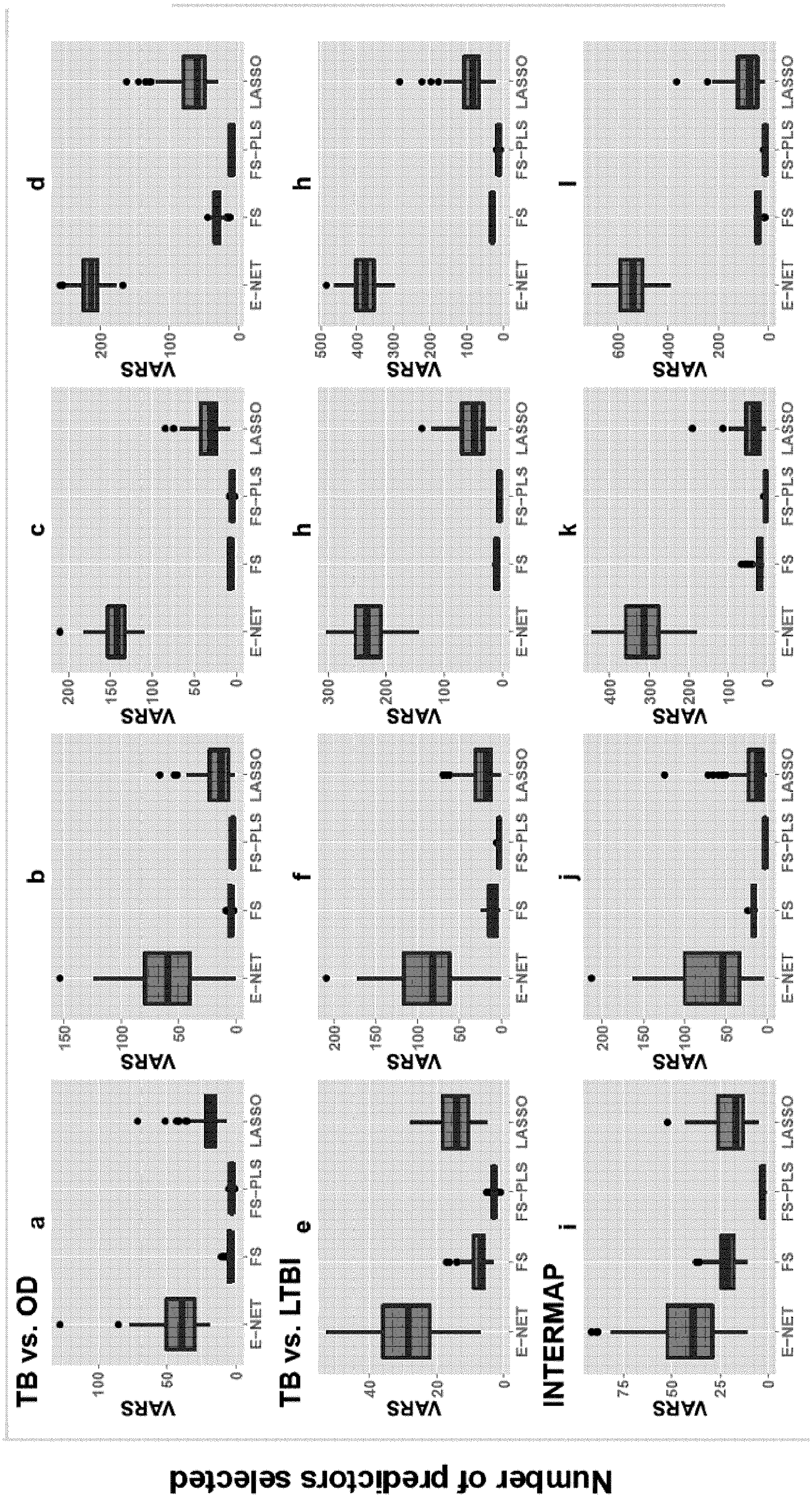
TB vs. OD
TB vs. LTBI
INTERMAP
a
b
c
d
e
f
h
h
i
j
k
l
VARS
E-NET
FS
FS-PLS
LASSO
Number of predictors selected

Figure 2B

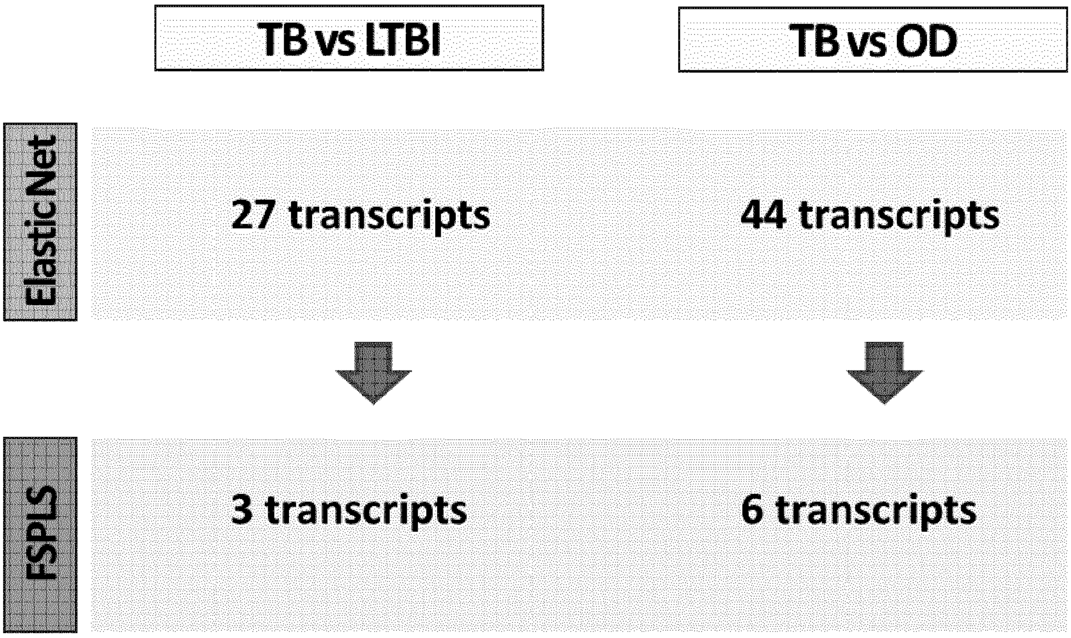
TB vs LTBI
TB vs OD
ElasticNet
27 transcripts
44 transcripts
FSPLS
3 transcripts
6 transcripts

Figure 3

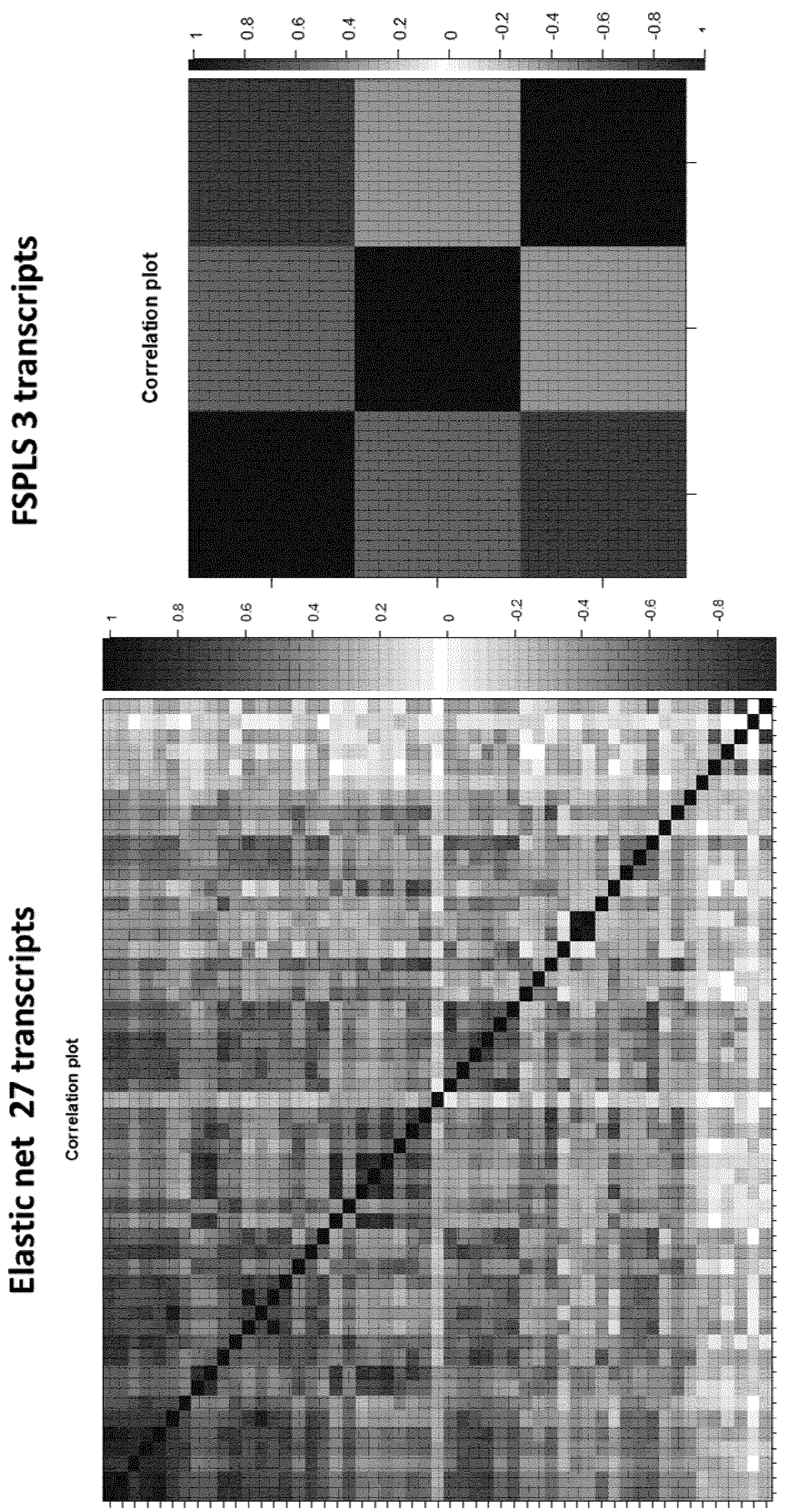
Elastic net 27 transcripts
Correlation plot
1
0.8
0.6
0.4
0.2
0
-0.2
-0.4
-0.6
-0.8
FSPLS 3 transcripts
Correlation plot
1
0.8
0.6
0.4
0.2
0
-0.2
-0.4
-0.6
-0.8

Figure 4

A

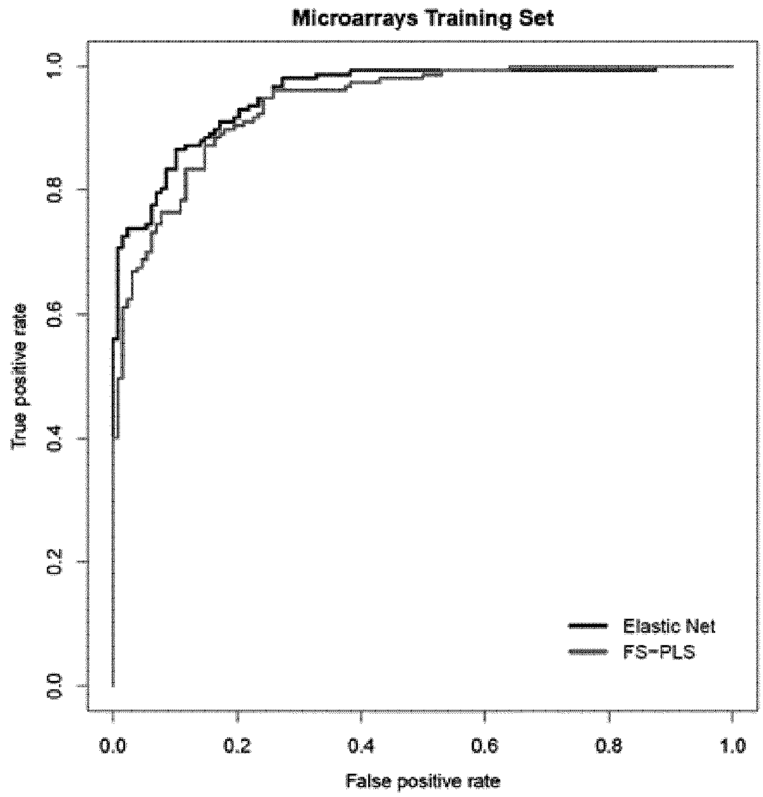
Microarrays Training Set
True positive rate
0.0
0.2
0.4
0.6
0.8
1.0
False positive rate
0.0
0.2
0.4
0.6
0.8
1.0
Elastic Net
FS−PLS

B

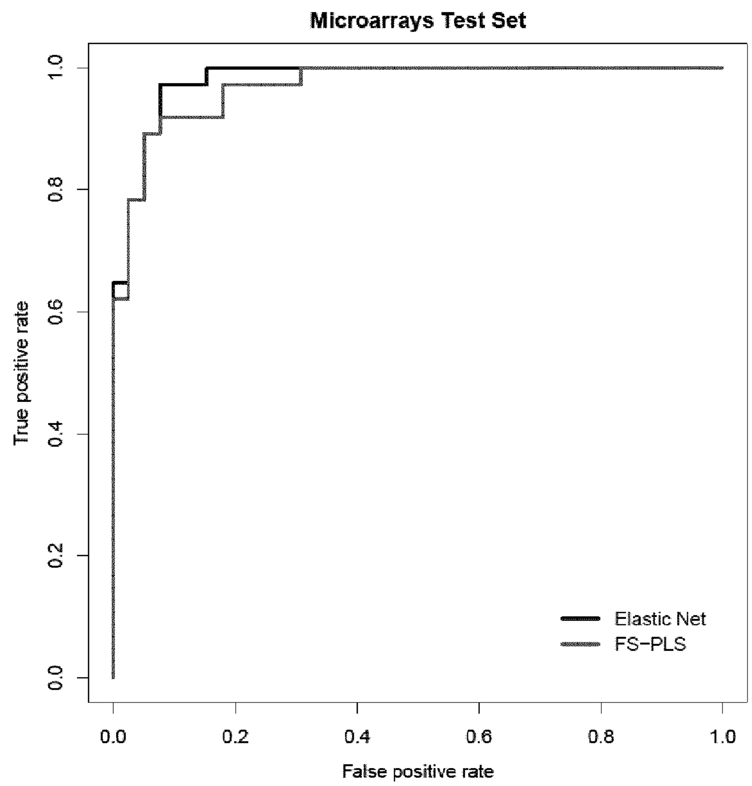
Microarrays Test Set
True positive rate
0.0
0.2
0.4
0.6
0.8
1.0
False positive rate
0.0
0.2
0.4
0.6
0.8
1.0
Elastic Net
FS−PLS

**Figure 5**

C

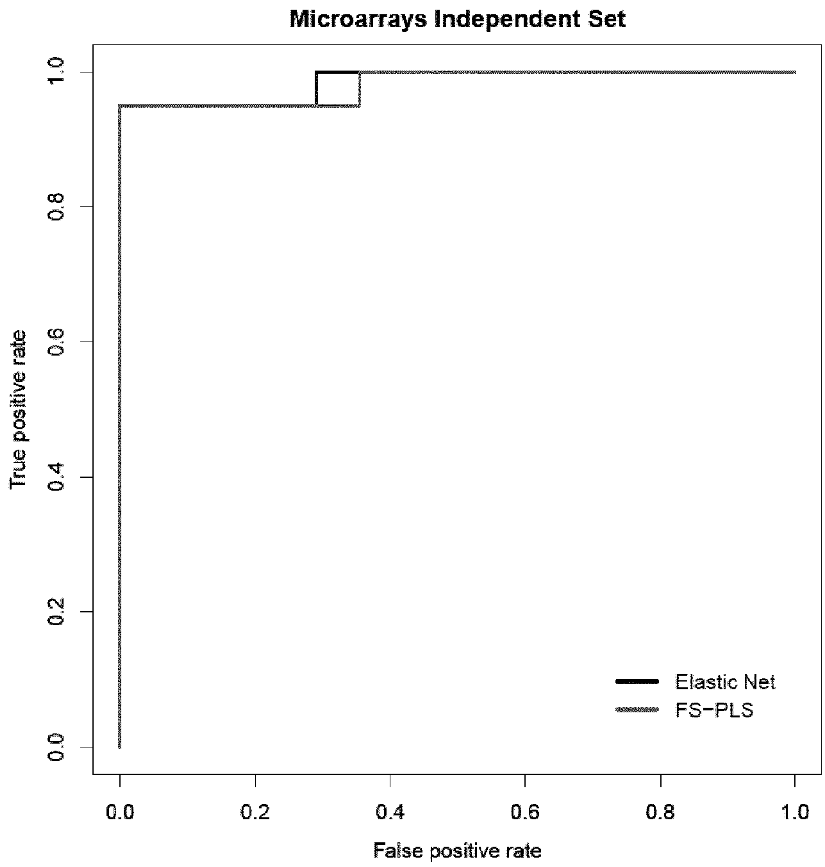
Microarrays Independent Set
True positive rate
0.0
0.2
0.4
0.6
0.8
1.0
False positive rate
0.0
0.2
0.4
0.6
0.8
1.0
Elastic Net
FS-PLS

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014019977 A


### Non-patent literature cited in the description


- **PARKER et al.** *J. Clinical Oncology,* 2009
- **PERU et al.** *Nature,* 2000
- *WHO report 2011 Global Tuberculosis Control,* 2011
- **SCHULTZ 2010 INTEGRATIVE GENOMIC PROFILING OF HUMAN PROSTATE CANCER CANCER.** *Schultz 2010 Integrative Genomic Profiling of Human Prostate Cancer Cancer Cell,* vol. 18 (1), 11-22
- **METCALFE et al.** Interferon-y release assays for active pulmonary tuberculosis diagnosis in adults in low- and middle-income countries: systematic review and meta-analysis. *The Journal of infectious diseases,* 2010, vol. 204 (4
- **BERRY MP ; GRAHAM CM ; MCNAB FW et al.** An interferon-inducible neutrophil-driven blood transcriptional signature in human tuberculosis. *Nature,* 2010, vol. 466, 973-7
- **DENOEUD F ; AURY JM ; DA SILVA C ; F; ARTIGUENAVE et al.** Annotating genomes with massive-scale RNA sequencing. *Genome Biol.,* 2008, vol. 9 (12), R175
- **VELCULESCU VE ; ZHANG L ; VOGELSTEIN B ; KINZLER KW.** Serial analysis of gene expression. *Science,* 1995, vol. 270 (5235), 484-7
- **IRIZARRY RA ; HOBBS B ; COLLIN F ; BEAZER-BARCLAY YD ; ANTONELLIS KJ ; SCHERF U ; SPEED TP.** Exploration, normalization, and summaries of high density oligonucleotide array probe level data. *Biostatistics,* April 2003, vol. 4 (2), 249-64
- **TUSHER, VIRGINIA GOSS ; TIBSHIRANI, ROBERT ; CHU, GILBERT.** Significance analysis of microarrays applied to the ionizing radiation response. *Proceedings of the National Academy of Sciences of the United States of America,* 2001, vol. 98 (18), 5116-5121
- **ZOU, H. ; HASTIE, T.** Regularization and variable selection via the elastic net. *J Roy Stat Soc Ser B,* 2005, vol. 67, 301-320
- **CRAMPIN AC ; FLOYD S ; MWAUNGULU F et al.** Comparison of two versus three smears in identifying culture-positive tuberculosis patients in a rural African setting with high HIV prevalence. *Int J Tuberc Lung Dis,* 2001, vol. 5, 994-9
- **HUSSAIN R ; KALEEM A ; SHAHID F et al.** Cytokine profiles using whole-blood assays can discriminate between tuberculosis patients and healthy endemic controls in a BCG-vaccinated population. *J Immunol Methods,* 2002, vol. 264, 95-108
- **FRANKEN KL ; HIEMSTRA HS ; VAN MEIJGAARDEN KE et al.** Purification of his-tagged proteins by immobilized chelate affinity chromatography: the benefits from the use of organic solvent. *Protein Expr Purif,* 2000, vol. 18, 95-9
- **BENJAMINI Y ; HOCHBERG Y.** Controlling the False Discovery Rate - a Practical and Powerful Approach to Multiple Testing. *J Roy Stat Soc B Met,* 1995, vol. 57, 289-300
- **JOOSTEN SA ; GOEMAN JJ ; SUTHERLAND JS et al.** Identification of biomarkers for tuberculosis disease using a novel dual-color RT-MLPA assay. *Genes Immun,* 2012, vol. 13, 71-82
- **ELDERING E ; SPEK CA ; ABERSON HL et al.** Expression profiling via novel multiplex assay allows rapid assessment of gene regulation in defined signalling pathways. *Nucleic Acids Res,* 2003, vol. 31, e153
- **MAERTZDORF J ; OTA M ; REPSILBER D et al.** Functional correlations of pathogenesis-driven gene expression signatures in tuberculosis. *PLoS One,* 2011, vol. 6, e26938
- **MAERTZDORF J ; REPSILBER D ; PARIDA SK et al.** Human gene expression profiles of susceptibility and resistance in tuberculosis. *Genes Immun,* 2011, vol. 12, 15-22
- **JACOBSEN M ; REPSILBER D ; GUTSCHMIDT A et al.** Candidate biomarkers for discrimination between infection and disease caused by Mycobacterium tuberculosis. *J Mol Med (Berl),* 2007, vol. 85, 613-21

- **COX JA ; LUKANDE RL ; LUCAS S ; NELSON AM ; VAN MARCK E ; COLEBUNDERS R.** Autopsy causes of death in HIV-positive individuals in sub-Saharan Africa and correlation with clinical diagnoses. *AIDS Rev,* 2010, vol. 12, 183-94
- **ANSARI NA ; KOMBE AH ; KENYON TA et al.** Pathology and causes of death in a group of 128 predominantly HIV-positive patients in Botswana. *Int J Tuberc Lung Dis,* 1997, vol. 6, 55-63
- **MAERTZDORF J ; WEINER J, 3RD ; MOLLENKOPF HJ et al.** Common patterns and disease-related signatures in tuberculosis and sarcoidosis. *Proc Natl Acad Sci U S A,* 2012, vol. 109, 7853-8
- **ROBIN X ; TURCK N ; HAINARD A ; TIBERTI N ; LISACEK F et al.** pROC: an open-source package for R and S+ to analyze and compare ROC curves. *BMC Bioinformatics,* 2011, vol. 12, 77
- **CARPENTER J ; BITHELL J.** Bootstrap confidence intervals: when, which, what? A practical guide for medical statisticians. *Stat Med,* 2000, vol. 19, 1141-1164
- **CLOPPER CJ ; PEARSON ES.** The use of confidence or fiducial limits illustrated in the case of the binomial. *Biometrika,* 1934, vol. 26, 404-413
- **ALTMAN DG ; BLAND JM.** Diagnostic tests 2: Predictive values. *BMJ,* 1994, vol. 309, 102
- **SIMEL DL ; SAMSA GP ; MATCHAR DB.** Likelihood ratios with confidence: sample size estimation for diagnostic test studies. *J Clin Epidemiol,* 1991, vol. 44, 763-770
- **IDEKER T ; GALITSKI T ; HOOD L.** A new approach to decoding life: Systems biology. *Annu. Rev. Genomics Hum. Genet.,* 2001, vol. 2, 343-372
- **CHADEAU-HYAM M et al.** Deciphering the complex: Methodological overview of statistical models to derive OMICS-based biomarkers. *Environ. Mol. Mutagen.,* 2013, vol. 54 (7), 542-557
- **GUYON I ; WESTON J ; BARNHILL S ; VAPNIK V.** Gene selection for cancer classification using support vector machines. *Mach. Learn,* 2002, vol. 46 (1-3), 389-422
- **GEURTS P et al.** Proteomic mass spectra classification using decision tree based ensemble methods. *Bioinformatics,* 2005, vol. 21 (14), 3138-3145
- **DUDOIT S ; FRIDLYAND J ; SPEED TP.** Comparison of discrimination methods for the classification of tumors using gene expression data. *J. Am. Stat. Assoc.,* 2002, vol. 97 (457), 77-87
- **GREENLAND S.** MODELING AND VARIABLE SELECTION IN EPIDEMIOLOGIC ANALYSIS. *Am. J. Public Health,* 1989, vol. 79 (3), 340-349
- **HOGGART CJ ; WHITTAKER JC ; DE LORIO M ; BALDING DJ.** Simultaneous Analysis of All SNPs in Genome-Wide and Re-Sequencing Association Studies. *PLoS Genet.,* 2008, vol. 4 (7), 8
- **TIBSHIRANI R.** Regression shrinkage and selection via the Lasso. *J. R. Stat. Soc. Ser. B-Methodol,* 1996, vol. 58 (1), 267-288
- **ZOU H ; HASTIE T.** Regularization and variable selection via the elastic net. *J. R. Stat. Soc. Ser. B-Stat. Methodol,* 2005, vol. 67, 301-320
- **ZHU J ; HASTIE T.** Classification of gene microarrays by penalized logistic regression. *Biostatistics,* 2004, vol. 5 (3), 427-443
- **WOLD S ; ESBENSEN K ; GELADI P.** PRINCIPAL COMPONENT ANALYSIS. *Chemometrics Intell. Lab. Syst.,* 1987, vol. 2 (1-3), 37-52
- **BARKER M ; RAYENS W.** Partial least squares for discrimination. *J. Chemometr.,* 2003, vol. 17 (3), 166-173
- **FORT G ; LAMBERT-LACROIX S.** Classification using partial least squares with penalized logistic regression. *Bioinformatics,* 2005, vol. 21 (7), 1104-1111
- **LE CAO KA ; ROSSOUW D ; ROBERT-GRANIE C ; BESSE P.** A Sparse PLS for Variable Selection when Integrating Omics Data. *Stat. Appl. Genet. Mol. Biol.,* 2008, vol. 7 (1), 32
- **BYLESJO M et al.** OPLS discriminant analysis: combining the strengths of PLS-DA and SIMCA classification. *J. Chemometr.,* 2006, vol. 20 (8-10), 341-351
- **WESTERHUIS JA et al.** Assessment of PLSDA cross validation. *Metabolomics,* 2008, vol. 4 (1), 81-89
- **GOLUB TR et al.** Molecular classification of cancer: class discovery and class prediction by gene expression monitoring. *Science,* 1999, vol. 286 (5439), 531-537
- **MEINSHAUSEN N ; BUHLMANN P.** Stability selection. *J. R. Stat. Soc. Ser. B-Stat. Methodol.,* 2010, vol. 72, 417-473
- **HUANG XH et al.** A comparative study of discriminating human heart failure etiology using gene expression profiles. *BMC Bioinformatics,* 2005, vol. 6, 15
- **LIU H ; LI J ; WONG L.** A comparative study on feature selection and classification methods using gene expression profiles and proteomic patterns. Genome informatics. *International Conference on Genome Informatics,* 2002, vol. 13, 51-60
- **SHI L et al.** The MicroArray Quality Control (MAQC) project shows inter- and intraplatform reproducibility of gene expression measurements. *Nature biotechnology,* 2006, vol. 24 (9), 1151-1161
- **VAN'T VEER LJ et al.** Gene expression profiling predicts clinical outcome of breast cancer. *Nature,* 2002, vol. 415 (6871), 530-536
- **PETRICOIN EF et al.** Serum proteomic patterns for detection of prostate cancer. *J. Natl. Cancer Inst.,* 2002, vol. 94 (20), 1576-1578
- **STAMLER J et al.** INTERMAP: background, aims, design, methods, and descriptive statistics (nondietary). *J. Hum. Hypertens.,* 2003, vol. 17 (9), 591-608

- **DENNIS B et al.** INTERMAP: the dietary data - process and quality control. *J. Hum. Hypertens.,* 2003, vol. 17 (9), 609-622
- **KAFOROU M et al.** Detection of tuberculosis in HIV-infected and -uninfected African adults using whole blood RNA expression signatures: a case-control study. *PLoS medicine,* 2013, vol. 10 (10), e1001538
- **BERRY MP et al.** An interferon-inducible neutrophil-driven blood transcriptional signature in human tuberculosis. *Nature,* 2010, vol. 466 (7309), 973-977
- **MOREY JS ; RYAN JC ; VAN DOLAH FM.** Microarray validation: factors influencing correlation between oligonucleotide microarrays and real-time PCR. *Biol Proced Online,* 2006, vol. 8, 175-193
- **SOMORJAI RL ; DOLENKO B ; BAUMGARTNER R.** Class prediction and discovery using gene microarray and proteomics mass spectroscopy data: curses, caveats, cautions. *Bioinformatics,* 2003, vol. 19 (12), 1484-1491
- **TIBSHIRANI R ; HASTIE T ; NARASIMHAN B ; CHU G.** Diagnosis of multiple cancer types by shrunken centroids of gene expression. *P Natl Acad Sci USA,* 2002, vol. 99 (10), 6567-6572
- **KAFOROU M et al.** Detection of Tuberculosis in HIV-Infected and -Uninfected African Adults Using Whole Blood RNA Expression Signatures: A Case-Control Study. *PLos Med.,* 2013, vol. 10 (10), 16
- **WANG Y ; ZHENG DL ; TAN QL ; WANG MX ; GU LQ.** Nanopore-based detection of circulating microRNAs in lung cancer patients. *Nat. Nanotechnol.,* 2011, vol. 6 (10), 668-674
- **DE LA RICA R ; STEVENS MM.** Plasmonic ELISA for the ultrasensitive detection of disease biomarkers with the naked eye. *Nat. Nanotechnol.,* 2012, vol. 7 (12), 821-824
- **LOWE SB ; DICK JAG ; COHEN BE ; STEVENS MM.** Multiplex Sensing of Protease and Kinase Enzyme Activity via Orthogonal Coupling of Quantum Dot Peptide Conjugates. *ACS Nano,* 2012, vol. 6 (1), 851-857
- **MORROW TJ ; LI MW ; KIM J ; MAYER TS ; KEATING CD.** Programmed Assembly of DNA-Coated Nanowire Devices. *Science,* 2009, vol. 323 (5912), 352-352
- **CLOAREC O et al.** Statistical total correlation spectroscopy: An exploratory approach for latent biomarker identification from metabolic H-1 NMR data sets. *Anal. Chem.,* 2005, vol. 77 (5), 1282-1289
- **ALTELAAR AFM ; MUNOZ J ; HECK AJR.** Next-generation proteomics: towards an integrative view of proteome dynamics. *Nat Rev Genet,* 2013, vol. 14 (1), 35-48
- **GUO L-H et al.** *Plasma Proteomics for the Identification of Alzheimer Disease,* 2013
- **STEIN DR ; BURGENER A ; BALL TB.** *Proteomics as a novel HIV immune monitoring tool,* 2013, vol. 8 (2), 140-146
- **DE WIT M ; FIJNEMAN RJA ; VERHEUL HMW ; MEIJER GA ; JIMENEZ CR.** Proteomics in colorectal cancer translational research: Biomarker discovery for clinical applications. *Clinical Biochemistry,* 2013
- **PAULO JA et al.** Mass spectrometry-based proteomics of endoscopically collected pancreatic fluid in chronic pancreatitis research. *Proteom. Clin. Appl.,* 2011, vol. 5 (3-4), 109-120
- **KENTSIS A et al.** Urine proteomics for discovery of improved diagnostic markers of Kawasaki disease. *EMBO Molecular Medicine,* 2012, vol. 5 (2), 210-220
- **MATTISON HA ; STEWART T ; ZHANG J.** Applying bioinformatics to proteomics: Is machine learning the answer to biomarker discovery for PD and MSA?. *Movement Disorders,* 2012, vol. 27 (13), 1595-1597
- **ZHAI X-H ; YU J-K ; LIN C ; WANG L-D ; ZHENG S.** Combining proteomics, serum biomarkers and bioinformatics to discriminate between esophageal squamous cell carcinoma and pre-cancerous lesion. *Journal of Zhejiang University-Science B,* 2013, vol. 13 (12), 964-971